# EUROPÄISCHE PATENTSCHRIFT

(11) **EP 0 710 837 B1**
(45) Veröffentlichungstag und Bekanntmachung des Hinweises auf die Patenterteilung: **01.04.1998**
(21) Anmeldenummer: 95117388.9
(22) Anmeldetag: 04.11.1995
(51) Int. Cl.: G01N 33/49, G01N 33/487, G06T 7/00, G06T 7/60, G01N 25/14

(54) **Verfahren zur Erzeugung von Kristallisaten aus Blut oder anderen menschlichen oder tierischen Flüssigkeiten, sowie zur Auswertung der resultierenden Kristallcluster für diagnostische Zwecke**
Method for producing crystallisation forms from blood or other human or animal body fluids, and for evaluating the resulting crystal clusters for diagnostic purposes
Méthode pour la production de formes de cristallisation de sang ou autres fluides corporels des êtres humains ou des animaux, et pour l'évaluation des agglomérats de cristaux résultants, pour des buts diagnostiques

(30) Priorität: 07.11.1994 DE 4439757
(43) Veröffentlichungstag der Anmeldung: 08.05.1996
(73) Patentinhaber: UJ Heinz AG, Walchwil, Kanton Zug (CH)
(72) Erfinder: Ulrich-Jürgen, Heinz, CCM-Centrum für Cluster Med., Postfach 48 - 72394 Haigerloch (DE)
(74) Vertreter: Jahn-Held, Wilhelm W. Dr.Dr.-Ing. Dipl.-Chem.

(56) Entgegenhaltungen:
- EP-A- 0 307 566
- WO-A-86/04141

## Beschreibung

Gegenstand der Erfindung ist Verfahren zur Erzeugung von Kristallisaten aus Blut oder anderen, menschlichen oder tierischen Flüssigkeiten, sowie zur Auswertung der resultierenden Kristallcluster für diagnostische Zwecke.

Die EP 0307566 beschreibt ein Verfahren zur kristallmorphologischen Blut- und Harnanalyse zur Frühdiagnose. Dieses Verfahren beschreibt Muller- und Tochterfiguren mit Abszisse und Ordinate. Diese Koordinaten entsprechen bestimmten, definierten geometrischen Figuren, die aus Kristallformen und - Strukturen abgeleitet sind. Diese Kristallisate sind durch ein Verfahren zur Kristallisation aus organischen Substanzen, wie Blut, erzeugt für einen Analogievergleich mit typbildenden Kristallbildern von Texturen und Formen aus pathologischen klinisch gesicherten Proben von Patientenblut.

Die durch Analogievergleich erstellte Diagnose wird mittels einfacher Texturvergleiche erstellt. Es wird dadurch die diagnostische Differenzierung eingeschränkt. Es können komplexe Texturen nur mit einfacher Texturanalogien beschrieben werden zum Nachteil der Sicherheit der Diagnose und der Verarbeitungsqualität.

Die DE 41102217 beschreibt ein Verfahren zur Diagnose von Krankheitszuständen des menschlichen Organismus unter Verwendung von Körperflüssigkeiten. Das Verfahren beschreibt die fotooptische Erfassung von als Kristallecluster ausgebildeten Kristallisaten und deten Digitalisierung und Ermittlung von morphologischen, colorimetrischen und densitometrischen Messparametern zu deren Darstellung als digitaler Datensalz unter Bildung eines Datenblockes. Die Datensätze des Datenblockes werden mit in einer Datenbank erfassten und aufbereiteten Datensätzen von anderen Datenblöcken von Kristallclustern, die aus Blut von Menschen, von denen klinisch oder laborantisch gesicherte Diagnosen vorliegen, verglichen, um die Datensätze zu ermitteln, welche die höchste Übereinstimmung aufweisen. Das Ergebnis wird als probabilistische Diagnose ausgegeben.

Dieses Verfahren arbeitet quantitativ und berücksichligt lediglich die quantifizier-baren Messparameter. Es bleiben charakterische Merkmale, wie topologische, strukturelle, texturelle Eigenschaften der Kristallcluster umberücksichtigt, wodurch die Genauigkeit der Diffenrenzierung, eingeschränkt wird. Die Erfassung und Verarbeitung der umfangreichen Datenblöcke erfordert einen hoben, zeitintensiven Rechenaufwand.

Diese Nachteile werden durch das Verfahren der Erfindung vermieden.

Dieses beansprucht eine Kombination von Stoffen als Trägermaterial, welches in kleinen Partikeln ebenso klar auskristallisiert, wie in grossen Clustern. Es wird durch diesen technischen Effekt eine fast lineare Entsprechung zwischen der Sättigung der Lösung und der Kristallisationsgrösse erzielt.

Die WO 86/ 04141 beschreibt ein Verfahren zur Herstellung und zum Vergleich von Kristallisaten. Dieses Verfahren erzeugt aus menschlichem Blut ein Destillat und aus dem Rückstand ein Kalzinat.Aus den beiden Stoffen wird eine Dispersion hergestellt.

Aus diesem wird durch Verdunsten auf einen Objektträger unter bestimmten Bedingungen ein Kristallisat erzeugt mit typenbildenden Kristallbildern mit Texturen und Formen in begrenzten Zonen.

Dieses aufwendige Verfahren mit einer durch Kalzination erzeugten Mischkomponente hat nicht angeregt, nach dem Verfahren der Erfindung Kristallisate herzustellen.

Dieses beansprucht eine Kombination von Stoffen als Trägermaterial, welches in kleinen Partikeln ebenso klar auskristallisiert, wie in grossen Clustern. Es wird durch diesen technischen Effekt eine fast lineare Entsprechung zwischen der Sättigung der Lösung und der Kristallisationsgrösse erzielt.

Die WO-A-8604141 beschreibt ein Verfahren zur Verwendung von Material zur Herstellung von Kristallbildern zur Erkennung von Krankheiten durch optischen Analogievergleich zu in gleicher Weise erzeugter, charakteristischer Typen solcher Kristallbilder.

Es wird aus Blut ein Destillat und aus dem Rückstand ein Kakzinat und daraus eine Dispersion erzeugt und das Stoffgemisch auf einen Objektträger zur Kristallisation gebracht. Die sich dabei bildenden Texturen und Formen werden zum optischen Analogievergleich verwendet.

Das Verfahren der Erfindung erzeugt dagegen mit Wasserdampf ein Macerat und daraus durch Veraschen unter Verwendung von Trägerstoffen aus anorganischen Salzen und organischen Stoffen im definierten Verhältnis ein Filtrat und aus diesem ein Kristallisat. Dieses wird ohne menschliches Mitwirken zur Ermittlung von Koordinaten und daraus einer Numerischen Sequenz verwendet. Diese wird mit anderen Sequenzen einer Datenbank verglichen zur Ermittlung einer Verdachtsdiagnose.

Die EP-A-0-307 566 beschreibt ein Verfahren zur Frühdiagnose durch die Verfahrensstufen der Destillation der Bildung eines Kalzinates. Das Gemisch wird nach Vermischen zur homogenen Phase auf einen mikroskopischen Objektträger aufgebracht unter Bildung von Kristall-Texturen und -Formen unter definierten Bedingungen. Es erfolgt danach deren Analyse bei verschiedenen Vergrößerungen und danach ein mikroskopischer und fotooptischer Analogievergleich mit Texturen und Formen eines gesicherten, pathologischen Zustandes.

Das Verfahren der Erfindung erzeugt dagegen mit destilliertem Wasserdampf und Auffangen des Kondensates ein Macerat. Dieses wird verascht und mit einem anorganischen und organischen Trägermaterial zu einer Suspension vermischt. Aus dieser wird durch Abdampfen eine kristalline Phase erzeugt. Diese wird nach Sannen automatisch durchmustert und daraus werden in Verbindung mit Tafeln mit definierten geometrischen Figuren Koordinaten ermittelt, die zu einer Numerischen Sequenz führen. Aus dieser werden durch Analogievergleich mit anderen Sequenzen in einer Datenbank eine probabilistische Diagnose erstellt.

Es ist die Aufgabe des Verfahrens der Erfindung den Stand der Technik zu verbessern und aus menschlichem oder tierischen Blut oder anderen Flüssigkeiten Kristallcluster herzustellen, aus denen Numerische Sequenzen über Koordinaten aus den Formen und Texturen des Clusters ermittelt werden können zum probabilistischem Vergleich mit in Datenbanken gespeicherten Numerischen Sequenzen aus gesicherten, klinischen Diagnosen zur Ermittlung einer prophylaktischen oder präventiven oder Verdachtsdiagnose. Das Verfahren der Erfindung ist im Patentanspruch 1 definiert. Die Unteransprüche beanspruchen die alternative und bevorzugte Ergänzung des Anspruches 1.

Das Verfahren der Erfindung beansprucht auch die Anwendung der Ermittlung Numerischer Sequenzen anderer, bildgebender, bildverarbeitender, mustererkennender, mustervergleichender Prozesse verschiedener Herkunft zu deren Klassierung und Prognostizierung ,insbesondere solcher Prozesse, denen ein alineares Funktionsmuster zugrunde liegt. Solche Prozesse sind beispielsweise der Einsatz zur Analyse menschlicher Verhaltensmuster und deren Risiken in der Kriminologie, Soziologie, Versicherungswirtschaft. Weitere Anwendungsgebiete sind beispielsweise die Ermittlung ähnlicher Strukturen und Muster in der Archäologie, Kunstgeschichte, Architektur für Expertisen, Kostennutzungsschätzungen, Erfolgsprognosen.

Unter den folgenden Begriffen wird verstanden:
- Formen:: Unter Formen werden einfache, geometrische Grundformen verstanden, die als Elemente einer zusammengesetzten Form vorzugsweise in einem Kristallisat identifizierbar sind.
- Texturen: Unter Texturen werden die vorzugsweise bei der Kristallisation erzeugten und geometrisch ermittelten oder die über Suchmuster morphogenetisch analogen, flächenhaften Aggregate verstanden.
- Fraktal :: Dieses definiert die Struktur der Ausbreitung einer Form oder Textur innerhalb einer markierbaren Fläche.
- Kontur:: Diese definiert das Grenzverhalten verschiedener Formen und Flächen in der Form oder Textur selbst oder dieser gegenüber ihrer Umgebung.
- Präfix :: Diese beschreibt das Verhältnis, wie dieses bei der Kristallisation von Formen und Texturen entsteht, in dem die Formen und/oder Texturen aus Tafel 2 zur Form oder Textur aus Tafel 1 steht.
- Postfix :: Dieses gibt die Anzahl an, in der die Formen und/oder Texturen aus Tafel 2 im Laufe einer Kristallisation erscheinen oder beschreibt den besonderen Winkel, in dem die Form und/ oder Textur aus Tafel 2 zur Form und/ oder Textur aus Tafel 1 steht.
- Lumen:: Abbildungsverhältnisse der Textur gegenüber einem oder mehreren Unter- bzw. Hintergründen.
- Cluster: Unter Cluster werden regelmäßig und/oder unregelmäßig strukturierte Anhäufungen biologischer oder anorganischer Masse mit einer räumlichen Ausdehnung verstanden.
- numerische Sequenzen (NS): Diese definieren Zahlenreihen und Zahlengruppen, die zusammen die Form oder Textur numerisch mit den Tafeln der Erfindung bestimmen.
- Muster: Unter Muster werden jene optisch sichtbaren Abbildungen unterschiedlicher Formen und Texturen verstanden, die formal und inhaltlich repräsentative und signifikante Anordnungen repräsentieren.
- Analogievergleich: Es werden unbekannte, zu suchende Abbildungen, wie Formen, Texturen, als optisch sichtbare Aggregate mit bekannten, in ihrer Bedeutung gesicherten Aggregaten verglichen, indem die Übereinstimmung Numerischer Sequenzen ermittelt wird.
- Abbildungsmasstab: Dieser bezeichnet die optische Auflösung eines Objektes, wie Kristallcluster
- Bilddatei: In dieser wird nach Formaten abgespeichert, z.B.
Bitmap (BMT), Fa. IBM
TIFF (TIF) Fa. ZIFF

### Texturstamm

Stärkster Vektor eines Dentriten in Ausbreitungsrichtung des Kristallisats.

### Texturzweige

Stärkster Einzelvektor oder Vektorenschar, die in einheitlicher Ausbreitungsrichtung vom Texturstamm abgehen.

### Innere Strukturierung

Morphologische Beschaffenheit einer Form und/oder Textur innerhalb ihrer Grenze.

### Äußere Grenzstrukturierung

Morphologische Beschaffenheit einer Form und/oder Textur an ihrer Grenze zu einer anderen Form und/oder Textur oder einer amorphen Umgebung.

### Relative Dichte

Anzahl gleicher Formen und/oder Strukturen auf einem definierten Raum im Verhältnis zu anderen Formen und/oder Strukturen in demselben Raum.

### Grenzbildung

Fähigkeit des Kristallisats, sich bei seiner Bildung gegen die Trägerumgebung oder andere, kristallisierende Felder abzugrenzen.

### Grenzstruktur

Morphologische Beschaffenheit einer Grenze.

### Innere Kontrastbildung

Maß der Schichtungsdichte des Kristallisats.

### Schwerpunkt des Ortes

Ortbestimmung der selektierten Form und/oder Textur in Bezug auf das gesamte kristalline Cluster.

### Gesamtform

Der aus den Formen und/oder Texturen und den Texturen A und B gebildete Komplex, der sich durch seine Innere Strukturierung und durch Grenzen von anderen Komplexen unterscheidet.

### Relative Verteilung

Maß der Häufigkeit des Erscheinens einer Form und/oder Textur im Verhältnis zu benachbarten anderen Formen und/oder Texturen.

### Organisationsform

Muster der Proportionierung der Formen und/oder Strukturen gegeneinander.

### Programm

Dieses ist ein Basisprogramm der Bildmustererkennung, welches über die Schnittstelle der Numerischen Sequenz mit Datenbanken verbunden ist.

Das Verfahren der Erfindung wird durch die folgenden Beispiele erläutert.

### Beispiel 1

Verfahren der Erfindung zur Herstellung eines Krisallisates aus Venenblut einer Patientin und Parameter zur Ermittlung der Numerischen Sequenz

Es werden 2 ml des Venenblutes der Patientin in einen Auschlusskolben eingefüllt und mit bidestilliertem Wasser in einer Menge von 25 ml vermischt. Danach werden je 3 g NaCl als Kochsalz und Zucker als Rohrzucker mit der Lösung homogen zu einer Suspension vermischt unter Rotieren mit 85 U/min in 60 min bei 20° C.

In dieses Gemisch wird 4-fach destilliertes Wasser als Dampf eingeleitet durch ein Einleitungsrohr unter Aufwirbeln und Kondensieren des aufsteigenden Wasserdampfes durch einen Intensivkühler.Es werden 10 ml Kondensat in einem vorgelegten Gefäss als Wasserdampfmazerat (WDM) aufgefangen.

Es werden 24 µl des WDM mit 10 mg eines bei 640°C unter Luftabschluss veraschten Gemisches aus NaCl, Hühnereiweiss, Fruktose,Glukose, Stärke im Verhältnis 1: 1 : 1 : 1 : 2 zu einer praktisch gesättigten Suspension vermischt. Die Suspension wird durch einen Mikrofilter gefiltert.

Das Filtrat wird mit einer Pipette auf einen mikroskopischen, gläsernen Objektträger in einem Durchmesser von etwa 8 mm aufgetropft.

Der Objektträger mit der aufgetropften Suspension wird auf eine auf 24°C vorgeheizte Präzisionsheizplatte aufgebracht. Die Suspension dampft in etwa 90 min zur kristallienen Phase ab.

Es werden danach die folgenden Verfahrensstufen gemäss Anspruch 1 durchgeführt:
Der Objektträger mit der kristallinen Phase wird unter ein Lichtmikroskop gelegt und im Dunkelfeld mit dem Objekt 4 auf die am häufigsten auftretenden, jedoch ähnliche Formen und/ oder Texturen durchgemustert. Nachdem diese Form und/oder Textur ermittelt ist, werden mit dem Objektiv 8 die gefundenen Formen und/oder Texturen auf singulär- individuelle Merkmale durchgemustert und die Form und/oder Textur selektiert, die bei ähnlicher morphologischer Struktur den höchsten Grad der Differentierung in seiner Binnenstruktur aufweist und Bildspeicherung, danach automatische Durchmusterung mit einem Programm zur Erkennung von Bildmustern zur Ermittlung folgender Werte als Form,Fraktal a,b,c; Cluster a,b,c,d; Kontur a,b,c,d; Präfix A, Textur A, Postfix A, Präfix B, Textur B, Postfix B, Lumen a,b,c,:
Ermittlung der Koordinaten der Form als Element möglicher Texturen aus Tafel 1, Form,
danach Ermittlung der Koordinaten der Anordnung der Formen aus Tafel 1, bezogen auf die Fläche x, y, aus Tafel 11,
Fraktal a,
   danach Ermittlung der vektoriellen Ausrichtung der Texturstämme aus Tafel 11 mit Tafel 111,
Fraktal b,
   danach Ermittlung der vektoriellen Ausrichtung der Texturzweige aus Tafel 11 mit Tafel 1111,
Fraktal c,
   danach Ermittlung der auf den Raum x, y, z bezogenen Anordnung der Formen aus Tafel 1 mit Tafel 12,
Cluster a,
   danach Ermittlung der inneren Struturierung der durch Tafel 12 bezeichneten Texturen mit Tafel 121,
Cluster b,
   danach Ermittlung der äusseren Grenzstrukturen der durch Tafel 12 bezeichneten Texturen mit Tafel 122,
Cluster c,
   danach Ermittlung der relativen Dichte und/ oder Strukturierung der durch die Tafel 12 definierten Textur mit Tafel 123,
Cluster d,
   danach Ermittlung der Grenzbildung der durch die Tafel 12 definierten Formen und/ oder Texturen durch Kontrast gegenüber der Umgebung mit Tafel 13,
Kontur a,
   danach Ermittlung der inneren Kontrastbildung der durch die Tafel 12 definierten Formen und/ oder Texturen mit Tafel 131,
Kontur b,
   danach Ermittlung des Schwerpunktes des Ortes der durch die Tafel 12 definierten Formen und/ oder Texturen, ausgedrückt in Sektoren mit Tafel 132,
Kontur c,
   danach Ermittlung der Gesamtform, die von allen gleichartigen Formen und/ oder Texturen nach Tafel 12 gebildet wird,
Kontur d,
   danach Ermittlung des Verhältnisses, in dem die Formen und/ oder Texturen aus Tafel 2 zu den Formen und/ Texturen aus Tafel 1 stehen mit Tafel 21,
Präfix a,
   danach Ermittlung der Formen und/ Texturen, die in einem durch Tafel 21 definierten, räumlichen Verhältnis zu den Formen oder Texturen der Tafel 1 stehen mit Tafel 2,
Textur a,
   danach Ermittlung der Anzahl des Auftretens der Formen und/ oder Texturen aus Tafel 2 und/ oder des besonderen Winkels, in dem die Formen und/ oder Texturen aus Tafel 2 zu den Formen und/ oder Texturen aus Tafel 1 stehen mit Tafel 22,
Postfix a,
   danach Ermittlung des Verhältnisses, in dem die Formen und/ oder Texturen aus Tafel 3 zu den Formen und/ oder Texturen aus Tafel 1 und/oder Tafel 2 stehen mit Tafel 31,
Präfix b,
   danach Ermittlung der Formen und/ oder Texturen, die in einem definierten, räumlichen Verhältnis zu den Formen und/ oder Texturen der Tafel 1 und/oder Tafel 2 stehen mit Tafel 3,
Textur b,
   danach Ermittlung der Anzahl des Auftretens der Formen und/oder Texturen aus Tafel 3 und/oder des besonderen Winkels, in dem die Form und/oder Textur aus Tafel 3 zu den Formen und/ oder Texturen aus Tafel 1 und/oder Tafel 2 stehen mit Tafel 32,
Postfix b,
   danach Ermittlung des Abbildungsverhältnisses der Formen und/ oder Texturen aus Tafel 1 gegenüber ihrem Untergrund oder Hintergrund mit Tafel 12,
Lumen a,
   danach Ermittlung des Abbildungsverhältnisses der Textur A aus Tafel 2 gegenüber ihrem Untergrund oder Hintergrund mit Tafel 22,
Lumen b,
   danach Ermittlung des Abbildungsverhältnisses der Textur B aus Tafel 3 gegenüber ihrem Untergrund oder Hintergrund mit Tafel 32,
Lumen c,
   zur Numerischen Sequenz,
   wobei die Reihenfolge der Ermittlung die gleiche ist und diese maschinell oder manuell durchgeführt wird, danach Eingabe der Numerischen Sequenz in eine Datenbank als Schnittstelle, in welcher die Numerischen Sequenzen aus klinisch gesicherten Diagnosen abgespeichert sind zur Ermittlung durch probabilistischen Vergleich einer prophylaktischen und/oder präventiven und/ oder Verdachtsdiganose.

### Beispiel 2

Verfahren der Erfindung zur Ermittlung der Numerischen Sequenz aus dem nach Anspruch 1 ermittelten Kristallcluster aus dem Blut einer Frau mit der klinisch gesicherten Diagnose " Mamma Carcinom mit Metastasen." Ermittlung der Numerischen Sequenz mit automatischer Bilderkennung. Es ist alternativ die visuelle Ermittlung gegeben.

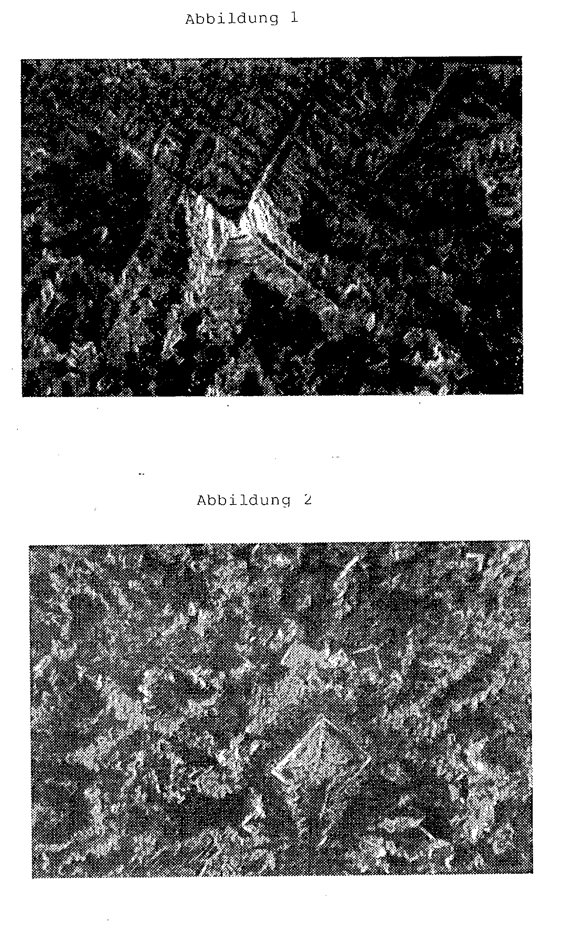

Die Ermittlung der Form der Textur nach Abbildung 1 entspricht in Tafel 1 den Koordinaten 3400, wobei die Ziffeen 34 die Raute bezeichnen und die Ziffern 00 bestätigen, dass eine kompakte Form ohne in- oder exklusive Momente vorliegt.

Die Raute und nicht ein Quadrat ergibt sich aus der durch die Auswucherungen an den Spitzen dargestellten Symmetrieachse.

Mit der Tafel 11 wird die Anordnung der Form 1 in ihrer Umgebung durch die Koordinaten 31 erfasst, indem zuerst der Wert der x- Achse und danach der Wert der y- Achse ermittelt wird und damit die astartigen Auswucherungen der Form an den Enden definiert werden.

Die Tafel 111 definiert die vektorielle Ausrichtung des Stammes der Textur, ausgehend vom Mittelpunkt durch den Wert 4 , also kreuzförmig symmetrisch.

Die Tafel 1111 definiert das Verhalten der Abzweigungen oder der Zweige gegenüber dem Stamm. Dieser ist nach der Abbildung vorzugsweise geradlinig , woraus die Ziffer 1 resultiert.

Es resultiert daraus für die Gesamtdarstellung die unter dem Begriff des Fraktals zusammengefasste Brechung und Ausbreitung der Grundform oder Grundtextur im flächigen Raum mit der Ziffer 3441.

Die Tafeln 12- 123 definieren die räumliche Anordnung der Gesamttextur.Mit Tafel 12 ist die Art der Zuordnung der Formenelemente, die Clusterbildung zueinander definiert.

Die Abbildung 1 zeigt symmetrische Verschmelzungsformen, die mit dem Ausdruck x = 1, y = 4, resultierend 14, definiert sind.

Die Tafel 121 definiert, in welchem Verhältnis die homogenen Elemente der Grundform zueinander stehen. Dies sind die in der Grundform aufgeschichteten, grundformähnlichen Rauten, welche die Ziffer 3 ergeben und darauf hinweisen, dass die Grundform mehr als dreimal über sich gestuft ist.

Die Tafel 122 definiert, inwieweit die Gesamttextur eine einheitliche Kontur aufweist, die eine Grundform erkennen lässt.

Abbildung 1 zeigt keine eindeutig als Form ermittelbare Kontur, woraus die Ziffer 9 resultiert, die beliebigen Konturformen entspricht.

Die Tafel 123 definiert homogene Kleinstelemente, wie punktartig mit in der Masse flächenbildenden Elementen, aus denen sich die Grundform und die Textur zusammensetzen.

Es sind dies weniger diese Elemente sondern das Raster, welches durch die Textur gebildet und mit der Ziffer 8 definiert ist.

Es resultiert für die Numerische Sequenz des Clusters die Ziffer 14398.

Die Tafeln 13 und 131 definieren das innere und das nach aussen gerichtete Kontrastverhalten der Textur des Kristallclusters.

Die Tafeln 132 und 133 definieren den Ort, an dem das ausgewählte Cluster auf dem Gesamtcluster liegt und welche Grossform dieses zur Unterscheidung von seiner Umgebung bildet.

Die Tafel 13 definiert den Kontrast der Textur zur Umgebung nach Abbildung 1, woraus die Ziffer 7 resultiert , da die Textur starken Kontrast bei unperiodischer Klüftung aufweist.

Der innere Kontrast der Textur weist Schlieren auf, woraus die Ziffer 4 für im wesentlichen parallel verlaufende Schlieren resultiert.

Aus der Tafel 132 ergibt sich die Ziffer 1, da die Textur im äusseren Segment sich befindet.

Da die ausgewählte Textur in Abbildung 1 keine umschriebene Gesamtform bildet, ergibt sich für die Tafel 133 der Wert 0.

Es resultiert für die Numerische Sequenz der Kontur die Ziffer 7410.

Die Tafel 14 definiert das Lumen. Diese ergibt die Erscheinungsform der Textur im mikroskopischen Dunkelfeld.

Es ergibt sich die Ziffer 11, da die Form auf dunklem Hintergrund sich befindet. Tafel 2 und 3 definieren die zur Form aus Tafel 1 ergänzenden Formen und Texturen.

Die Abbildung 1 zeigt zwei unterschiedliche Texturen, die von der Form aus Tafel 1 ausgehen und zwar die obere Textur mit Rasterformen und die untere Textur als eine gerichtete Schichtung. Die obere ist die Textur A mit der Tafel 2. Es ist die gegenständige Figur x = 13 und y= 12 definiert.

Es ist das Präfix zu Tafel 2 mit Tafel 21 definiert. Dieses ist der Ausdruck für die Durchdringung der Formen aus Tafel 1 durch die Form aus Tafel 2, woraus die Ziffer 8 resultiert.

Die Durchdringung liegt nicht in der Symmetrieachse der Form aus Tafel 1, sondern diese liegt im rechten Winkel dazu um 90° versetzt.

Es resultiert das Postfix mit der Ziffer 90.

Es resultiert die Numerische Sequenz der Textur A mit der Ziffer 8131290.

Tafel 23 definiert die Erscheinungsform der Textur A unter dem Mikroskop mit der Ziffer 11.

Es stellt die Textur B die untere, rechtwinklige, von der Form aus Tafel 1 abgezweigte Textur dar. Es resultiert für die ineinander geschichteten, eingewinkelten Dreiecke nach Tafel 3 x = 13 und y = 24.

Diese Formen sind der Form auf Tafel 1 angelagert. Diese sind mit Ziffer 6 der Präfixe aus Tafel 31 definiert.Diese Textur existiert zweimal, was mit dem Postfix 02 aus Tafel 32 definiert ist.

Es resultiert die Numerische Sequenz der Textur B mit der Ziffer 6132402.

Die Erscheinungsform unter dem Mikroskop, Lumen b, wird mit der Ziffer 11 definiert. Es erhält damit das Gesamtlumen als Ausdruck der Einzellumina a,b, c die Numerische Sequenz 111111.

Es ist nunmehr die Numerische Sequenz der Abbildung 1 definiert. Diese ist durch automatische, bilderkennende Erstellung über geometrischen Mustervergleich nach probabilistischer Berechnung oder auch visuell erstellt.

Die ermittelte Numerische Sequenz ist die folgende:

| Tafel | Parameter | Sequenz |
|---|---|---|
| 1 | Form | 3400 |
| II | Fraktal a | 34 |
| III | Fraktal b | 4 |
| IIII, | Fraktal c | 1 |
| | | |
| 12 | Cluster a | 14 |
| 121 | Cluster b | 3 |
| 122 | Cluster c | 9 |
| 123 | Cluster d | 8 |
| 13 | Kontur a | 7 |
| 131 | Kontur b | 4 |
| 132 | Kontur c | 1 |
| 133 | Kontur d | 0 |
| 14 | Lumen a | 11 |
| 2 | Textur A | 1312 |
| | | |
| 21 | Präfix A | 8 |
| 22 | Postfix A | 90 |
| 23 | Lumen b | 11 |
| 3 | Textur B | 1324 |
| 31 | Präfix B | 6 |
| 32 | Postfix B | 02 |
| 33 | Lumen c | 11 |

Die ermittelte Numerische Sequenz ist in linearer Schreibweise wie folgt definiert:
(Form) (Fraktal a,b,c ) (Cluster a,b,c,d ) (Kontur a,b,c,d,) ( Präfix A ) (Textur A) ( Postfix A) ( Präfix B) ( Textur B) ( Postfix B) ( Lumen a,b,c ) :
3400 3441 14398 7410 8131290 6132402 111111 .

Mit der ermittelten Numerischen Sequenz werden Kristallcluster aus den Bluten von Patienten mit unbekannter Diagnose auf Übereinstimmung automatisch verglichen. Es wird eine Aufschluss - oder Hinweisdiagnose im vorklinischen Bereich zur Prophylaxe und Prävention als Ergebnis erhalten.

### Beispiel 3

Verfahren der Erfindung zur Ermittlung der Numerischen Sequenz aus dem nach Anspruch 1 ermittelten Kristallcluster aus dem Blut eines Mannes mit der klinisch gesicherten Diagnose _{"}Morbus Bechterew". Ermittlung der Sequenz mit automatischer Bilderkennung. Es ist alternativ die visuelle Ermittlung gegeben.

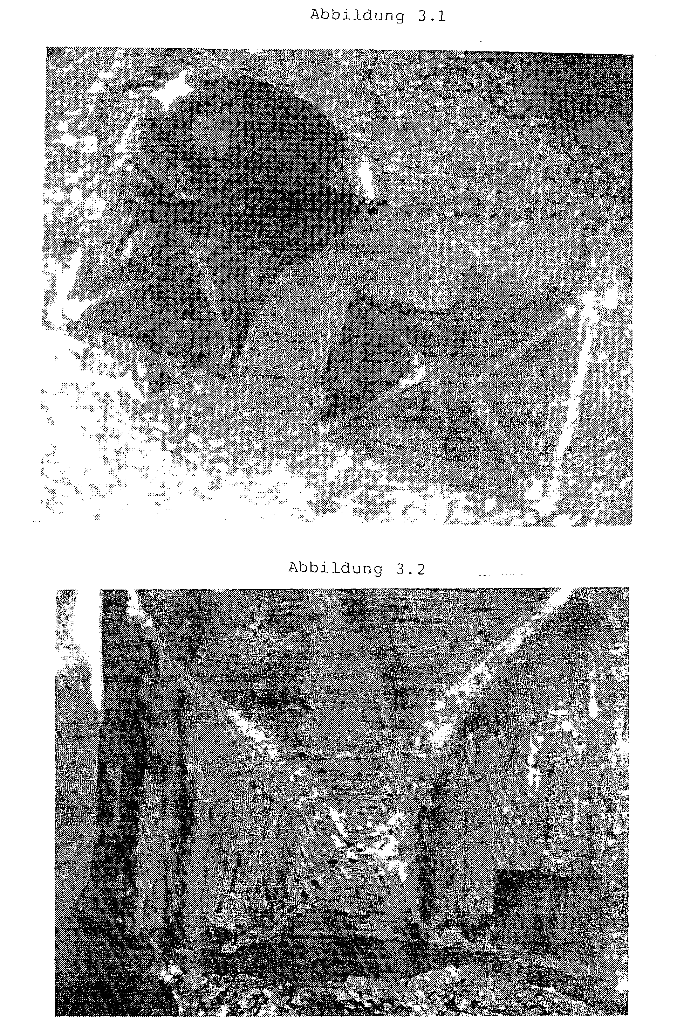

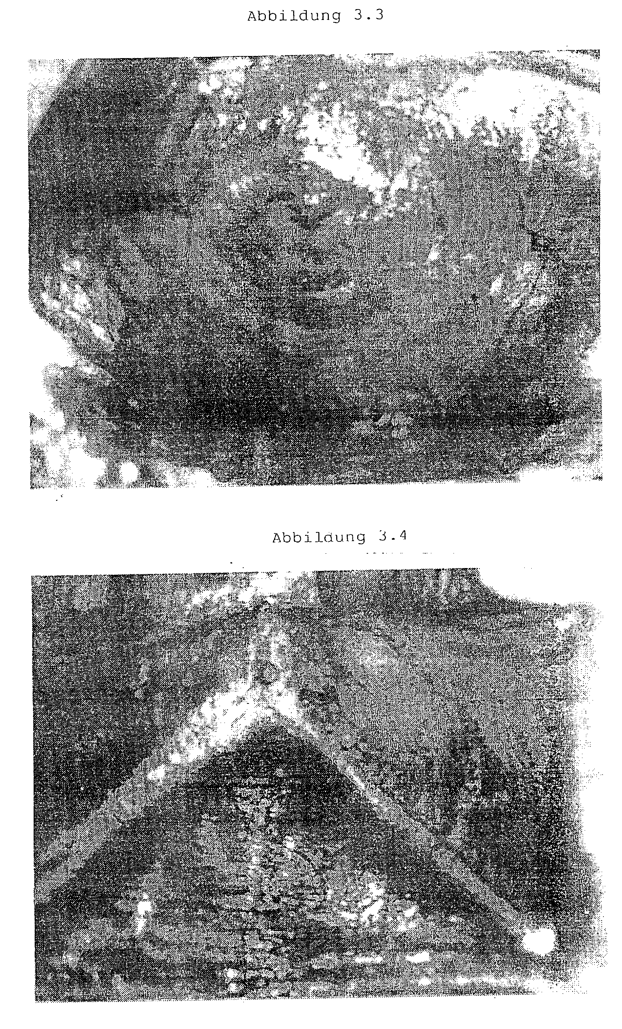

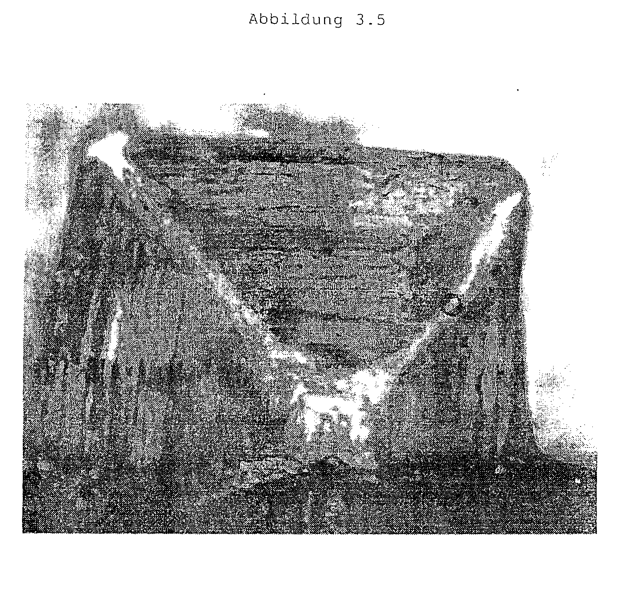

Das Kristallcluster wird auf seinem Objektträger unter das Lichtmikroskop gelegt und unter der Beleuchtung des Dunkelfeldes mit einer elektronischen Kamera zur Erstellung einer Gesamtübersicht im Abbildungsmaßstab 1 unter Verwendung eines Mikroskopobjektives mit der Vergrößerung 1 und einem vor die Kamera geschalteten Okular mit der Vergrößerung 10 gescannt. Abbildung 3-1 zeigt das so erzielte Ergebnis mit zwei getrennt auftretenden Clustergruppen. Die linke Gruppe besteht aus zwei rechteckigen, ineinandergeschichteten Clustern, die rechte Gruppe aus einem in eine eliptoide Form hineingeschichtetes Rechteck. Nach dem Verfahren der Erfindung wird nun jene Form und/oder Textur gesucht, die am häufigsten auftaucht, hier das Rechteck, und daraus jene Form und/oder Textur, die dabei am differenziertesten ist, in diesem Falle das Rechteck der rechten Gruppe mit der aufgesetzten Linse. Zur Differenzierung der Binnenzeichnung der Formen und/oder Texturen werden nun die Einzelteile der beiden Gruppen mit dem Objektiv 4 gescannt. Das Ergebnis sind die Abbildung 3-2 für das Rechteck der rechten Gruppe und die Abbildung 3-3 für das eliptoide Vieleck, sowie die Abbildung 3-4 für das größere Rechteck der linken Gruppe und Abbildung 3-5 für das kleinere. Die Vergrößerung zeigt, daß die beiden Rechtecke der linken Gruppe auch eine aufgesetzte Rundform, jedoch nicht eliptoid, besitzen, wohingegen durch den geringeren Kontrast der höheren Vergrößerung die eliptoid-ähnliche Form des Rechteckes der rechten Gruppe diffuser erscheint.

Zur Bestimmung der Numerischen Sequenz werden die Koordinaten der Rechteckes der rechten Gruppe genommen, da diese Form und/oder Textur aus der Menge der meisten Gleichen die höchste, formale Differenzierung aufweist.

Da das zu bestimmende Rechteck nicht allein auftritt, sondern mit dem Vieleck verbunden ist, wird nicht die Einzelform des Rechteckes allein bestimmt, sondern das zu Bestimmende als Textur, also als komplexe Form aufgefaßt, die aus einem dominanten Rechteck und einem subdominanten Vieleck besteht.

Das Rechteck wird definiert mit 3122 auf Tafel 1, seine flächige Anordnung mit dem Vieleck mit 85 aus Tafel 11 und, da weder der Clusterstamm, hier dargestellt durch die Längsachse des Rechteckes, eine Ausdehnungsrichtung zeigt, noch der Clusterzweig, hier dargestellt durch die Querachse des Rechteckes, werden die Werte für die Tafel 111 und 1111 jeweils mit 0 ermittelt. Das Rechteck der rechten Gruppe schiebt sich über das Vieleck, was nach Tafel 12 mit 21 definiert wird.

Das Rechteck zeigt einen vielfach gestuften Aufbau, was nach Tafel 121 mit 3 definiert wird, die äußere Grenzform der rechten Gruppe ist nicht symmetrisch und regelmäßig und wird daher nach Tafel 122 mit 9 definiert. Da das Rechteck der rechten Gruppe in seinem Aufbau eine zentralradiale Schichtung zeigt, wird dies nach Tafel 123 mit 7 definiert.

Die rechte Gruppe liegt in einem diffusen Umfeld, wodurch seine Grenze nach Tafel 13 durch 4 definiert wird. Der innere Kontrast ist stark bis schwach, was nach Tafel 131 durch 2 definiert wird. Die Gruppe liegt im Sektor 1 und 2 des Gesamtclusters, was nach Tafel 132 durch 3 definiert wird. Die aus dem Rechteck und dem Vieleck resultierende Gesamtform der Gruppe ist keiner regelmäßigen geometrischen Struktur zuzuordnen, was nach Tafel 133 durch den Wert 0 definiert wird.

Rechteck ist eine eliptoide Form mit spitzen Scheiteln aufgelagert, was nach der Tafel 21 mit dem Wert 1, nach der Tafel 2 mit dem Wert 0112 und nach der Tafel 22 mit dem Wert 01 definiert wird. Das Vieleck unterläuft das Rechteck an seiner äußeren Grenze und wird definiert aus Tafel 31 mit dem Wert 6, aus Tafel 3 mit dem Wert 7111 und aus der Tafel 32 mit dem Wert 15, da die Längsachse des Rechteckes gegenüber der des Vieleckes einen Winkel von etwa 15° aufweist. Das Rechteck stellt sich geschichtet plastisch dar, sein aufgesetzter Eliptoid plastisch auf umgebender Schichtung und das angrenzende Vieleck als plastisch, was nach der Tafel 14 durch den Wert 11, nach Tafel 23 durch den Wert 40, nach Tafel 33 durch den Wert 10 definiert wird.

Damit ist die Numerische Sequenz für die rechte Gruppe des Kristallclusters und damit nach dem Verfahren der Erfindung für das Kristallcluster wie folgt in linearer Schreibweise definiert:
(Form)(Fraktal a,b,c)(Cluster a,b,c,d)(Kontur a,b,c,d)(Präfix A) (Textur A)(Postfix A)(Präfix B)(Textur B)(Postfix B)(Lumen a,b,c): 3122 8500 21397 4230 1011201 6711115 114010.

Damit ist die Numerische Sequenz für das Kranheitsbild Morbus Bechterew für den Patienten mit gesicherter Diagnose ermittelt.

In gleicher Weise wird nach dem Verfahren der Erfindung die Numerische Sequenz aus Kristallclustern von Bluten mit unbekannter Diagnose ermittelt. Diese wird danach in eine Datenbank eingegeben, in der die Numerischen Sequenzen von gesicherten, klinischen Diagnosen eingespeichert sind. Aus dem Grad der Übereinstimmung der Numerischen Sequenzen resultiert eine probabilistische Diagnose für die Prophylaxe und Prävention.

Um eine Auflösung feinerer, unterscheidender und gegen andere Formen und/ oder Texturen abgrenzende Binnenstruktur zu ermitteln, kann auch der Abbildungsmasstab 16 erforderlich werden.

### Beispiel 4

Fotooptisches Erfassen mit einem handelsüblichen Scannertisch und einer handelsüblichen, elektronischen Kamera unter Verwendung handelsüblicher Software zur Bildverarbeitung.

Digitalisieren des erfaßten und gescannten Bildes zu einer Datei, vorzugsweise im Format *.bmp.

Optimieren des gescannten Bildes auf maximalen Kontrast bei optimaler Schärfe mit dem Bildverarbeitungsprogramm.-

Ermitteln der geometrischen Meßparameter des gescannten Bildes durch die Bildanalyse des Bildverarbeitungsprogrammes.

Erstellen einer Statistik der gemessenen Werte des gescannten Bildes durch den Statistikmodul des Bildverarbeitungsprogramms.

Auswahl der geometrischen Form mit der größten Anzahl von Repräsentanten bei möglichst geringem Abstand zum gemessenen größten Wert einer Form durch den Statistikmodul des Bildverarbeitungsprogrammes als Bezugsform.

Nach der Ermittlung der Bezugsform wird diese als Suchraster auf das gesamte gescannte Bild mit Hilfe des Bildverarbeitungsprogrammes aufgelegt.

Alle Formen, die der Bezugsform entsprechen, werden markiert und dann auf ihren Binnenkontrast im Verhältnis zu den Kontrastbildenden Partikeln der gemessenen Binnenfläche als Ausdruck ihrer Binnendifferenzierung hin vom Bildverarbeitungsprogramm untersucht. Jene Form mit der höchsten Binnendifferenzierung gilt als gewonnene Referenzform des betreffenden Kristallisats.

Diese gewonnene Referenzform wird abgespeichert als Referenzdatensatz des betreffenden Kristallisats.

Die gewonnene Referenzform wird vom Expertensystem anhand der in das System eingespeicherten grafischen Tafeln in eine Numerische Sequenz (NSQ) umgesetzt. Dies geschieht durch probabilistischen Vergleich der gewonnenen Referenzform mit gespeicherten, früher gewonnenen Referenzformen. Das Vergleichergebnis ist eine _{"}Sieger"-Referenzform mit ihrer gespeicherten _{"}Sieger"-NSQ. Diese Sieger-NSQ wird der gewonnenen Referenzform als Ergebnis wahrscheinlichster Übereinstimmung zugeordnet.

Die Sieger-NSQ wird vom Expertensystem mit den in ihm gespeicherten Diagnoseergebnissen probabilistisch verglichen. Dies führt zu einer nach der Wahrscheinlichkeit geordneten Ergebnisliste der Übereinstimmungen zwischen der Sieger-NSQ und den Diagnoseinhalten.

Das Ergebnis wird als probabilistische Diagnose ausgedruckt.

Die Abbildungen stellen unter dem Lichtmikroskop im Dunkelfeld hergestellte Kristallcluster dar.
Abbildung 1 entspricht dem Beispiel 1 im Abbildungsmasstab 4,
Abbildung 2 entspricht dem Beispiel 2 im Abbildungsmasstab 4,

Die Abbildungen 3.1 bis 3.5 entsprechen dem Beispiel 3 und zwar:
Abbildung 3.1: Übersichtsabbildung des Kristallclusters im Abbildungsmasstab 1.
Abbildung 3.2: Abbildung des Teilclusters 1 im Abbildungsmasstab 4,
Abbildung 3.3: Abbildung des Teilclusters 2 im Abbildungsmasstab 4,
Abbildung 3.4: Abbildung des Teilclusters 3 im Abbildungsmasstab 4,
Abbildung 3.5: Abbildung des Teilclusters 4 im Abbildungsmasstab 4.

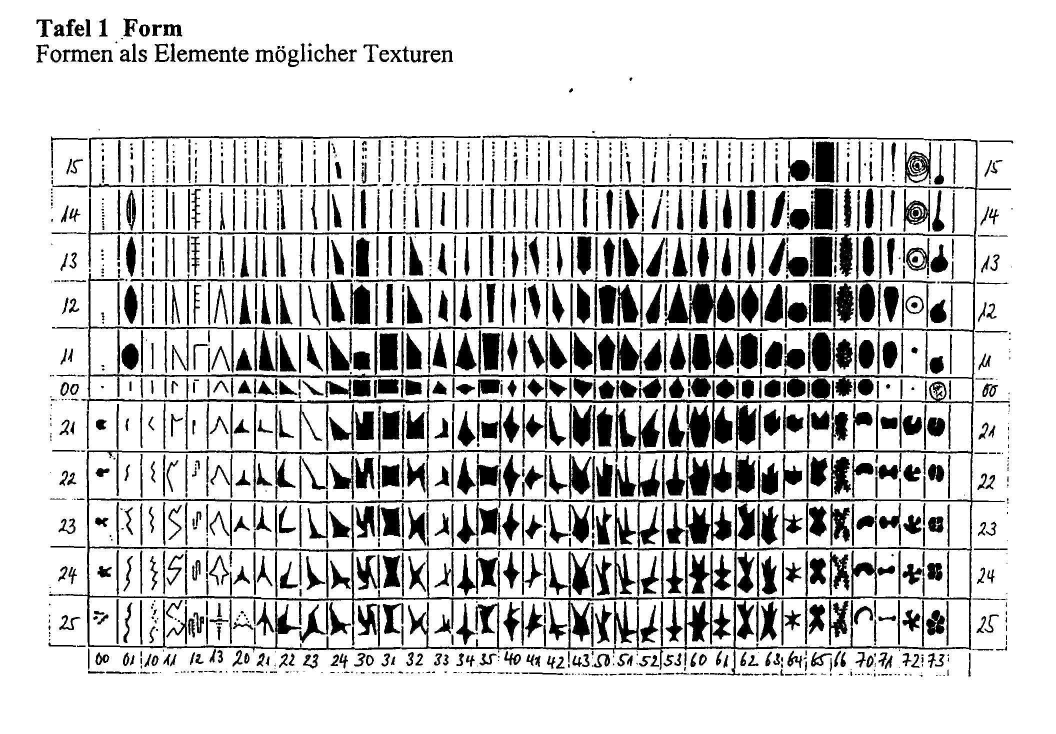

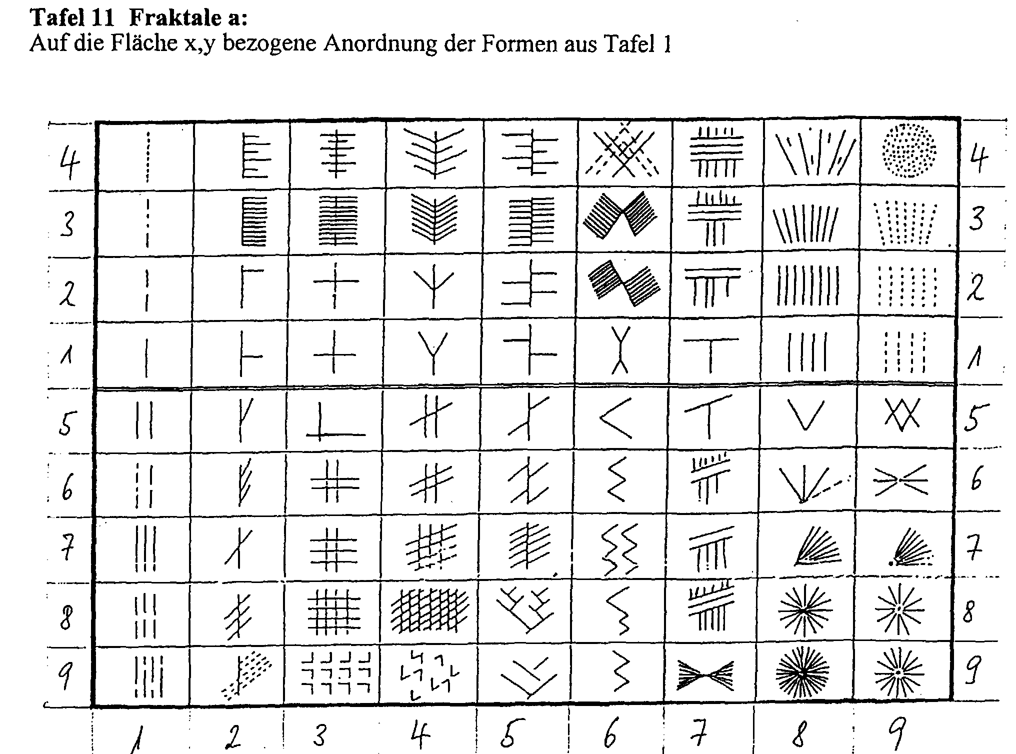

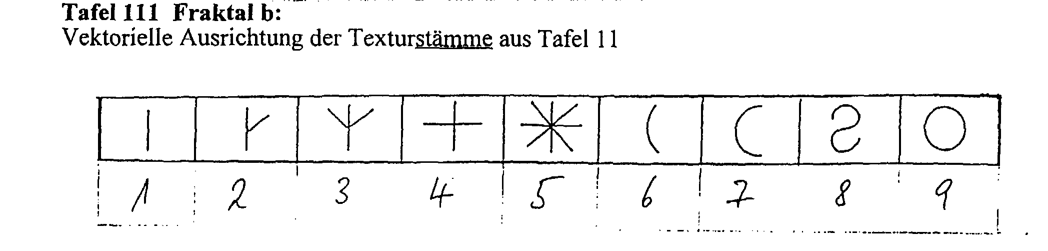

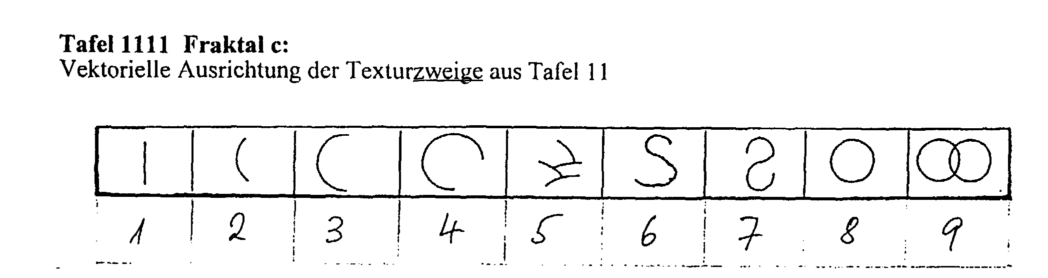

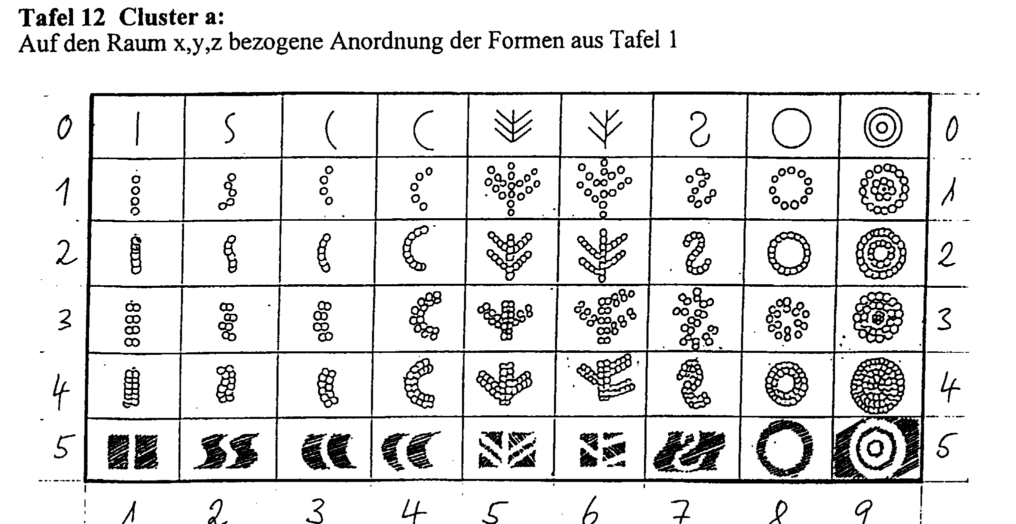

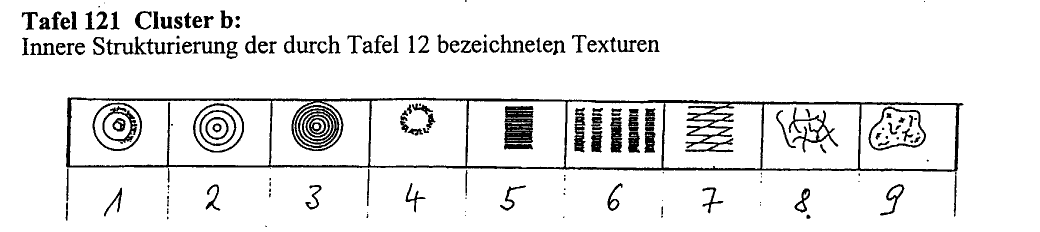

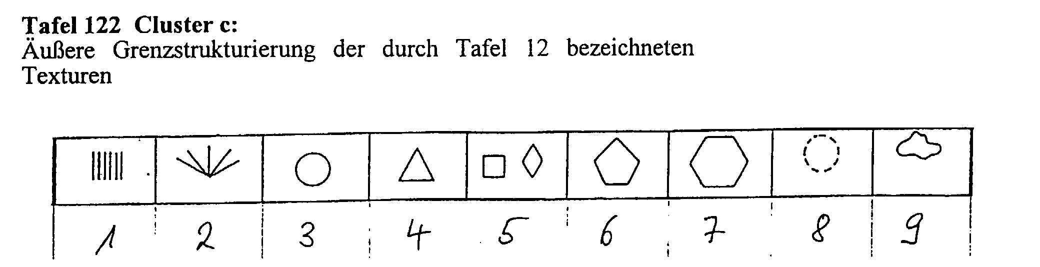

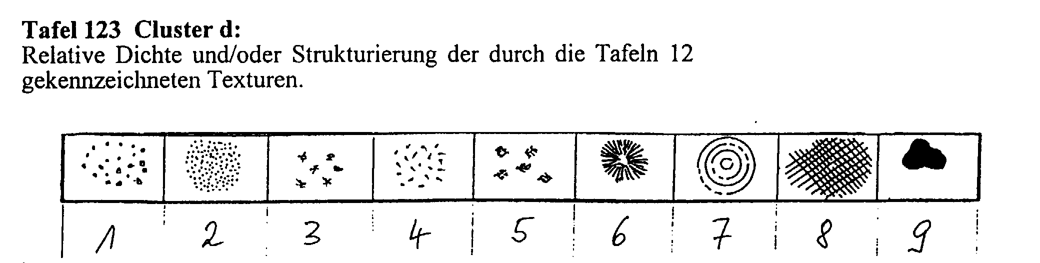

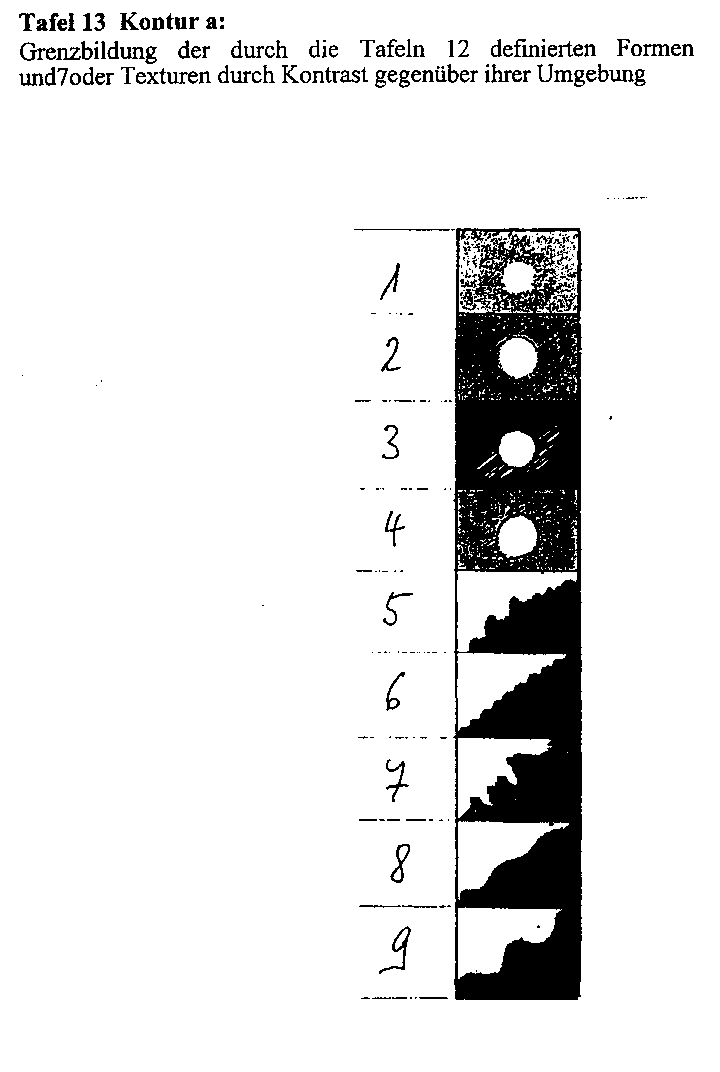

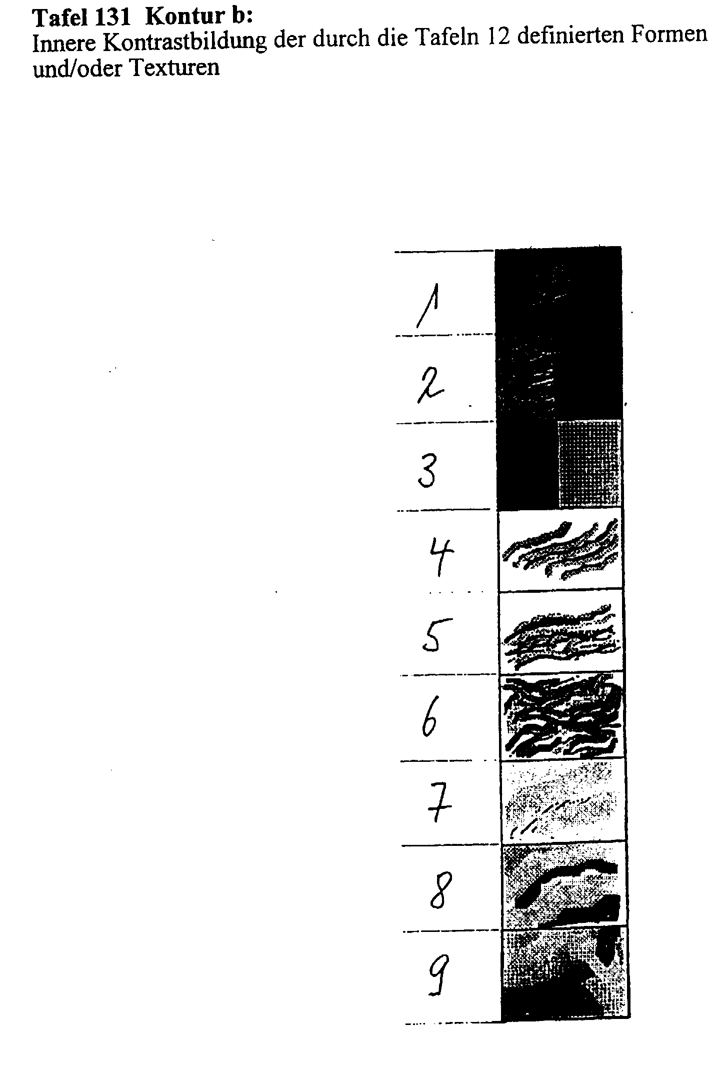

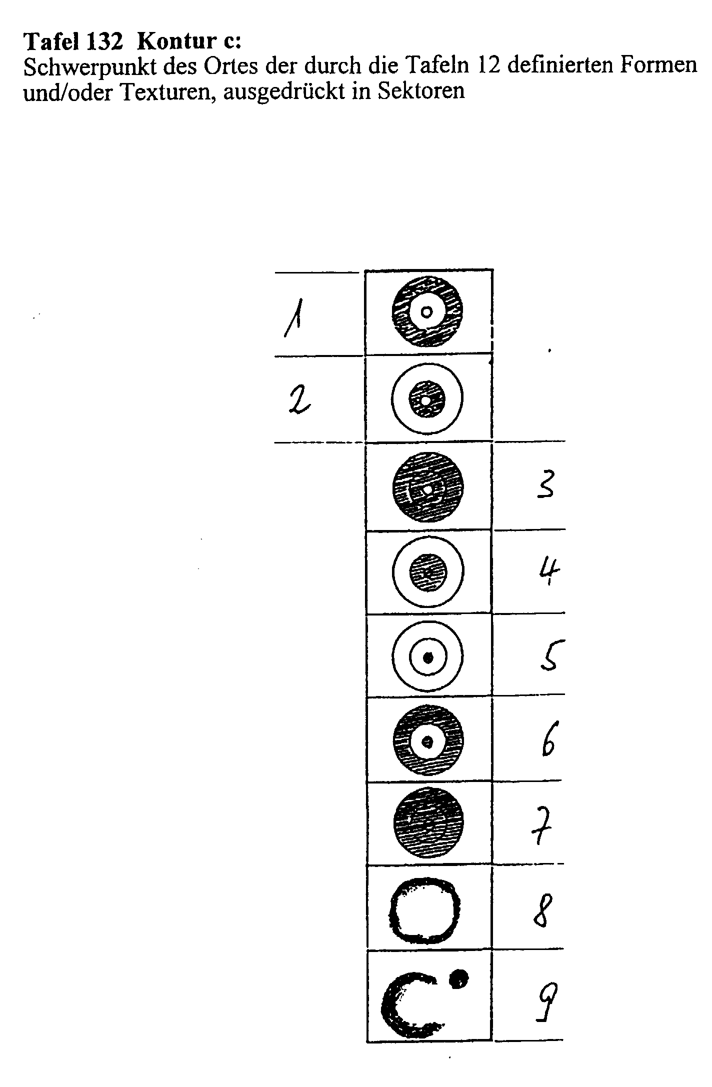

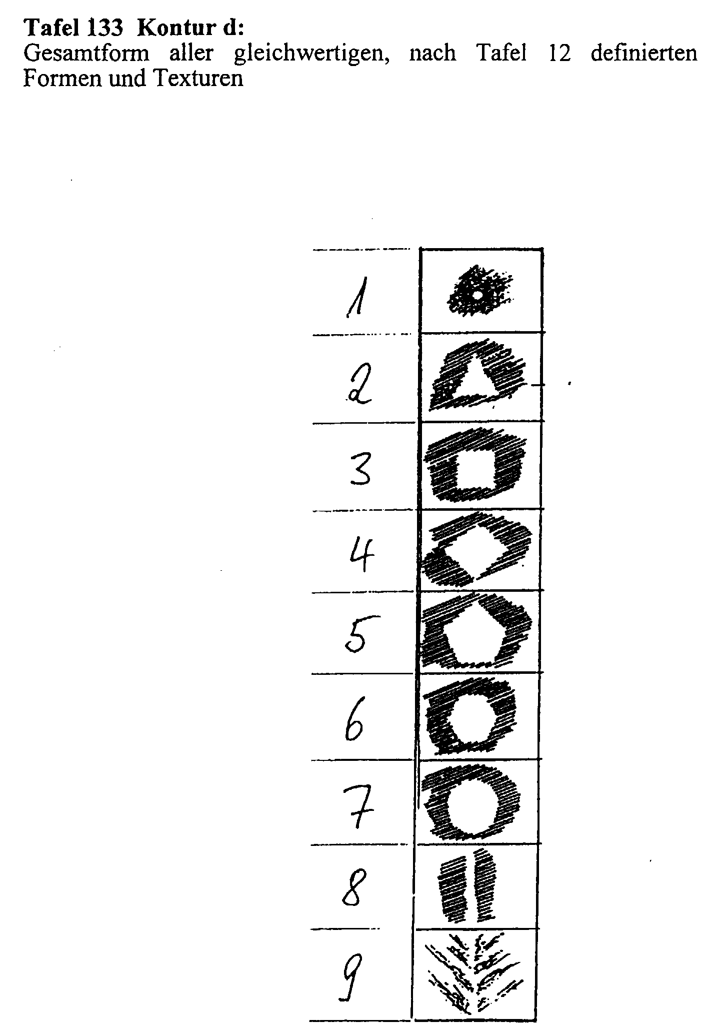

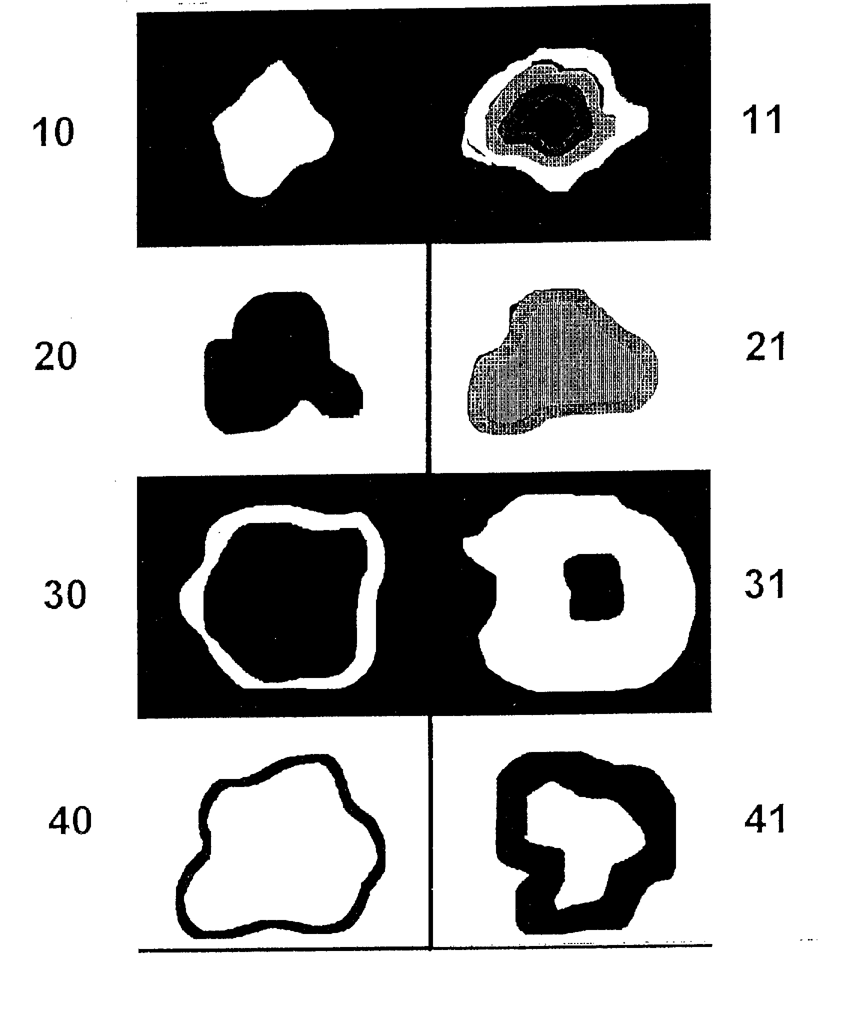

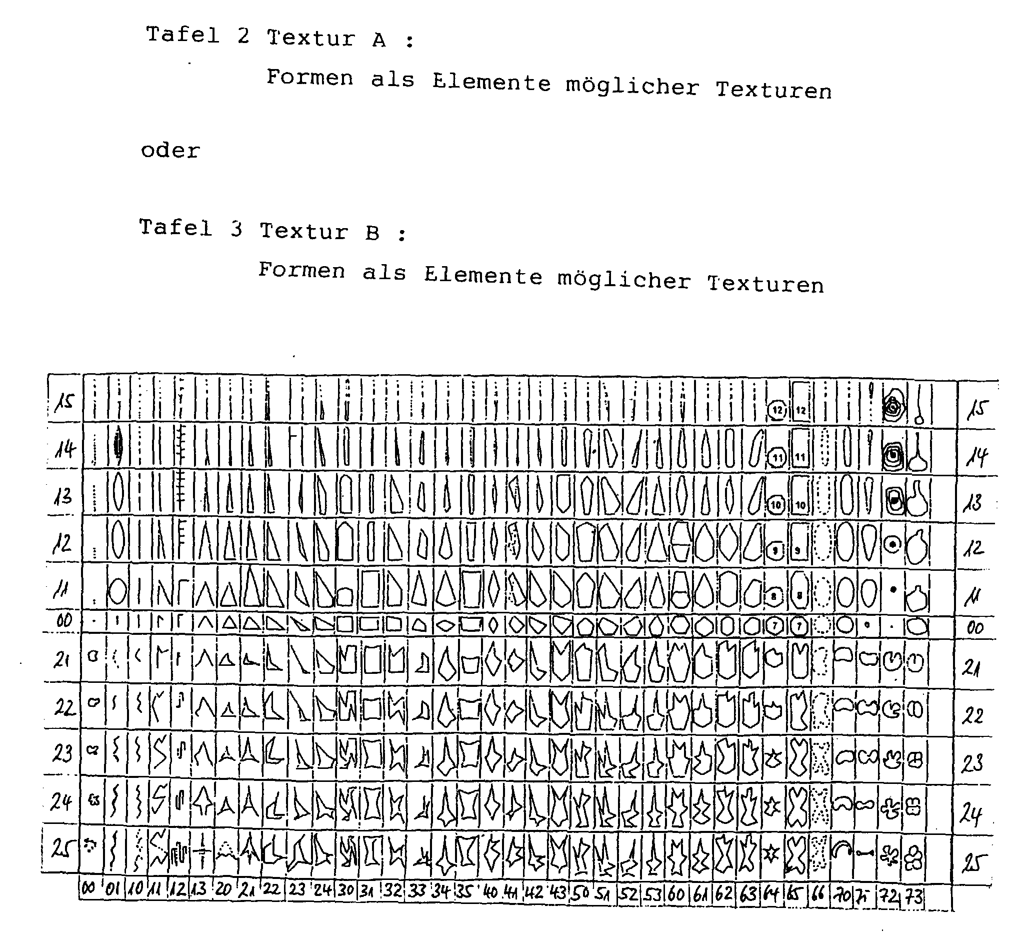

### Tafel 21 Präfix A zu den Formen der Tafel 2, Textur A

Verhältnis der Textur a aus Tafel 2 zur Form aus Tafel 1
21-1 Die Textur aus Tafel 2 wird der Form aus Tafel 1 auf gleicher Ebene hinzugefügt
21-2 Die Textur aus Tafel 2 wird der Form aus Tafel 1 auf unterschiedlicher Ebene hinzugefügt
21-3 Die Textur aus Tafel 2 durchdringt die Form aus Tafel 1 in gleicher Ebene
21-4 Die Textur aus Tafel 2 bildet eine Binnenform in der Form aus Tafel 1
21-5 Die Textur aus Tafel 2 liegt zwischen Teilen der Form aus Tafel 1
21-6 Die Textur aus Tafel 2 lagert sich an die Form aus Tafel 1 an
21-7 Die Textur aus Tafel 2 bildet eine Binnenform in mehreren gleochen Formen aus Tafel 1
21-8 Die Textur aus Tafel 2 durchdringt die Form aus Tafel 1 auf verschiedenen Ebenen
21-9 Die Textur aus Tafel 2 schichtet sich mehrfach über die Form aus Tafel 1
oder

### Tafel 23 Postfix A zu den Formen der Tafel 2, Textur A

23 nn Eine Zahl zwischen 0 und 98 gibt die Anzahl an, ausgenommen sind die Zahlwerte 15, 30, 45, 60, 75, 90 and 99
23-15 Die Zahl 15 gibt den Winkel von vorzugsweise 15 Grad an.
23-30 Die Zahl 30 gibt den Winkel von vorzugsweise 30 Grad an.
23-45 Die Zahl 45 gibt den Winkel von vorzugsweise 45 Grad an.
23-60 Die Zahl 60 gibt den Winkel von vorzugsweise 60 Grad an.
23-75 Die Zahl 75 gibt den Winkel von vorzugsweise 75 Grad an.
23-90 Die Zahl 90 gibt den Winkel von vorzugsweise 90 Grad an.
23-99 Die Zahl 99 gibt eine unbestimmte Anzahl über 98 an

### Tafel 31 Präfix B zu den Formen der Tafel 3, Textur B

Anzahl der Formen aus Tafel 2 und/oder ihre Winkelposition zur Form aus Tafel 1
31-nn Die Zahl mm zwischen 0 und 98 gibt die Anzahl der zählbaren Texturen an, ausgenommen sind die Zahlwerte 15, 30, 45, 60, 75, 90 und 99
31-15 Die Zahl 15 gibt den Winkel an, in dem sich die Textur a: aus Tafel 2 zu der Form aus Tafel 1 befindet
31-30 Die Zahl 30 gibt den Winkel an, in dem sich Textur a: aus Tafel 2 zu der Form aus Tafel 1 befindet
31-45 Die Zahl 45 gibt den Winkel an, in dem sich die Textur a: aus Tafel 2 zu der Form aus Tafel 1 befindet
31-60 Die Zahl 60 gibt den Winkel an, in dem sich die Textur a: aus Tafel 2 zu der Form aus Tafel 1 befindet
31-75 Die Zahl 75 gibt den Winkel an, in dem sich die Textur a: aus Tafel 2 zu der Form aus Tafel 1 befindet
31-90 Die Zahl 90 gibt den Winkel an, in dem sich die Textur a: aus Tafel 2 zu der Form aus Tafel 1 befindet
31-99 Diese Zahl gibt eine unbestimmte Anzahl über 98 an
oder

### Tafel 33 Postfix B zu den Formen der Tafel 3, Textur B

33-nn Eine Zahl zwischen 0 und 98 gibt die Anzahl an, ausgenommen sind die Zahlwerte 15, 30, 45, 60, 75, 90 und 99
33-15 Die Zahl 15 gibt den Winkel von vorzugsweise 15 Grad an.
33-30 Die Zahl 30 gibt den Winkel von vorzugsweise 30 Grad an.
33-45 Die Zahl 45 gibt den Winkel von vorzugsweise 45 Grad an.
33-60 Die Zahl 60 gibt den Winkel von vorzugsweise 60 Grad an.
33-75 Die Zahl 75 gibt den Winkel von vorzugsweise 75 Grad an.
33-90 Die Zahl 90 gibt den Winkel von vorzugsweise 90 Grad an.
33-99 Die Zahl 99 gibt eine unbestimmte Anzahl über 98 an

### Tafel 14 Lumen a:

Abbildungsverhältnisse der Form und/oder Texturen aus Tafel 1 gegenüber ihren Unter- und Hintergründen
oder

### Tafel 22 Lumen b:

Abbildungverhältnisse der Textur A aus Tafel 2 gegenuber ihren Unter- und Hintergründen
oder

### Tafel 32 Lumen c:

Abbildungsverhältnisse der Textur B aus Tafel 3 gegenüber ihren Unter - und Hintergründen

Die Tafel beansprucht 8 Felder unterschiedlicher Flächen - und Raumkonstellation, die während einer Kristallisation und bei der Ausbildung von kristalinen Formen und Texturen der Tafel 3 entstehen:
Feld 10: Helle, kleine, formbeliebige Struktur auf größerer; dunkler, formbeliebiger Struktur
Feld 11: Helle, große, formbeliebige Struktur auf schmalerer, dunkler, formbeliebiger Struktur
Feld 20: Hellere, formbeliebige Struktur auf hellerer, größerer und formbeliebiger Struktur, wobei diese sich vor einem dunkleren Hintergrund darstellt
Feld 21: Dunkle, formbeliebige Struktur auf hellerer, größerer und formbeliebiger Struktur, wobei diese sich vor einem dunkleren Hinkergrund darstellt
Feld 30: Gegenüber dem Hintergrund als leicht dunkler differenzierte Struktur
Feld 31: Gegenüber dem Hintergrund als deutlich dunkler differenzierte Struktur
Feld 40: Helle, plastiche Struktur mit deutlichen Grenzen auf einer ebenso hellen, plastischen Struktur mit deutlichen Grenzen in einer formbeliebigen, hellen Struktur, die auf einem dunkleren Hintergrund liegt
Feld 41: Helle, große und formbeliebige Struktur auf einem dunklen Hintergrund.
Auflistung der Formen und/oder Texturen :

### Tafel 1Form Formen als Elemente möglicher Texturen

Die Abszisse der Tafel beginnt bei 1-00 und endet bei 1-73.
Die Ordinate hat einen positiven Teil von 1-11 - 1-15 und einen negativen von 1-21 - 1-25.
Die positive Ordinate stellt Stufen der Komplizierung (Formen der Abszisse 00-20) oder der vektoriellen Ausdehnung (Exduktion) einer Form dar:
Die negative Ordinate stellt Stufen der vektoriellen Eindellung (Induktion) einer Form dar:
Auf der Abszisse werden Stadien geometrische Grundformen dargestellt, beginnend mit dem Punkt, über die Linie, zum Winkel, Dreieck, Viereck, zur Raute, zum Trapez, Fünfeck, Sechseck, Vieleck und zum Kreis. Die Stufen zwischen den regelmäßig symmetrischen Formen werden durch asymmetrische Ableitungen besetzt. Die einzelnen Stufen der Abszisse sind:

### Abszisse 1-00: Punkt

Ordinate 1-11: In einer Linie verdoppelter Punkt
Ordinate 1-12: In einer Linie vervielfacher Punkt
Ordinate 1-13: Zwei, in einer Linie liegende Punktgruppen
Ordinate 1-14: Lange, regelmäßige Punktgruppe
Ordinate 1-15: Lange, unregelmäßige Punktgruppe
Ordinate 1-21: Vergrößerter Punkt mit einer Einkerbung (Induktion)
Ordinate 1-22: Vergrößerter Punkt mit zwei Induktionen
Ordinate 1-23: Vergrößerter Punkt mit drei Induktionen
Ordinate 1-24: Vergrößerter Punkt mit vier Induktionen
Ordinate 1-25: Vergrößerter Punkt mit fünf und mehr Induktionen.

### Abszisse 1-01: in Längsrichtung und ansatzweise in Querrichtung ausgedehnter Punkt

Ordinate 1-11: Ausdehnung des Punktes zu einen symmetrischeh Oval
Ordinate 1-12: Längsausdehnung des Punktes zu einen symmetrischen Oval
Ordinate 1-13: Weitere Längsausdehnung des Punktes zu einem symmetrischen Oval
Ordinate 1-14: Auflösung der korpuskulären Struktur des Oval
Ordinate 1-15: Unregelmäßige Längsausdehung des Ovals zur räumlichen Linie
Ordinate 1-21: Einfache Induktion der Linie (Bogen)
Ordinate 1-22: Zweifache Induktion der Linie (Schlange)
Ordinate 1-23: Dreifache Induktion der Linie (Zickzack)
Ordinate 1-24: Vierfache Induktion der Linie mit leichter Glättung der Ecken
Ordinate 1-25: Fünf- und mehrfache Induktion der Linie mit Glättung der Ecken

### Abszisse 1-10: in Längsrichtung ausgedehnter Punkt: Linie

Ordinate 1-11: Gerade
Ordinate 1-12: Einfach unterbrochene Gerade
Ordinate 1-13: Mehrfach unterbrochene Gerade aus regelmäßigen Geraderesten
Ordinate 1-14: Mehrfach unterbrochene Gerade aus unregelmäßigen Geraderesten
Ordinate 1-15: Gebrochene Gerade aus vielfachen, unregelmäßigen Geraderesten
Ordinate 1-21: Gerade mit einer winkeligen Induktion
Ordinate 1-22: Gerade mit zwei winkeligen Induktionen
Ordinate 1-23: Gerade mit drei und mehr winkeligen Induktionens
Ordinate 1-24: Gerade mit vier und mehr winkeligen Induktionen mit Überstand am Scheitel
Ordinate 1-25: Auflösende Gerade mit fünf und mehr winkeligen Induktionen

### Abszisse 1-11: Linie mit freiwinkeliger Abzweigung

Ordinate 1-11: Gerade mit spitzwinkeliger Abzweigung
Ordinate 1-12: Gerade mit sehr spitzwinkeliger Abzweigung
Ordinate 1-13: Gerade mit extrem spitzwinkeliger Abzweigung
Ordinate 1-14: Gerade mit in die Gerade einlaufender Abzweigung
Ordinate 1-15: Unregelmäßig ausgedehnte Gerade mit in sie einlaufender Abzweigung
Ordinate 1-21: Gerade mit spitzwinkeliger Abzweigung und einer Induktion
Ordinate 1-22: Gerade mit spitzwinkeliger Abzweigung und zwei Induktionen
Ordinate 1-23: Gerade mit spitzwinkeliger Abzweigung und drei Induktionen
Ordinate 1-24: Gerade mit spitzwinkeliger Abzweigung und vier Induktionen
Ordinate 1-25: Gerade mit spitzwinkeliger Abzweigung und fünf oder mehr Induktionen

### Abszisse 1-12: Linie mit rechtwinkeliger Abzweigung

Ordinate 1-11: Gerade mit einer rechtwinkeligen, längeren Abzweigung
Ordinate 1-12: Gerade mit mehreren, rechtwinkeligen Abzweigungen
Ordinate 1-13: Gerade mit mehreren, rechtwinkeligen, gegenständigen Abzweigungen
Ordinate 1-14: Gerade mit mehreren, rechtwinkeligen, wechselständigen Abzweigungen
Ordinate 1-15: Gerade mit mehreren, rechtwinkeligen, unterschiedlichständigen Abzweigungen
Ordinate 1-21: Gerade mit zwei rechtwinkligen Brechungen
Ordinate 1-22: Gerade mit drei rechtwinkeligen Brechungen
Ordinate 1-23: Gerade mit vier rechtwinkligen Brechungen
Ordinate 1-24: Gerade mit fünf und mehr rechtwinkligen Brechungen
Ordinate 1-25: Gerade mit beliebig vielen rechtwinkligen Brechungen

### Abszisse 1-13: Linie mit gleichschenkliger Abzweigung

Ordinate 1-11: Symmetrische Winkelgerade mit großem Winkel
Ordinate 1-12: Symmetrische Winkelgerade mit kleinem Winkel
Ordinate 1-13: Symmetrische Winkelgerade mit sehr kleinem Winkel
Ordinate 1-14: Symmetrische Winkelgerade mit extrem kleinem Winkel
Ordinate 1-15: Symmetrische, unregelmäßig verlängerte Winkelgerade mit minimalen Winkel
Ordinate 1-21: Symmetrische Winkelgerade mit einer Induktion
Ordinate 1-22: Symmetrische Winkelgerade mit zwei Induktionen
Ordinate 1-23: Symmetrische Winkelgerade mit drei Induktionen
Ordinate 1-24: Symmetrische Winkelgelade mit vier, symmetrischen Induktionen
Ordinate 1-25: Symmetrische Winkelgelade mit vier, symmetrisch reduzierenden Induktionen

### Abszisse 1-20: Gleichseiteiges Dreieck

Ordinate 1-11: Gleichseitiges Dreieck mit leichter Verlängerung der senkrechten Symmetrieachse
Ordinate 1-12: Gleichseitiges Dreieck mit Verlängerung der senkrechten Symmetrieachse
Ordinate 1-13: Gleichseitiges Dreieck mit starker Verlängerung der senkrechten Symmetrieachse
Ordinate 1-14: Gleichseitiges Dreieck mit extremer Verlängerung der Symmetrieachse
Ordinate 1-15: Gleichseitiges Dreieck mit unregelmäßiger Verlängerung der Symmetrieachse
Ordinate 1-21: Gleichseitiges Dreieck mit einer Induktion
Ordinate 1-22: Gleichseitiges Dreieck mit zwei Induktionen
Ordinate 1-23: Gleichseitiges Dreieck mit drei Induktionen
Ordinate 1-24: Gleichseitiges Dreieck mit vier Induktionen
Ordinate 1-25: Gleichseitiges Dreieck mit fünf und mehr Induktionen

### Abszisse 1-21: Ungleichseitiges Dreieck

Ordinate 1-11: Ungleichseitiges Dreieck mit leichter Verlängerung der senkrechten Achse
Ordinate 1-12: Ungleichseitiges Dreieck mit Verlängerung der senkrechten Achse
Ordinate 1-13: Ungleichseitiges Dreieck mit starker Verlängerung der senkrechten Achse
Ordinate 1-14: Ungleichseitiges Dreieck mit extremer Verlängerung der Achse
Ordinate 1-15: Ungleichseitiges Dreieck mit unregelmäßiger Verlängerung der Achse
Ordinate 1-21: Ungleichseitiges Dreieck mit einer Induktion
Ordinate 1-22: Ungleichseitiges Dreieck mit zwei Induktionen
Ordinate 1-23: Ungleichseitiges Dreieck mit drei Induktionen
Ordinate 1-24: Ungleichseitiges Dreieck mit vier Induktionen Ordinate 1-25: Ungleichseitiges Dreieck mit fünf und mehr Induktionen

### Abszisse 1-22: Rechtwinkeliges Dreieck

Ordinate 1-11: Rechtwinkeliges Dreieck mit leichter Verlängerung der senkrechten Achse
Ordinate 1-12: Rechtwinkeliges Dreieck mit Verlängerung der senkrechten Achse
Ordinate 1-13: Rechtwinkeliges Dreieck mit starker Verlängerung der senktrechten Achse
Ordinate 1-14: Rechtwinkeliges Dreieck mit extremer Verlängerung der Achse
Ordinate 1-15: Rechtwinkeliges Dreieck mit unregelmäßiger Verlängerung der Achse
Ordinate 1-21: Rechtwinkeliges Dreieck mit einer Induktion
Ordinate 1-22: Rechtwinkeliges Dreieck mit zwei Induktionen
Ordinate 1-23: Rechtwinkeliges Dreieck mit drei Induktionen
Ordinate 1-24: Rechtwinkeliges Dreieck mit vier Induktionen
Ordinate 1-25: Rechtwinkeliges Dreieck mit fünf und mehr Induktionen

### Abszisse 1-23: Spitzwinkeliges Dreieck

Ordinate 1-11: Spitzwinkeliges Dreieck mit leichter Verlängerung der senkrechten Achse
Ordinate 1-12: Spitzwinkeliges Dreieck mit Verlängerung der senkrechten Achse
Ordinate 1-13: Spitzwinkeliges Dreieck mit starker Verlängerung der senkrechten Achse
Ordinate 1-14: Spitzwinkeliges Dreieck mit extremer Verlängerung der Achse
Ordinate 1-15: Spitzwinkeliges Dreieck mit unregelmäßiger Verlängerung der Achse
Ordinate 1-21:Spitzwinkeliges Dreieck mit einer Induktion
Ordinate 1-22: Spitzwinkeliges Dreieck mit zwei Induktionen
Ordinate 1-23: Spitzwinkeliges Dreieck mit drei Induktionen
Ordinate 1-24: Spitzwinkeliges Dreieck mit vier Induktionen
Ordinate 1-25: Spitzwinkeliges Dreieck mit fünf und mehr Induktionen

### Abszisse 1-24: Spitzwinkeliges Dreieck mit einer gespreizten Ecke zum beginnenden Viereck

Ordinate 1-11: Spitzwinkeliges Dreieck mit leichter Verlängerung der senktrechten Achse
Ordinate 1-12: Spitzwinkeliges Dreieck mit Verlängerung der senkrechten Achse
Ordinate 1-13: Spitzwinkeliges Dreieck mit starker Verlängerung der senktrechten Achse
Ordinate 1-14: Spitzwinkeliges Dreieck mit extremer Verlängerung der Achse
Ordinate 1-15: Spitzwinkeliges Dreieck mit unregelmäßiger Verlängerung der Achse
Ordinate 1-21: Spitzwinkeliges Dreieck mit einer Induktion
Ordinate 1-22: Spitzwinkeliges Dreieck mit zwei Induktionen
Ordinate 1-23: Spitzwinkeliges Dreieck mit drei Induktionen
Ordinate 1-24: Spitzwinkeliges Dreieck mit vier Induktionen
Ordinate 1-25: Spitzwinkeliges Dreieck mit fünf und mehr Induktionen

### Abszisse 1-30: Gleichseitiges Viereck, Quadrat

Ordinate 1-11: Gleichseitiges Viereck mit leichter Verlängerung der senkrechten Symmetrieachse
Ordinate 1-12: Gleichseitiges Viereck mit Verlängerung der senkrechten Symmetrieachse
Ordinate 1-13: Gleichseitiges Viereck mit starker Verlängerung der senkrechten Symmetrieachse
Ordinate 1-14: Gleichseitiges Viereck mit extremer Verlängerung der Symmetrieachse
Ordinate 1-15: Gleichseitiges Viereck mit unregelmäßiger Verlängerung der Symmetrieachse
Ordinate 1-21: Gleichseitiges Viereck mit einer Induktion
Ordinate 1-22: Gleichseitiges Viereck mit zwei Induktionen
Ordinate 1-23: Gleichseitiges Viereck mit drei Induktionen
Ordinate 1-24: Gleichseitiges Viereck mit vier Induktionen
Ordinate 1-25: Gleichseitiges Viereck mit fünf und mehr Induktionen

### Abszisse 1-31: Regelmäßiges, symmetrisches Viereck, Rechteck

Ordinate 1-11: Regelmäßiges Viereck mit leichter Verlängerung der senktrechten Symmetrieachse
Ordinate 1-12: Regelmäßiges Viereck mit Verlängerung der senkrechten Symmetrieachse
Ordinate 1-13: Regelmäßiges Viereck mit starker Verlängerung der senkrechten Symmetrieachse
Ordinate 1-14: Regelmäßiges Viereck mit extremer Verlängerung der Symmetrieachse
Ordinate 1-15: Regelmäßiges Viereck mit unregelmäßiger Verlängerung der Symmetrieachse
Ordinate 1-21: Regelmäßiges Viereck mit einer Induktion
Ordinate 1-22: Regelmäßiges Viereck mit zwei Induktionen
Ordinate 1-23: Regelmäßiges Viereck mit drei Induktionen
Ordinate 1-24: Regelmäßiges Viereck mit vier Induktionen
Ordinate 1-25: Regelmäßiges Viereck mit fünf mehr Induktionen

### Abszisse 1-32: Unsymmetrisches Viereck

Ordinate 1-11: Unsymmetrisches Viereck mit leichter Verlängerung der senkrechten Symmetrieachse
Ordinate 1-12: Unsymmetrisches Viereck mit Verlängerung der senkrechten Symmetrieachse
Ordinate 1-13: Unsymmetrisches Viereck mit starker Verlängerung der senkrechten Symmetrieachse
Ordinate 1-14: Unsymmetrisches Viereck mit extremer Verlängerung der Symmetrieachse
Ordinate 1-15: Unsymmetrisches Viereck mit unregelmäßiger Verlängerung der Symmetrieachse
Ordinate 1-21: Unsymmetrisches Viereck mit einer Induktion
Ordinate 1-22: Unsymmetrisches Viereck mit zwei Induktionen
Ordinate 1-23: Unsymmetrisches Viereck mit drei Induktionen
Ordinate 1-24: Unsymmetrisches Viereck mit vier Induktionens
Ordinate 1-25: Unsymmetrisches Viereck mit fünf und mehr Induktionen

### Abszisse 1-33: Stark unsymmetrisches Viereck

Ordinate 1-11: Stark unsymmetrisches Viereck mit leichter Verlängerung der senkrechten Symmetrieachse
Ordinate 1-12: Stark unsymmetrisches Viereck mit Verlängerung der senkrechten Symmetrieachse
Ordinate 1-13: Stark unsymmetrisches Viereck mit starker Verlängerung der senkrechten Symmetrieachse
Ordinate 1-14: Stark unsymmetrisches Viereck mit extremer Verlängerung der Symmetrieachse
Ordinate 1-15: Stark unsymmetrisches Viereck mit Stark unregelmäßiger Verlängerung der Symmetrieachse
Ordinate 1-21: Stark unsymmetrisches Viereck mit einer Induktion
Ordinate 1-22: Stark unsymmetrisches Viereck mit zwei Induktionen
Ordinate 1-23: Stark unsymmetrisches Viereck mit drei Induktionen
Ordinate 1-24: Stark unsymmetrisches Viereck mit vier Induktionen
Ordinate 1-25: Stark unsymmetrisches Viereck mit fünf Stark und mehr Induktionen

### Abszisse 1-34: Liegende Raute

Ordinate 1-11: Liegende Raute mit leichter Verlängerung der senkrechten Symmetrieachse
Ordinate 1-12: Liegende Raute mit Verlängerung der senkrechten Symmetrieachse
Ordinate 1-13: Liegende Raute mit starker Verlängerung der senkrechten Symmetrieachse
Ordinate 1-14: Liegende Raute mit extremer Verlängerung der Symmetrieachse
Ordinate 1-15: Liegende Raute mit unregelmäßiger Verlängerung der Symmetrieachse
Ordinate 1-21: Liegende Raute mit einer Induktion
Ordinate 1-22: Liegende Raute mit zwei Induktionen
Ordinate 1-23: Liegende Raute mit drei Induktionen
Ordinate 1-24: Liegende Raute mit vier Induktionen
Ordinate 1-25: Liegende Raute mit fünf und mehr Induktionen

### Abszisse 1-35: Trapez

Ordinate 1-11: Trapez mit leichter Verlängerung der senkrechten Symmetrieachse
Ordinate 1-12: Trapez mit Verlängerung der senkrechten Symmetrieachse
Ordinate 1-13: Trapez mit starker Verlängerung der senkrechten Symmetrieachse
Ordinate 1-14: Trapez mit extremer Verlängerung der Symmetrieachse
Ordinate 1-15: Trapez mit unregelmäßiger Verlängerung der Symmetrieachse
Ordinate 1-21: Trapez mit einer Induktion
Ordinate 1-22: Trapez mit zwei Induktionen
Ordinate 1-23: Trapez mit drei Induktionen
Ordinate 1-24: Trapez mit vier Induktionen
Ordinate 1-25: Trapez mit fünf und mehr Induktionen

### Abszisse 1-40: Stehende Raute

Ordinate 1-11: Stehende Raute mit leichter Verlängerung der senkrechten Symmetrieachse
Ordinate 1-12: Stehende Raute mit Verlängerung der senkrechten Symmetrieachse
Ordinate 1-13: Stehende Raute mit starker Verlängerung der senkrechten Symmetrieachse
Ordinate 1-14: Stehende Raute mit extremer Verlängerung der Symmetrieachse
Ordinate 1-15: Stehende Raute mit unregelmäßiger Verlängerung der Symmetrieachse
Ordinate 1-21: Stehende Raute mit einer Induktion
Ordinate 1-22: Stehende Raute mit zwei Induktionen
Ordinate 1-23: Stehende Raute mit drei Induktionen
Ordinate 1-24: Stehende Raute mit vier Induktionen
Ordinate 1-25: Stehende Raute mit fünf und mehr Induktionen

### Abszisse 1-41: Asymmetrische, stehende Raute

Ordinate 1-11: Asymmetrisch stehende Raute mit leichter Verlängerung der senkrechten Achse
Ordinate 1-12: Asymmetrisch stehende Raute mit Verlängerung der senkrechten Achse
Ordinate 1-13: Asymmetrisch stehende Raute mit starker Verlängerung der senkrechten Achse
Ordinate 1-14: Asymmetrisch stehende Raute mit extremer Verlängerung der Achse
Ordinate 1-15: Asymmetrisch stehende Raute mit unregelmäßiger Verlängerung der Achse
Ordinate 1-21: Asymmetrisch stehende Raute mit einer Induktion
Ordinate 1-22: Asymmetrisch stehende Raute mit zwei Induktionen
Ordinate 1-23: Asymmetrisch stehende Raute mit drei Induktionen
Ordinate 1-24: Asymmetrisch stehende Raute mit vier Induktionen
Ordinate 1-25: Asymmetrisch stehende Raute mit fünf und mehr Induktionen

### Abszisse 1-42: Stark asymmetrische, stehende Raute

Ordinate 1-11: Stark asymmetrisch stehende Raute mit leichter Verlängerung der senkrechten Achse
Ordinate 1-12: Stark asymmetrisch stehende Raute mit Verlängerung der senkrechten Achse
Ordinate 1-13: Stark asymmetrisch stehende Raute mit starker Verlängerung der senkrechten Achse
Ordinate 1-14: Stark asymmetrisch stehende Raute mit extremer Verlängerung der Achse
Ordinate 1-15: Stark asymmetrisch stehende Raute mit unregelmäßiger Verlängerung der Achse
Ordinate 1-21: Stark asymmetrisch stehende Raute mit einer Induktion
Ordinate 1-22: Stark asymmetrisch stehende Raute mit zwei Induktionen
Ordinate 1-23: Stark asymmetrisch stehende Raute mit drei Induktionen
Ordinate 1-24: Stark asymmetrisch stehende Raute mit vier Induktionen
Ordinate 1-25: Stark asymmetrisch stehende Raute mit fünf und mehr Induktionen

### Abszisse 1-43: Stark asymmetrische, stehende Raute mit einer gespreizten Ecke zum Fünfeck

Ordinate 1-11: Stark asymmetrisch stehende Raute mit einer gespreizten Ecke zum Fünfeck und mit leichter Verlängerung der senkrechten Achse
Ordinate 1-12: Stark asymmetrisch stehende Raute mit einer gespreizten Ecke zum Fünfeck und mit Verlängerung der senkrechten Achse
Ordinate 1-13: Stark asymmetrisch stehende Raute mit einer gespreizten Ecke zum Fünfeck und mit starker Verlängerung der senkrechten Achse
Ordinate 1-14: Stark asymmetrisch stehende Raute mit einer gespreizten Ecke zum Fünfeck und mit extremer Verlängerung der Achse
Ordinate 1-15: Stark asymmetrisch stehende Raute mit einer gespreizten Ecke zum Fünfeck und mit unregelmäßiger Verlängerung der Achse
Ordinate 1-21: Stark asymmetrisch stehende Raute mit einer gespreizten Ecke zum Fünfeck und mit einer Induktion
Ordinate 1-22: Stark asymmetrisch stehende Raute mit einer gespreizten Ecke zum Fünfeck und mit zwei Induktionen
Ordinate 1-23: Stark asymmetrisch stehende Raute mit einer gespreizten Ecke zum Fünfeck und mit drei Induktionen
Ordinate 1-24: Stark asymmetrisch stehende Raute mit einer gespreizten Ecke zum Fünfeck und mit vier Induktionen
Ordinate 1-25: Stark asymmetrisch stehende Raute mit einer gespreizten Ecke zum Fünfeck und mit fünf und mehr Induktionen

### Abszisse 1-50: Gleichseitiges Fünfeck

Ordinate 1-11: Gleichseitiges Fünfeck mit leichter Verlängerung der senkrechten Symmetrieachse
Ordinate 1-12: Gleichseitiges Fünfeck mit Verlängerung der senkrechten Symmetrieachse
Ordinate 1-13: Gleichseitiges Fünfeck mit starker Verlängerung der senkrechten Symmetrieachse
Ordinate 1-14: Gleichseitiges Fünfeck mit extremer Verlängerung der Symmetrieachse
Ordinate 1-15: Gleichseitiges Fünfeck mit unregelmäßiger Verlängerung der Symmetrieachse
Ordinate 1-21: Gleichseitiges Fünfeck mit einer Induktion
Ordinate 1-22: Gleichseitiges Fünfeck mit zwei Induktionen
Ordinate 1-23: Gleichseitiges Fünfeck mit drei Induktionen
Ordinate 1-24: Gleichseitiges Fünfeck mit vier Induktionen
Ordinate 1-25: Gleichseitiges Fünfeck mit fünf und mehr Induktionen

### Abszisse 1-51: Asymmetrisches Fünfeck

Ordinate 1-11: Asymmetrisches Fünfeck mit leichter Verlängerung der senkrechten Achse
Ordinate 1-12: Asymmetrisches Fünfeck mit Verlängerung der senkrechten Achse
Ordinate 1-13: Asymmetrisches Fünfeck mit starker Verlängerung der senkrechten Achse
Ordinate 1-14: Asymmetrisches Fünfeck mit extremer Verlängerung der Achse
Ordinate 1-15: Asymmetrisches Fünfeck mit unregelmäßiger Verlängerung der Achse
Ordinate 1-21: Asymmetrisches Fünfeck mit einer Induktion
Ordinate 1-22: Asymmetrisches Fünfeck mit zwei Induktionen
Ordinate 1-23: Asymmetrisches Fünfeck mit drei Induktionen
Ordinate 1-24: Asymmetrisches Fünfeck mit vier Induktionen
Ordinate 1-25: Asymmetrisches Fünfeck mit fünf und mehr Induktionen

### Abszisse 1-52: Stark asymmetrisches Fünfeck

Ordinate 1-11: Stark asymmetrisches Fünfeck mit leichter Verlängerung der senkrechten Symmetrieachse
Ordinate 1-12: Stark asymmetrisches Fünfeck mit Verlängerung der senkrechten Symmetrieachse
Ordinate 1-13: Stark asymmetrisches Fünfeck mit starker Verlängerung der senkrechten Symmetrieachse
Ordinate 1-14: Stark asymmetrisches Fünfeck mit extremer Verlängerung der Symmetrieachse
Ordinate 1-15: Stark asymmetrisches Fünfeck mit unregelmäßiger Verlängerung der Symmetrieachse
Ordinate 1-21: Stark asymmetrisches Fünfeck mit einer Induktion
Ordinate 1-22: Stark asymmetrisches Fünfeck mit zwei Induktionen
Ordinate 1-23: Stark asymmetrisches Fünfeck mit drei Induktionen
Ordinate 1-24: Stark asymmetrisches Fünfeck mit vier Induktionen
Ordinate 1-25: Stark asymmetrisches Fünfeck mit fünf und mehr Induktionen

### Abszisse 1-53: Einfach symmetrisches Fünfeck

Ordinate 1-11: Einfach symmetrisches Fünfeck mit leichter Verlängerung der senkrechten Symmetrieachse
Ordinate 1-12: Einfach symmetrisches Fünfeck mit Verlängerung der senkrechten Symmetrieachse
Ordinate 1-13: Einfach symmetrisches Fünfeck mit starker Verlängerung der senkrechten Symmetrieachse
Ordinate 1-14: Einfach symmetrisches Fünfeck mit extremer Verlängerung der Symmetrieachse
Ordinate 1-15: Einfach symmetrisches Fünfeck mit unregelmäßiger Verlängerung der Symmetrieachse
Ordinate 1-21: Einfach symmetrisches Fünfeck mit einer Induktion
Ordinate 1-22: Einfach symmetrisches Fünfeck mit zwei Induktionen
Ordinate 1-23: Einfach symmetrisches Fünfeck mit drei Induktionen
Ordinate 1-24: Einfach symmetrisches Fünfeck mit vier Induktionen
Ordinate 1-25: Einfach symmetrisches Fünfeck mit fünf und mehr Induktionen

### Abszisse 1-60: Gleichseitiges Sechseck

Ordinate 1-11: Gleichseitiges Sechseck mit leichter Verlängerung der senkrechten Symmetrieachse
Ordinate 1-12: Gleichseitiges Sechseck mit Verlängerung der senkrechten Symmetrieachse
Ordinate 1-13: Gleichseitiges Sechseck mit starker Verlängerung der senkrechten Symmetrieachse
Ordinate 1-14: Gleichseitiges Sechseck mit extremer Verlängerung der Symmetrieachse
Ordinate 1-15: Gleichseitiges Sechseck mit unregelmäßiger Verlängerung der Symmetrieachse
Ordinate 1-21: Gleichseitiges Sechseck mit einer Induktion
Ordinate 1-22: Gleichseitiges Sechseck mit zwei Induktionen
Ordinate 1-23: Gleichseitiges Sechseck mit drei Induktionen
Ordinate 1-24: Gleichseitiges Sechseck mit vier Induktionen
Ordinate 1-25: Gleichseitiges Sechseck mit fünf und mehr Induktionen

### Abszisse 1-61: Doppelt symmetrisches Sechseck

Ordinate 1-11: Doppelt symmetrisches Seckseck mit leichter Verlängerung der senkrechten Symmetrieachse
Ordinate 1-12: Doppelt symmetrisches Sechseck mit Verlängerung der senkrechten Symmetrieachse
Ordinate 1-13: Doppelt symmetrisches Sechseck mit starker Verlängerung der senkrechten Symmetrieachse
Ordinate 1-14: Doppelt symmetrisches Sechseck mit extremer Verlängerung der Symmetrieachse
Ordinate 1-15: Doppelt symmetrisches Sechseck mit unregelmäßiger Verlängerung der Symmetrieachse
Ordinate 1-21: Doppelt symmetrisches Sechseck mit einer Induktion
Ordinate 1-22: Doppelt symmetrisches Sechseck mit zwei Induktionen
Ordinate 1-23: Doppelt symmetrisches Sechseck mit drei Induktionen
Ordinate 1-24: Doppelt symmetrisches Sechseck mit vier Induktionen
Ordinate 1-25: Doppelt symmetrisches Sechseck mit fünf und mehr Induktionen

### Abszisse 1-62: Doppelt symmetrisches Sechseck mit verlängerter Längsachse

Ordinate 1-11: Doppelt symmetrisches Sechseck mit verlängerter Längsachse und mit leichter Verlängerung der senkrechten Symmetrieachse
Ordinate 1-12: Doppelt symmetrisches Sechseck mit verlängerter Längsachse und mit Verlängerung der senkrechten Symmetrieachse
Ordinate 1-13: Doppelt symmetrisches Sechseck mit verlängerter Längsachse und mit starker Verlängerung der senkrechten Symmetrieachse
Ordinate 1-14: Doppelt symmetrisches Sechseck mit verlängerter Längsachse und mit extremer Verlängerung der Symmetrieachse
Ordinate 1-15: Doppelt symmetrisches Sechseck mit verlängerter Längsachse und mit unregelmäßiger Verlängerung der Symmetrieachse
Ordinate 1-21: Doppelt symmetrisches Sechseck mit verlängerter Längsachse und mit einer Induktion
Ordinate 1-22: Doppelt symmetrisches Sechseck mit verlängerter Längsachse und mit zwei Induktionen
Ordinate 1-23: Doppelt symmetrisches Sechseck mit verlängerter Längsachse und mit drei Induktionen
Ordinate 1-24: Doppelt symmetrisches Sechseck mit verlängerter Längsachse und mit vier Induktionen
Ordinate 1-25: Doppelt symmetrisches Sechseck mit verlängerter Längsachse und mit fünf und mehr Induktionen

### Abszisse 1-63: Asymmetrisches Sechseck

Ordinate 1-11: Asymmetrisches Sechseck mit leichter Verlängerung der senkrechten Achse
Ordinate 1-12: Asymmetrisches Sechseck mit Verlängerung der senkrechten Achse
Ordinate 1-13: Asymmetrisches Sechseck mit starker Verlängerung der senkrechten Achse
Ordinate 1-14: Asymmetrisches Sechseck mit extremer Verlängerung der Achse
Ordinate 1-15: Asymmetrisches Sechseck mit unregelmäßiger Verlängerung der Achse
Ordinate 1-21: Asymmetrisches Sechseck mit einer Induktion
Ordinate 1-22: Asymmetrisches Sechseck mit zwei Induktionen
Ordinate 1-23: Asymmetrisches Sechseck mit drei Induktionen
Ordinate 1-24: Asymmetrisches Sechseck mit vier Induktionen
Ordinate 1-25: Asymmetrisches Sechseck mit fünf und mehr Induktionen

### Abszisse 1-64: Gleichseitige Vielecke, hier: Siebeneck

Ordinate 1-11: Gleichseitige Achtecke
Ordinate 1-12: Gleichseitige Neunecke
Ordinate 1-13: Gleichseitige Zehnecke
Ordinate 1-14: Gleichseitige Zwölfecke
Ordinate 1-15: Gleichseitige Zwanzigecke
Ordinate 1-21: Gleichseitige Vielecke mit einer Induktion
Ordinate 1-22: Gleichseitige Vielecke mit zwei Induktionen
Ordinate 1-23: Gleichseitige Vielecke mit drei Induktionen
Ordinate 1-24: Gleichseitige Vielecke mit vier Induktionen
Ordinate 1-25: Gleichseitige Vielecke mit fünf und mehr Induktionen

### Abszisse 1-65: Ungleichseitige, symmetrische Vielecke mit verlängerter Längsachse

Ordinate 1-11: Ungleichseitige, symmetrische Achtecke
Ordinate 1-12: Ungleichseitige, symmetrische Neunecke
Ordinate 1-13: Ungleichseitige, symmetrische Zehnecke
Ordinate 1-14: Ungleichseitige, symmetrische Zwölfecke
Ordinate 1-15: Ungleichseitige, symmetrische Zwanzigecke
Ordinate 1-21: Ungleichseitige, symmetrische Vielecke mit einer Induktion
Ordinate 1-22: Ungleichseitige, symmetrische Vielecke mit zwei Induktionen
Ordinate 1-23: Ungleichseitige, symmetrische Vielecke mit drei Induktionen
Ordinate 1-24: Ungleichseitige, symmetrische Vielecke mit vier Induktionen
Ordinate 1-25: Ungleichseitige, symmetrische Vielecke mit fünf und mehr Induktionen

### Abszisse 1-66: Teilsymmetrische Vielecke mit unbestimmten Eckenzahlen

Ordinate 1-11: Teilsymmetrische Vielecke mit unbestimmten Eckenzahlen und einer schwach verlängerten Längsachse
Ordinate 1-12: Teilsymmetrische Vielecke mit unbestimmten Eckenzahlen und einer verlängerten Längsachse
Ordinate 1-13: Teilsymmetrische Vielecke mit unbestimmten Eckenzahlen und einer stark verlängerten Längsachse
Ordinate 1-14: Teilsymmetrische Vielecke mit unbestimmten Eckenzahlen und einer sehr stark verlängerten Längsachse
Ordinate 1-15: Teilsymmetrische Vielecke mit unbestimmten Eckenzahlen und einer extrem verlängerten Längsachse Ordinate 1-21: Teilsymmetrische Vielecke mit unbestimmten Eckenzahlen und einer Induktion
Ordinate 1-22: Teilsymmetrische Vielecke mit unbestimmten Eckenzahlen und zwei Induktionen
Ordinate 1-23: Teilsymmetrische Vielecke mit unbestimmten Eckenzahlen und drei Induktionen
Ordinate 1-24: Teilsymmetrische Vielecke mit unbestimmten Eckenzahlen und vier Induktionen
Ordinate 1-25: Teilsymmetrische Vielecke mit unbestimmten Eckenzahlen und fünf und mehr Induktionen

### Abszisse 1-70: Voller Kreis

Ordinate 1-11: Voller Kreis mit leichter Verlängerung der senkrechten Symmetrieachse
Ordinate 1-12: Voller Kreis mit Verlängerung der senkrechten Symmetrieachse
Ordinate 1-13: Voller Kreis mit starker Verlängerung der senkrechten Symmetrieachse
Ordinate 1-14: Voller Kreis mit extremer Verlängerung der Symmetrieachse
Ordinate 1-15:Voller Kreis mit unregelmäßiger Verlängerung der Symmetrieachse
Ordinate 1-21: Voller Kreis mit einer leichten Induktion
Ordinate 1-22: Voller Kreis mit einer stärkeren Induktion
Ordinate 1-23: Voller Kreis mit einer starken Induktion
Ordinate 1-24: Voller Kreis mit einer sehr starken Induktion
Ordinate 1-25: Voller Kreis mit einer deformierenden Induktion

### Abszisse 1-71: Kreisförmig Figur

Ordinate 1-11: Kreisförmige, einfach symmetrische Figur mit leichter Verlängerung der senkrechten Symmetrieachse
Ordinate 1-12: Kreisförmige, einfach symmetrische Figur mit Verlängerung der senkrechten Symmetrieachse
Ordinate 1-13: Kreisförmige, einfach symmetrische Figur mit starker Verlängerung der senkrechten Symmetrieachse
Ordinate 1-14: Kreisförmige, einfach symmetrische Figur mit extremer Verlängerung der Symmetrieachse
Ordinate 1-15: Kreisförmige, einfach symmetrische Figur mit unregelmäßiger Verlängerung der Symmetrieachse
Ordinate 1-21: Kreisförmige, einfach symmetrische Figur mit einer leichten Induktion
Ordinate 1-22: Kreisförmige, einfach symmetrische Figur mit einer stärken Induktion
Ordinate 1-23: Kreisförmige, einfach symmetrische Figur mit einer starken Induktion
Ordinate 1-24: Kreisförmige, einfach symmetrische Figur mit einer sehr starken Induktion
Ordinate 1-25: Kreisförmige, einfach symmetrische Figur mit einer deformierenden Induktion

### Abszisse 1-72: Reduzierter Kreis

Ordinate 1-11: Kreisförmige Figur mit Bildung eines kleinen Innenhofes
Ordinate 1-12: Kreisförmige Figur mit Bildung eines Außenhofes
Ordinate 1-13: Kreisförmige Figur mit Bildung zweier Außenhöfe
Ordinate 1-14: Kreisförmige Figur mit Bildung dreier Außenhöfe
Ordinate 1-15: Kreisförmige Figur mit Bildung von vier und mehrerer Außenhöfe
Ordinate 1-21: Kreisförmige Figur mit einer tiefen Induktion
Ordinate 1-22: Kreisförmige Figur mit zwei tiefen Induktionen
Ordinate 1-23: Kreisförmige Figur mit drei tiefen Induktionen
Ordinate 1-24: Kreisförmige Figur mit vier tiefen Induktionen
Ordinate 1-25: Kreisförmige Figur mit fünf und mehr tiefen Induktionen

### Abszisse 1-73: Leerer Kreis

Ordinate 1-11: Leerer Kreis mit einer fingerartigen Verlängerung
Ordinate 1-12: Leerer Kreis mit einer längeren, fingerartigen Verlängerung
Ordinate 1-13: Leerer Kreis mit einer langen, fingerartigen Verlängerung
Ordinate 1-14: Leerer Kreis mit einer sehr langen, fingerartigen Verlängerung
Ordinate 1-15: Leerer Kreis mit einer linienförmig, fingerartigen Verlängerung
Ordinate 1-21: Leerer Kreis mit einem Halbschnitt
Ordinate 1-22: Leerer Kreis mit einem Ganzschnitt
Ordinate 1-23: Leerer Kreis mit zwei Ganzschnitten
Ordinate 1-24: Leerer Kreis mit einem Kreuzschnitt
Ordinate 1-25: Leerer Kreis mit einem Vielfachschnitt

### Tafel 11 Fraktal a:

Geometrische Strukturen: Muster flächiger Anordnung und/oder Organisation der Formen aus Tafel 1, wobei die Strukturen sich aus dem Kristallwchstum in der Fläche ergeben:
Die Abszisse geht von 1 - 9 :
Der positive Teil der Ordinate geht von 11-(1-4), der negative Teil von 11-(5-9).

### Abszisse 11-1: Gerade und parallele Gerade

Ordinate 11-1: einfache, ununterbrochene Gerade
Ordinate 11-2: einfache, einmal unterbrochene Gerade
Ordinate 11-3: einfache, zweifach unterbrochene Gerade
Ordinate 11-4: einfache, vielfach unterbrochene Gerade
Ordinate 11-5: doppelte, ununterbrochene Gerade
Ordinate 11-6: doppelte, unterbrochene Gerade
Ordinate 11-7: dreifache, ununterbrochene Gerade
Ordinate 11-8: dreifache, unterbrochene Gerade
Ordinate 11-9: vier- und mehrfache, unterbrochene Gerade

### Abszisse 11-2:

Ordinate 11-1: Gerade mit einer rechtwinkeligen, ungefähr mittigen Abzweigung
Ordinate 11-2: Gerade mit einer endständigen, rechtwinkeligen Abzweigung
Ordinate 11-3: Gerade mit mehreren, rechtwinkeligen, gleichlangen Abzweigungen
Ordinate 11-4: Gerade mit mehreren, rechtwinkligen, ungleichlangen Abzweigungen
Ordinate 11-5: Gerade mit einer unrechtwinkeligen Abzweigung
Ordinate 11-6: Gerade mit mehreren ungleichwinkeligen Abzweigungen
Ordinate 11-7: Gerade mit einer unrechtwinkligen Sekante
Ordinate 11-8: Gerade mit mehreren, unrechtwinkligen Sekanten
Ordinate 11-9: Gerade mit mehreren, unrechtwinkligen Abwinkelungen und Sekanten

### Abszisse 11-3:

Ordinate 11-1: Gerade mit einer rechtwinkeligen, mittständigen Sekante
Ordinate 11-2: Gerade mit einer rechtwinkeligen, nichtmittständigen Sekante
Ordinate 11-3: Gerade mit mehreren, gleichlangen Sekanten
Ordinate 11-4: Gerade mit mehreren, ungleichlangen Sekanten
Ordinate 11-5: Gerade mit einer rechtwinkeligen, endständigen Sekante
Ordinate 11-6: doppelte Gerade mit zwei rechtwinkeligen, mittständigen Sekanten
Ordinate 11-7: doppelte Gerade mit drei ung mehr rechtwinkeligen, mittständigen Sekanten
Ordinate 11-8: mehrere Geraden mit mehreren, rechtwinkeligen Sekanten
Ordinate 11-9: mehrere, gebrochene Geraden mit mehreren, rechtwinkeligen und gebrochenen Sekanten

### Abszisse 11-4:

Ordinate 11-1: Gerade mit einer zweifachen, endständigen Abzweigung
Ordinate 11-2: Gerade mit zwei symmetrischen Abzweigungen
Ordinate 11-3: Gerade mit mehreren, symmetrischen, gleichlangen Abzweigungen
Ordinate 11-4: Gerade mit mehreren, symmetrischen, ungleichlangen Abzweigungen
Ordinate 11-5: doppelte Gerade mit einer unrechtwinkeligen Sekante
Ordinate 11-6: doppelte Gerade mit zwei unrechtwinkeligen Sekanten
Ordinate 11-7: dreifache Gerade mit drei unrechtwinkeligen Sekanten
Ordinate 11-8: mehrfache Gerade mit mehreren, unrechtwinkligen Sekanten
Ordinate 11-9: mehrere, gebrochene Geraden mit mehreren, unrechtwinkeligen und gebrochenen Sekanten

### Abszisse 11-5:

Ordinate 11-1: Gerade mit zwei wechselständigen, rechtwinkeligen Abzweigungen
Ordinate 11-2: Gerade mit vier wechselständigen, rechtwinkeligen Abzweigungen
Ordinate 11-3: Gerade mit mehreren wechselständigen, gleichlangen, rechtwinkeligen Abzweigungen
Ordinate 11-4: Gerade mit mehreren wechselständigen, ungleichlangen, rechtwinkeligen Abzweigungen
Ordinate 11-5: Gerade mit zwei wechselständigen, unrechtwinkeligen, Abzweigungen
Ordinate 11-6: Gerade mit vier wechselständigen, unrechtwinkeligen, Abzweigungen
Ordinate 11-7: Gerade mit mehreren wechselständigen, ungleichlangen unrechtwinkeligen Abzweigungen
Ordinate 11-8: gewinkelte Geraden mit je einem rechtwinkeligen Treppenalgorithmus
Ordinate 11-9: gewinkelte Geraden mit einem einfachen, symmetrischen Treppenalgorithmus

### Abszisse 11-6:

Ordinate 11-1: Gerade mit zwei endständigen, freiwinkeligen Abzweigungen
Ordinate 11-2: Gerade mit zwei Gruppen gegenläufiger, rechtwinkeliger Abzweigungen
Ordinate 11-3: gewinkelte Gerade mit je einer Gruppe rechtwinkeliger Abzweigungen
Ordinate 11-4: gewinkelte Geraden mit einem einfachen, symmetrischen Treppenalgorithmus, gespiegelt und teilweise gebrochen aufeinanderliegend
Ordinate 11-6: Winkel mit horizontaler Symmetrieachse
Ordinate 11-7: Vertikal gereihte Winkel mit horizontaler Symmetrieachse
Ordinate 11-8: Zwei und mehrere, vertikal gereihte Winkel mit horizontaler Symmetrieachse
Ordinate 11-9: Unterschiedlich große, vertikal gereihte Winkel mit je horizontaler Symmetrieachse

### Abszisse 11-7:

Ordinate 11-1: Gerade mit endständiger, rechtwinkeliger Quergerade (T-förmig)
Ordinate 11-2: Mehrere Geraden mit mehreren, rechtwinkeligen, endständigen Quergeraden
Ordinate 11-3: Mehrere Geraden, die sich an mehreren, endständigen, rechtwinkeligen Quergeraden in unterschiedlicher Anzahl, jedoch rechtwinklig spiegeln
Ordinate 11-4: Mehrere Geraden, die sich an mehreren, endständigen, rechtwinkeligen Quergeraden in gleicher Anzahl, jedoch rechtwinklig spiegeln
Ordinate 11-5: Gerade mit endständiger, unrechtwinkeliger Quergerade
Ordinate 11-6: Mehrere Geraden, die sich an mehreren, endständigen, unrechtwinkeligen Quergeraden in unterschiedlicher Anzahl spiegeln
Ordinate 11-7: Mehrere Geraden, die Sich an mehreren, endständigen, unrechtwinkeligen Quergeraden in unterschiedlicher Anzahl spiegeln
Ordinate 11-8: Mehrere Geraden, die sich an mehreren, endständigen, unrechtwinkeligen Quergeraden in gleicher Anzahl spiegeln
Ordinate 11-9: Mehrere gebündelte, symmetrisch angeordnete Geraden

### Abszisse 11-8:

Ordinate 11-1: Niedrigzahlige, parallele Schar
Ordinate 11-2: Hochzahlige, parallele Schar
Ordinate 11-3: Beliebigzahlige, nichtparallele Schar
Ordinate 11-4: Beliebigzahlige, nichtparallele Schar mit deutlich unterschiedlichen Längen der Geraden
Ordinate 11-5: Winkel mit vertikaler Symmetrieachse
Ordinate 11-6: Geringzahlige Winkelschar
Ordinate 11-7: Hochzahlige Winkelschar
Ordinate 11-8: Sternförmige, geringzahlige Winkelschar
Ordinate 11-9: Sternförmige, hochzahlige Winkelschar

### Abszisse 11-9:

Ordinate 11-1: Niedrigzahlige, parallele Schar gebrochener Geraden
Ordinate 11-2: Hochzahlige, parallele Schar gebrochener Geraden
Ordinate 11-3: Beliebigzahlige, nichtparallele Schar gebrochener Geraden
Ordinate 11-4: Beliebigzahlige, nichtparallele bis haufenförmige Schar mit deutlich verkürzten, punktförmigen Längen
Ordinate 11-5: Zwei gegeneinander gespiegelte Winkel mit vertikaler Symmetrieachse
Ordinate 11-6: Geringzahlige Bündelschar mit einer punktförmigen Zentrum
Ordinate 11-7: Hochzahlige Winkelschar mit einem punktförmigen Zentrum
Ordinate 11-8: Sternförmige, geringzahlige Winkelschar mit einem punktförmigen Zentrum
Ordinate 11-9: Sternförmige, hochzahlige Winkelschar mit einem punktförmigen Zentrum

### Tafel 111 Fraktal b:

Die Tafel besteht aus einer Abszisse von 111-(1-9) und bezeichnet die Hauptausdehungsrichtung (Stamm) der Strukturen aus Tafel 2. Diese Strukturen der Ausrichtung beziehen sich auf den stärksten Dentriden einer wachsenden Kristallstruktur
Abszisse 111-1: lineare Ausbreitungsrichtung
Abszisse 111-2: einfach verzweigte Ausbreitungsrichtung
Abszisse 111-3: zweifach, symmetrisch verzweigte Ausbreitungsrichtung
Abszisse 111-4: kreuzförmige Ausbreitungsrichtung
Abszisse 111-5: sternförmige Ausbreitungsrichtung
Abszisse 111-6: leicht gebogene Ausbreitungsrichtung
Abszisse 111-7: stark gebogene Ausbreitungsrichtung
Abszisse 111-8: s-förmige Ausbreitungsrichtung
Abszisse 111-9: kreisförmige Ausbreitungsrichtung

### Tafel 1111 Fraktal c:

Die Tafel besteht aus einer Abszisse von 1111-(1-9) und bezeichnet die Nebenausdehungsrichtung (Zweig) der Strukturen aus Tafel 2. Diese Strukturen der Ausrichtung beziehen sich auf die abzweigenden oder untergeordneten Dentriten einer wachsenden Kristallstruktur.
Abszisse 1111-1: lineare Ausbreitungsrichtung
Abszisse 1111-2: leicht gebogene Ausbreitungsrichtung
Abszisse 1111-3: stark gebogene Ausbreitungsrichtung
Abszisse 1111-4: kreisnahe Ausbreitungsrichtung
Abszisse 1111-5: bogig getreppte Ausbreitungsrichtung
Abszisse 1111-6: links-rechts-drehende, s-förmige Ausbreitungsrichtung
Abszisse 1111-7: rechts-links-drehende, s-förmige Ausbreitungsrichtung
Abszisse 1111-8: kreisförmige Ausbreitungsrichtung
Abszisse 1111-9: achförmige Ausbreitungsrichtung

### Tafel 12 Cluster a:

Die geometrischen Strukturen zeigen die während einer Kristallisation entstehenden, räumlichen Grundmuster der Anfügung.
Überlappung oder Verbindung aus Tafel 1.
Die Abszisse geht von 12-1 bis 12-9, die Ordinate von 12-(1-5).

### Ordinate 12-0: Grundformen

Abszisse 12-1: Grundform Linie
Abszisse 12-2: Grundform Welle
Abszisse 12-3: Grundform Bogen
Abszisse 12-4: Grundform Halbkreis
Abszisse 12-5: Grundform Ast mit gegenständigen Zweigen
Abszisse 12-6: Grundform Ast mit wechselständigen Zweigen
Abszisse 12-7: Grundform Vortex
Abszisse 12-8: Grundform Kreise (Kugel)
Abszisse 12-9: Grundform Kreisringe

### Ordinate 12-1: Einzelne, nichtzusammenhängende Elemente

Abszisse 12-1: Linie aus einzelnen, nichtzusammenhängenden Elementen
Abszisse 12-2: Welle aus einzelnen, nichtzusammenhängenden Elementen
Abszisse 12-3: Bogen aus einzelnen, nichtzusammehängenden Elementen
Abszisse 12-4: Halbkreis aus einzelnen, nichtzusammenhängenden Elementen
Abszisse 12-5: Ast mit gegenständigen Zweigen aus einzelnen, nichtzusammenhängenden Elementen
Abszisse 12-6: Ast mit wechselständigen Zweigen aus einzelnen, nichtzusammenhängenden Elementen
Abszisse 12-7: Vortex aus einzelnen, nichtzusammenhängenden Elementen
Abszisse 12-8: Kreis aus einzelnen, nichtzusammenhängenden Elementen
Abszisse 12-9: Kreisringe aus einzelnen, nichtzusammenhängenden Elementen

### Ordinate 12-2: Einzelne, zusammenhängende Elemente

Abszisse 12-1: Linie aus einzelnen, zusammenhängenden Elementen
Abszisse 12-2: Welle aus einzelnen, zusammenhängenden Elementen
Abszisse 12-3: Bogen aus einzelnen, zusammenhängenden Elementen
Abszisse 12-4: Halbkreis aus einzelnen, zusammenhängenden Elementen
Abszisse 12-5: Ast mit gegenständigen Zweigen aus einzelnen, zusammenhängenden Elementen
Abszisse 12-6: Ast mit wechselständigen Zweigen aus einzelnen, zusammenhängenden Elementen
Abszisse 12-7: Vortex aus einzelnen, zusammenhängenden Elementen
Abszisse 12-8: Kreis aus einzelnen, zusammenhängenden Elementen
Abszisse 12-9: Kreisringe aus einzelnen, zusammenhängenden Elementen

### Ordinate 12-3: Mehrere, nichtzusammenhängende Elemente

Abszisse 12-1: Linie aus mehreren, nichtzusammenhängenden Elementen
Abszisse 12-2: Welle aus mehreren nichtzusammenhängenden Elementen
Abszisse 12-3: Bogen aus mehreren, nichtzusammenhängenden Elementen
Abszisse 12-4: Halbkreis aus mehreren, nichtzusammenhängenden Elementen
Abszisse 12-5: Ast mit gegenständigen Zweigen aus mehreren, nichtzusammenhängenden Elementen
Abszisse 12-6: Ast mit wechselständigen Zweigen aus mehreren, nichtzusammenhängenden Elementen
Abszisse 12-7: Vortex aus mehreren, nichtzusammenhängenden Elementen
Abszisse 12-8: Kreis aus mehreren, nichtzusammenhängenden Elementen
Abszisse 12-9: Kreisringe aus mehreren, nichtzusammenhängenden Elementen

### Ordinate 12-4: Mehrere, zusammenhängende Elemente

Abszisse 12-1: Linie aus mehreren, zusammenhängenden Elementen
Abszisse 12-2: Welle aus mehreren, zusammenhängenden Elementen
Abszisse 12-3: Bogen aus mehreren, zusammenhängenden Elementen
Abszisse 12-4: Halbkreis aus mehreren, zusammenhängenden Elementen
Abszisse 12-5: Ast mit gegenständigen Zweigen aus mehreren, zusammenhängenden Elementen
Abszisse 12-6: Ast mit wechselständigen Zweigen aus mehreren, zusammenhängenden Elementen
Abszisse 12-7: Vortex aus mehreren, zusammenhängenden Elementen
Abszisse 12-8: Kreis aus mehreren, zusammenhängenden Elementen Abszisse 12-9: Kreisringe aus mehreren, zusammenhängenden Elementen

### Ordinate 12-5: Negative der Grundformen aus Ordinate 12-0

Abszisse 12-1: Negative Grundform Linie
Abszisse 12-2: Negative Grundform Welle
Abszisse 12-3: Negative Grundform Bogen
Abszisse 12-4: Negative Grundform Halbkreis
Abszisse 12-5: Negative Grundform Ast mit gegenständigen Zweigen
Abszisse 12-6: Negative Grundform Ast mit wechselständigen Zweigen
Abszisse 12-7: Negative Grundform Vortex
Abszisse 12-8: Negative Grundform Kreis (Kugel)
Abszisse 12-9: Negative Grundform Kreisringe

### Tafel 121 Cluster b:

Die Tafel 121 besteht aus einer Abszisse von 121-(1-9) und bezeichnet die während einer Kristallisation auftretenden Modalitäten der inneren,räumlichen Anordnung der durch Tafel 12 definierten Texturen
Abszisse 121-1: ein/zweifach geschichtet
Abszisse 121-2: drei/vierfach geschichtet
Abszisse 121-3: vielfach geschichtet
Abszisse 121-4: kreisförmig angeordnet
Abszisse 121-5: bandförmig angeordnet
Abszisse 121-6: scharenbändrig angeordnet
Abszisse 121-7: kreuzbändrig angeordnet
Abszisse 121-8: netzartig angeordnet
Abszisse 121-9: haufenartig angeordnet

### Tafel 122 Cluster c:

Die Tafel 122 besteht aus einer Abszisse von 122-(1-9) und bezeichnet die während einer Kristallisation auftretenden äusseren Grenzstrukturen der durch Tafel 12 definierten Texturen
Abszisse 122-1: Parallele Scharen
Abszisse 122-2: Winkelscharen
Abszisse 122-3: Kreisflächen
Abszisse 122-4: Dreiecksflächen
Abszisse 122-5: Vierecks- und Rautenflächen
Abszisse 122-6: Fünfecksflächen
Abszisse 122-7: Sechsecksflächen
Abszisse 122-8: Vielecksflächen
Abszisse 122-9: Beliebige Flächen

### Tafel 123 Cluster d:

Die Tafel besteht aus einer Abszisse von 123-(1-9) und bezeichnet die während einer Kristallisation entstehenden Felder aus den Formen und Strukturen der Tafeln 1 und 12 in ihrer relativen Verteilung und/oder als Organisationsform
Abszisse 123-1: Ringformen
Abszisse 123-2: Punktformen
Abszisse 123-3: Haufenformen
Abszisse 123-4: Fadenformen
Abszisse 123-5: Gewebeformen
Abszisse 123-6: Strahlenformen
Abszisse 123-7: Schalenformen
Abszisse 123-8: Rasterformen
Abszisse 123-9: Flächenformen

### Tafel 13 Kontur a:

Die Tafel besteht aus einer Ordinate von 13-(1-9) und bezeichnet den während einer Kristallisation entstehenden Kontrast einer Form oder Textur aus der Tafel 12 im Verhältnis zu seiner/ihrer unmittelbaren Umgebung im Sinne einer Grenze
Ordinate 13-1: fließende Grenze mit geringem Kontrast zwischen den beiden Grenzflächen
Ordinate 13-2: definierte Grenze mit merklichem Kontrast zwischen den beiden Grenzflächen
Ordinate 13-3: markante Grenzen mit starkem Kontrast zwischen den beiden Grenzflächen
Ordinate 13-4: Grenze zwischen einer homogenen und einer inhomogenen Fläche
Ordinate 13-5: Grenze mit unregelmäßigem Profil
Ordinate 13-6: Grenze mit regelmäßigem, kleinförmigem Profil
Ordinate 13-7: Grenze mit unregelmäßigem, großförmigem Profil
Ordinate 13-8: Grenze mit periodischem Profil bei einer kleinen Profilamplitude
Ordinate 13-9: Grenze mit periodischem Profil bei einer großen Profilamplitude

### Tafel 131 Kontur b:

Die Tafel besteht aus einer Ordinate von 131-(1-9) und bezeichnet die während einer Kristallisation gefundenen Flächenformen im Sinne eines Binnenkontrasts im Inneren der einfachen oder zusammengesetzten Formen und Texturen aus der Tafel 12.
Ordinate 131-1: Ton in Ton
Ordinate 131-2: Dichter gegen weniger dicht
Ordinate 131-3: Sehr dicht gegen wenig dicht
Ordinate 131-4: Einfache Schlierenform
Ordinate 131-5: Zusammengesetzte Schlierenform
Ordinate 131-6: Komplexe Schlierenform
Ordinate 131-7: Einfache Schleierform
Ordinate 131-8: Zusammengesetzte Schleierform
Ordinate 131-9: Komplexe Schleierform

### Tafel 132 Kontur c:

Die Tafel besteht aus einer Ordinate von 132-(1-9) und bezeichnet den gefundenen Sektor, in dem sich die Formen und/oder Texturen aus Tafel 12 befinden.
Ordinate 132-1: Äußerste Domäne einer Bildfläche
Ordinate 132-2: Mittlere Domäne einer Bildfläche
Ordinate 132-3: Äußerste und mittlere Domäne einer Bildfläche
Ordinate 132-4: Mittlere und innerste Domäne einer Bildfläche
Ordinate 132-5: Innerste Domäne einer Bildfläche
Ordinate 132-6: Äußerste und innerste Domäne einer Bildfläche
Ordinate 132-7: Alle Domänen einer Bildfläche
Ordinate 132-8: Rand einer Bildfläche
Ordinate 132-9: Zerrissene Bildfläche mit oder ohne Satellit

### Tafel 133 Kontur d:

Die Tafel besteht aus einer Ordinate von 133-(1-9) und bezeichnet die während einer Kristallisation entstehenden Großtexturen, bestehend aus den gleichartigen Formen und/oder Texturen aus Tafel 12.
Ordinate 133-1: Punktuelle, wenig ausgedehnte Gesamtform
Ordinate 133-2: Dreieckartige Gesamtform
Ordinate 133-3: Viereckartige Gesamtform
Ordinate 133-4: Raufenartige Gesamtform
Ordinate 133-5: Fünfeckartige Gesamtform
Ordinate 133-6: Sechseckartige Gesamtform
Ordinate 133-7: Vieleckartige bis kreisartige Gesamtform
Ordinate 133-8: Bahnartige Gesamtform
Ordinate 133-9: Dendritenartige Gesamtform

### Tafel 2 Textur A:

Formen, entsprechend den Formen der **Tafel 1**, wie sie während einer Kristallisation entstehen und sich einer Grundform oder Grundstruktur aus Tafel 1 räumlich assoziieren.

### Abszisse 2-00: Punkt

Ordinate 2-11: In einer Linie verdoppelter Punkt
Ordinate 2-12: In einer Line vervielfachter Punkt
Ordinate 2-13: Zwei, in einer Linie liegende Punktgruppen
Ordinate 2-14: Lange, regelmäßige Punktgruppe
Ordinate 2-15 Lange, unregelmäßige Punktgruppe
Ordinate 2-21: Vergrößerter Punkt mit einer Einkerbung
(Induktion)
Ordinate 2-22: Vergrößerter Punkt mit zwei Induktionen
Ordinate 2-23: Vergrößerter Punkt mit drei Induktionen
Ordinate 2-24: Vergrößerter Punkt mit vier Induktionen
Ordinate 2-25: Vergrößerter Punkt mit fünf und mehr Induktionen.

### Abszisse 2-01: in Längsrichtung und ansatzweise in Querrichtung ausgedehnter Punkt

Ordinate 2-11: Ausdehnung des Punktes zu einem symmetrischen Oval
Ordinate 2-12: Längsausdehnung des Punktes zu einem symmetrischen Oval
Ordinate 2-13: Weitere Längsausdehnung des Punktes zu einem symmetrischen Oval
Ordinate 2-14: Auflösung der korpuskulären Struktur des Oval
Ordinate 2-15: Unregelmäßige Längsausdehung des Ovals zur räumlichen Linie
Ordinate 2-21: Einfache Induktion der Linie (Bogen)
Ordinate 2-22: Zweifache Induktion der Linie (Schlange)
Ordinate 2-23: Dreifache Induktion der Linie (Zickzack)
Ordinate 2-24: Vierfache Induktion der Linie mit leichter Glättung der Ecken
Ordinate 2-25: Fünf- und mehrfache Induktion der Linie mit Glättung der Ecken

### Abszisse 2-10: in Längsrichtung ausgedehnter Punkt: Linie

Ordinate 2-11: Gerade
Ordinate 2-12: Einfach unterbrochene Gerade
Ordinate 2-13: Mehrfach unterbrochene Gerade aus regelmäßigen Geraderesten
Ordinate 2-14: Mehrfach unterbrochene Gerade aus unregelmäßigen Geraderesten
Ordinate 2-15: Gebrochene Gerade aus vielfachen, unregelmäßigen Geraderesten
Ordinate 2-21: Gerade mit einer winkeligen Induktion
Ordinate 2-22: Gerade mit zwei winkeligen Induktionen
Ordinate 2-23: Gerade mit drei und mehr winkeligen Induktionen Ordinate 2-24: Gerade mit vier und mehr winkeligen Induktionen mit Überstand am Scheitel Ordinate 2-25: Auflösende Gerade mit fünf und mehr winkeligen Induktionen

### Abszisse 2-11: Linie mit freiwinkeliger Abzweigung

Ordinate 2-11: Gerade mit spitzwinkeliger Abzweigung
Ordinate 2-12: Gerade mit sehr spitzwinkeliger Abzweigung
Ordinate 2-13: Gerade mit extrem spitzwinkeliger Abzweigung
Ordinate 2-14: Gerade mit in die Gerade einlaufender Abzweigung
Ordinate 2-15: Unregelmäßig ausgedehnte Gerade mit in sie einlaufender Abzweigung
Ordinate 2-21: Gerade mit spitzwinkeliger Abzweigung und einer Induktion
Ordinate 2-22: Gerade mit spitzwinkeliger Abzweigung und zwei Induktionen
Ordinate 2-23: Gerade mit spitzwinkeliger Abzweigung und drei Induktionen
Ordinate 2-24: Gerade mit spitzwinkeliger Abzweigung und vier Induktionen
Ordinate 2-25: Gerade mit spitzwinkeliger Abzweigung und fünf oder mehr Induktionen

### Abszisse 2-12: Linie mit rechtwinkeliger Abzweigung

Ordinate 2-11: Gerade mit einer rechtwinkeligen, längeren Abzweigung
Ordinate 2-12: Gerade mit mehreren, rechtwinkeligen Abzweigungen
Ordinate 2-13: Gerade mit mehreren, rechtwinkeligen, gegenständigen Abzweigungen
Ordinate 2-14: Gerade mit mehreren, rechtwinkeligen, wechselständigen Abzweigungen
Ordinate 2-15: Gerade mit mehreren, rechtwinkeligen, unterschiedlichständigen Abzweigungen
Ordinate 2-21: Gerade mit zwei rechtwinkligen Brechungen
Ordinate 2-22: Gerade mit drei rechtwinkligen Brechungen
Ordinate 2-23: Gerade mit vier rechtwinkligen Brechungen
Ordinate 2-24: Gerade mit fünf und mehr rechtwinkligen Brechungen
Ordinate 2-25: Gerade mit beliebig vielen rechtwinkligen Brechungen

### Abszisse 2-13: Linie mit gleichschenkliger Abzweigung

Ordinate 2-11: Symmetrische Winkelgerade mit großem Winkel
Ordinate 2-12: Symmetrischie Winkelgerade mit kleinem Winkel
Ordinate 2-13: Symmetrische Winkelgerade mit sehr kleinem Winkel
Ordinate 2-14: Symmetrische Winkelgerade mit extrem kleinem Winkel
Ordinate 2-15: Symmetrische, unregelmäßig verlängerte Winkelgerade mit minimalem Winkel
Ordinate 2-21: Symmetrische Winkelgerade mit einer Induktion
Ordinate 2-22: Symmetrische Winkelgerade mit zwei Induktionen
Ordinate 2-23: Symmetrische Winkelgerade mii drei Induktionen
Ordinate 2-24: Symmetrische Winkelgerade mit vier, symmetrischen Induktionen
Ordinate 2-25: Symmetrische Winkelgerade mit vier, symmetrisch reduzierenden Induktionen

### Abszisse 2-20: Gleichseitiges Dreieck

Ordinate 2-11: Gleichseitiges Dreieck mit leichter Verlängerung der senkrechten Symmetrieachse
Ordinate 2-12: Gleichseitiges Dreieck mit Verlängerung der senkrechten Symmetrieachse
Ordinate 2-13: Gleichseitiges Dreieck mit starker Verlängerung der senkrechten Symmetrieachse
Ordinate 2-14: Gleichseitiges Dreieck mit extreme Verlängerung der Symmetrieachse
Ordinate 2-15: Gleichseitiges Dreieck mit unregelmäßiger Verlängerung der Symmetrieachse
Ordinate 2-21: Gleichseitiges Dreieck mit einer Induktion
Ordinate 2-22: Gleichseitiges Dreieck mit zwei Induktionen
Ordinate 2-23: Gleichseitiges Dreieck mit drei Induktionen
Ordinate 2-24: Gleichseitiges Dreieck mit vier Induktionen
Ordinate 2-25: Gleichseitiges Dreieck mit fünf und mehr Induktionen

### Abszisse 2-21: Ungleichseitiges Dreieck

Ordinate 2-11: Ungleichseitiges Dreieck mit leichter Verlängerung der senkrechten Achse
Ordinate 2-12: Ungleichseitiges Dreieck mit Verlängerung der senkrechten Achse
Ordinate 2-13: Ungleichseitiges Dreieck mit starker Verlängerung der senkrechten Achse
Ordinate 2-14: Ungleichseitiges Dreieck in extremer Verlängerung der Achse
Ordinate 2-15: Ungleichseitiges Dreieck mit unregelmäßiger Verlängerung der Achse
Ordinate 2-21: Ungleichseitiges Dreieck mit einer Induktion
Ordinate 2-22: Ungleichseitiges Dreieck mit zwei Induktionen
Ordinate 2-23: Ungleichseitiges Dreieck mit drei Induktionen
Ordinate 2-24: Ungleichseitiges Dreieck mit vier Induktionen
Ordinate 2-25: Ungleichseitiges Dreieck mit fünf und mehr Induktionen

### Abszisse 2-22: Rechtwinkeliges Dreieck

Ordinate 2-11: Rechtwinkeliges Dreieck mit leichter Verlängerung der senkrechten Achse
Ordinate 2-12: Rechtwinkeliges Dreieck mit Verlängerung der senkrechten Achse
Ordinate 2-13: Rechtwinkeliges Dreieck mit starker Verlängerung der senkrechten Achse
Ordinate 2-14: Rechtwinkeliges Dreieck mit extremer Verlängerung der Achse
Ordinate 2-15: Rechtwinkeliges Dreieck mit unregelmäßiger Verlängerung der Achse
Ordinate 2-21: Rechtwinkeliges Dreieck mit einer Induktion
Ordinate 2-22: Rechtwinkeliges Dreieck mit zwei Induktionen
Ordinate 2-23: Rechtwinkeliges Dreieck mit drei Induktionen
Ordinate 2-24: Rechtwinkeliges Dreieck mit vier Induktionen
Ordinate 2-25: Rechtwinkeliges Dreieck mit fünf und mehr Induktionen

### Abszisse 2-23: Spitzwinkeliges Dreieck

Ordinate 2-11: Spitzwinkeliges Dreieck mit leichter Verlängerung der senkrechten Achse
Ordinate 2-12: Spitzwinkeliges Dreieck mit Verlängerung der senkrechten Achse
Ordinate 2-13: Spitzwinkeliges Dreieck mit starker Verlängerung der senkrechten Achse
Ordinate 2-14: Spitzwinkeliges Dreieck mit extremer Verlängerung der Achse
Ordinate 2-15: Spitzwinkeliges Dreieck mit unregelmäßiger Verlängerung der Achse
Ordinate 2-21: Spitzwinkeliges Dreieck mit einer Induktion
Ordinate 2-22: Spitzwinkeliges Dreieck mit zwei Induktionen
Ordinate 2-23: Spitzwinkeliges Dreieck mit drei Induktionen
Ordinate 2-24: Spitzwinkeliges Dreieck mit vier Induktionen
Ordinate 2-25: Spitzwinkeliges Dreieck mit fünf und mehr Induktionen

### Abszisse 2-24: Spitzwinkeliges Dreieck mit einer gespreizten Ecke zum beginnenden Viereck

Ordinate 2-11: Spitzwinkeliges Dreieck mit leichter Verlängerung der senkrechten Achse
Ordinate 2-12: Spitzwinkeliges Dreieck mit Verlängerung der senkrechten Achse
Ordinate 2-13: Spitzwinkeliges Dreieck mit starker Verlängerung der senkrechten Achse
Ordinate 2-14: Spitzwinkeliges Dreieck mit extremer Verlängerung der Achse
Ordinate 2-15: Spitzwinkeliges Dreieck mit unregelmäßiger Verlängerung der Achse
Ordinate 2-21: Spitzwinkeliges Dreieck mit einer Induktion
Ordinate 2-22: Spitzwinkeliges Dreieck mit zwei Induktionen
Ordinate 2-23: Spitzwinkeliges Dreieck mit drei Induktionen
Ordinate 2-24: Spitzwinkeliges Dreieck mit vier Induktionen
Ordinate 2-25: Spitzwinkeliges Dreieck mit fünf und mehr Induktionen

### Abszisse 2-30: Gleichseitiges Viereck, Quadrat

Ordinate 2-11: Gleichseitiges Viereck mit leichter Verlängerung der senkrechten Symmetrieachse
Ordinate 2-12: Gleichseitiges Viereck mit Verlängerung der senkrechten Symmetrieachse
Ordinate 2-13: Gleichseitiges Viereck mit starker Verlängerung der senkrechten Symmetrieachse
Ordinate 2-14: Gleichseitiges Viereck mit extremer Verlängerung der Symmetrieachse
Ordinate 2-15: Gleichseitiges Viereck mit unregelmäßiger Verlängerung der Symmetrieachse
Ordinate 2-21: Gleichseitiges Viereck mit einer Induktion
Ordinate 2-22: Gleichseitiges Viereck mit zwei Induktionen
Ordinate 2-23: Gleichseitiges Viereck mit drei Induktionen
Ordinate 2-24: Gleichseitiges Viereck mit vier Induktionen
Ordinate 2-25: Gleichseitiges Viereck mit fünf und mehr Induktionen

### Abszisse 2-31: Regelmäßiges, symmetrisches Viereck, Rechteck

Ordinate 2-11: Regelmäßiges Viereck mit leichter Verlängerung der senkrechten Symmetrieachse
Ordinate 2-12: Regelmäßiges Viereck mit Verlängerung der senkrechten Symmetrieachse
Ordinate 2-13: Regelmäßiges Viereck mit starker Verlängerung der senkrechten Symmetrieachse
Ordinate 2-14: Regelmäßiges Viereck mit extremer Verlängerung der Symmetrieachse
Ordinate 2-15: Regelmäßiges Viereck mit unregelmäßiger Verlängerung der Symmetrieachse
Ordinate 2-21: Regelmäßiges Viereck mit einer Induktion
Ordinate 2-22: Regelmäßiges Viereck mit zwei Induktionen
Ordinate 2-23: Regelmäßiges Viereck mit drei Induktionen
Ordinate 2-24: Regelmäßiges Viereck mit vier Induktionen
Ordinate 2-25: Regelmäßiges Viereck mit fünf und mehr Induktionen

### Abszisse 2-32: Unsymmetrisches Viereck

Ordinate 2-11: Unsymmetrisches Viereck mit leichter Verlängerung der senkrechten Symmetrieachse
Ordinate 2-12: Unsymmetrisches Viereck mit Verlängerung der senkrechten Symmetrieachse
Ordinate 2-13: Unsymmetrisches Viereck mit starker Verlängerung der senkrechten Symmetrieachse
Ordinate 2-14: Unsymmetrisches Viereck mit extremer Verlängerung der Symmetrieachse
Ordinate 2-15: Unsymmetrisches Viereck mit unregelmäßiger Verlängerung der Symmetrieachse
Ordinate 2-21: Unsymmetrisches Viereck mit einer Induktion
Ordinate 2-22: Unsymmetrisches Viereck mit zwei Induktionen
Ordinate 2-23: Unsymmetrisches Viereck mit drei Induktionen
Ordinate 2-24: Unsymmetrisches Viereck mit vier Induktionen
Ordinate 2-25: Unsymmetrisches Viereck mit fünf und mehr Induktionen

### Abszisse 2-33: Stark unsymmetrisches Viereck

Ordinate 2-11: Stark unsymmetrisches Viereck mit leichter Verlängerung der senkrechten Symmetrieachse
Ordinate 2-12: Stark unsymmetrisches Viereck mit Verlängerung der senkrechten Symmetrieachse
Ordinate 2-13: Stark unsymmetrisches Viereck mit starker Verlängerung der senkrechten Symmetrieachse
Ordinate 2-14: Stark unsymmetrisches Viereck mit extremer Verlängerung der Symmetrieachse
Ordinate 2-15: Stark unsymmetrisches Viereck mit Stark unregelmäßiger Verlängerung der Symmetrieachse
Ordinate 2-21: Stark unsymmetrisches Viereck mit einer Induktion
Ordinate 2-22: Stark unsymmetrisches Viereck mit zwei Induktionen
Ordinate 2-23: Stark unsymmetrisches Viereck mit drei Induktionen
Ordinate 2-24: Stark unsymmetrisches Viereck mit vier Induktionen
Ordinate 2-25: Stark unsymmetrisches Viereck mit fünf Stark und mehr Induktionen

### Abszisse 2-34: Liegende Raute

Ordinate 2-11: Liegende Raute mit leichter Verlängerung der senkrechten Symmetrieachse
Ordinate 2-12: Liegende Raute mit Verlängerung der senkrechten Symmetrieachse
Ordinate 2-13: Liegende Raute starker Verlängerung der senkrechten Symmetrieachse
Ordinate 2-14: Liegende Raute mit extremer Verlängerung der Symmetrieachse
Ordinate 2-15: Liegende Raute mit unregelmäßiger Verlängerung der Symmetrieachse
Ordinate 2-21: Liegende Raute mit einer Induktion
Ordinate 2-22: Liegende Raute mit zwei Induktionen
Ordinate 2-23: Liegende Raute mit drei Induktionen
Ordinate 2-24: Liegende Raute mit vier Induktionen
Ordinate 2-25: Liegende Raute mit fünf und mehr Induktionen

### Abszisse 2-35: Trapez

Ordinate 2-11: Trapez mit leichter Verlängerung der senkrechten Symmetrieachse
Ordinate 2-12: Trapez mit Verlängerung der senkrechten Symmetrieachse
Ordinate 2-13: Trapez mit starker Verlängerung der senkrechten Symmetrieachse
Ordinate 2-14: Trapez mit extremer Verlängerung der Symmetrieachse
Ordinate 2-15: Trapez mit unregelmäßiger Verlängerung der Symmetrieachse
Ordinate 2-21: Trapez mit einer Induktion
Ordinate 2-22: Trapez mit zwei Induktionen
Ordinate 2-23: Trapez mit drei Induktionen
Ordinate 2-24: Trapez mit vier Induktionen
Ordinate 2-25: Trapez mit fünf und mehr Induktionen

### Abszisse 2-40: Stehende Raute

Ordinate 2-11: Stehende Raute mit leichter Verlängerung der senkrechten Symmetrieachse
Ordinate 2-12: Stehende Raute mit Verlängerung der senkrechten Symmetrieachse
Ordinate 2-13: Stehende Raute mit starker Verlängerung der senkrechten Symmetrieachse
Ordinate 2-14: Stehende Raute mit extremer Verlängerung der Symmetrieachse
Ordinate 2-15: Stehende Raute mit unregelmäßiger Verlängerung der Symmetrieachse
Ordinate 2-21: Stehende Raute mit einer Induktion
Ordinate 2-22: Stehende Raute mit zwei Induktionen
Ordinate 2-23: Stehende Raute mit drei Induktionen
Ordinate 2-24: Stehende Raute mit vier Induktionen
Ordinate 2-25: Stehende Raute mit fünf und mehr Induktionen

### Abszisse 2-41: Asymmetrische, stehende Raute

Ordinate 2-11: Asymmetrisch stehende Raute mit leichter Verlängerung der senkrechten Achse
Ordinate 2-12: Asymmetrisch stehende Raute mit Verlängerung der senkrechten Achse
Ordinate 2-13: Asymmetrisch stehende Raute mit starker Verlängerung der senkrechten Achse
Ordinate 2-14: Asymmetrisch stehende Raute mit extremer Verlängerung der Achse
Ordinate 2-15: Asymmetrisch stehende Raute mit unregelmäßiger Verlängerung der Achse
Ordinate 2-21: Asymmetrisch stehende Raute mit einer Induktion
Ordinate 2-22: Asymmetrisch stehende Raute mit zwei Induktionen
Ordinate 2-23: Asymmetrisch stehende Raute mit drei Induktionen
Ordinate 2-24: Asymmetrisch stehende Raute mit vier Induktionen
Ordinate 2-25: Asymmetrisch stehende Raute mit fünf und mehr Induktionen

### Abszisse 2-42: Stark asymmetrisch, stehende Raute

Ordinate 2-11: Stark asymmetrisch stehende Raute mit leichter Verlängerung der senkrechten Achse
Ordinate 2-12: Stark asymmetrisch stehende Raute mit Verlängerung der senkrechten Achse
Ordinate 2-13: Stark asymmetrisch stehende Raute mit starker Verlängerung der senkrechten Achse
Ordinate 2-14: Stark asymmetrisch stehende Raute mit extremer Verlängerung der Achse
Ordinate 2-15: Stark asymmetrisch stehende Raute mit unregelmäßiger Verlängerung der Achse
Ordinate 2-21: Stark asymmetrisch stehende Raute mit einer Induktion
Ordinate 2-22: Stark asymmetrisch stehende Raute mit zwei Induktionen
Ordinate 2-23: Stark asymmetrisch stehende Raute mit drei Induktionen
Ordinate 2-24: Stark asymmetrisch stehende Raute mit vier Induktionen
Ordinate 2-25: Stark asymmetrisch stehende Raute mit fünf und mehr Induktionen

### Abszisse 2-43: Stark asymmetrische, stehende Raute mit einer gespreizten Ecke zum Fünfeck

Ordinate 2-11: Stark asymmetrisch stehende Raute mit einer gespreizten Ecke zum Fünfeck und mit leichter Verlängerung der senkrechten Achse
Ordinate 2-12: Stark asymmetrisch stehende Raute mit einer gespreizten Ecke zum Fünfeck und mit Verlängerung der senkrechten Achse
Ordinate 2-13: Stark asymmetrisch stehende Raute mit einer gespreizten Ecke zum Fünfeck und mit startker Verlängerung der senkrechten Achse
Ordinate 2-14: Stark asymmetrisch stehende Raute mit einer gespreizten Ecke zum Fünfeck und mit extremer Verlängerung der Achse
Ordinate 2-15: Stark asymmetrisch stehende Raute mit einer gespreizten Ecke zum Fünfeck und mit unregelmäßiger Verlängerung der Achse
Ordinate 2-21: Stark asymmetrisch stehende Raute mit einer gespreizten Ecke zum Fünfeck und mit einer Induktion
Ordinate 2-22: Stark asymmetrisch stehende Raute mit einer gespreizten Ecke zum Fünfeck und mit zwei Induktionen
Ordinate 2-23: Stark asymmetrisch stehende Raute mit einer gespreizten Ecke zum Fünfeck und mit drei Induktionen
Ordinate 2-24: Stark asymmetrisch stehende Raute mit einer gespreizten Ecke zum Fünfeck und mit vier Induktionen
Ordinate 2-25: Stark asymmetrisch stehende Raute mit einer gespreizten Ecke zum Fünfeck und mit fünf und mehr Induktionen

### Abszisse 2-50: Gleichseitiges Fünfeck

Ordinate 2-11: Gleichseitiges Fünfeck mit leichter Verlängerung der senkrechten Symmetrieachse
Ordinate 2-12: Gleichseitiges Fünfeck mit Verlängerung der senkrechten Symmetrieachse
Ordinate 2-13: Gleichseitiges Fünfeck mit starker Verlängerung der senkrechten Symmetrieachse
Ordinate 2-14: Gleichseitiges Fünfeck mit extremer Verlängerung der Symmetrieachse
Ordinate 2-15: Gleichseitiges Fünfeck mit unregelmäßiger Verlängerung der Symmetrieachse
Ordinate 2-21: Gleichseitiges Fünfeck mit einer Induktion
Ordinate 2-22: Gleichseitiges Fünfeck mit zwei Induktionen
Ordinate 2-23: Gleichseitiges Fünfeck mit drei Induktionen
Ordinate 2-24: Gleichseitiges Fünfeck mit vier Induktionen
Ordinate 2-25: Gleichseitiges Fünfeck mit fünf und mehr Induktionen

### Abszisse 2-51: Asymmetrisches Fünfeck

Ordinate 2-11: Asymmetrisches Fünfeck mit leichter Verlängerung der senkrechten Achse
Ordinate 2-12: Asymmetrisches Fünfeck mit Verlängerung der senkrechten Achse
Ordinate 2-13: Asymmetrisches Fünfeck mit starker Verlängerung der senkrechten Achse
Ordinate 2-14: Asymmetrisches Fünfeck mit extremer Verlängerung der Achse
Ordinate 2-15: Asymmetrisches Fünfeck mit unregelmäßiger Verlängerung längerung der Achse
Ordinate 2-21: Asymmetrisches Fünfeck mit einer Induktion
Ordinate 2-22: Asymmetrisches Fünfeck mit zwei Induktionen
Ordinate 2-23: Asymmetrisches Fünfeck mit drei Induktionen
Ordinate 2-24: Asymmetrisches Fünfeck mit vier Induktionen
Ordinate 2-25: Asymmetrisches Fünfeck mit fünf und mehr Induktionen

### Abszisse 2-52: Stark asymmetrisches Fünfeck

Ordinate 2-11: Stark asymmetrisches Fünfeck mit leichter Verlängerung der senkrechten Symmetrieachse
Ordinate 2-12: Stark asymmetrisches Fünfeck mit Verlängerung der senkrechten Symmetrieachse
Ordinate 2-13: Stark asymmetrisches Fünfeck mit starker Verlängerung der senkrechten Symmetrieachse
Ordinate 2-14: Stark asymmetrisches Fünfeck mit extremer Verlängerung der Symmetrieachse
Ordinate 2-15: Stark asymmetrisches Fünfeck mit unregelmäßiger Verlängerung der Symmetrieachse
Ordinate 2-21: Stark asymmetrisches Fünfeck mit einer Induktion
Ordinate 2-22: Stark asymmetrisches Fünfeck mit zwei Induktionen
Ordinate 2-23: Stark asymmetrisches Fünfeck mit drei Induktionen
Ordinate 2-24: Stark asymmetrisches Fünfeck mit vier Induktionen
Ordinate 2-25: Stark asymmetrisches Fünfeck mit fünf und mehr Induktionen

### Abszisse 2-53: Einfach symmetrisches Fünfeck

Ordinate 2-11: Einfach symmetrisches Fünfeck mit leichter Verlängerung der senkrechten Symmetrieachse
Ordinate 2-12: Einfach symmetrisches Fünfeck mit Verlängerung der senkrechten Symmetrieachse
Ordinate 2-13: Einfach symmetrisches Fünfeck mit starker Verlängerung gerung der senkrechten Symmetrieachse
Ordinate 2-14: Einfach symmetrisches Fünfeck mit extremer Verlängerung der Symmetrieachse
Ordinate 2-15: Einfach symmetrisches Fünfeck mit unregelmäßiger Verlängerung der Symmetrieachse
Ordinate 2-21: Einfach symmetrisches Fünfeck mit einer Induktion
Ordinate 2-22: Einfach symmetrisches Fünfeck mit zwei Induktionen
Ordinate 2-23: Einfach symmetrisches Fünfeck mit drei Induktionen
Ordinate 2-24: Einfach symmetrisches Fünfeck mit vier Induktionen
Ordinate 2-25: Einfach symmetrisches Fünfeck mit fünf und mehr Induktionen

### Abszisse 2-60: Gleichseitiges Sechseck

Ordinate 2-11: Gleichseitiges Sechseck mit leichter Verlängerung der senkrechten Symmetrieachse
Ordinate 2-12: Gleichseitiges Sechseck mit Verlängerung der senkrechten Symmetrieachse
Ordinate 2-13: Gleichseitiges Sechseck mit starker Verlängerung der senkrechten Symmetrieachse
Ordinate 2-14: Gleichseitiges Sechseck mit extremer Verlängerung der Symmetrieachse
Ordinate 2-15: Gleichseitiges Sechseck mit unregelmäßiger Verlängerung der Symmetrieachse
Ordinate 2-21: Gleichseitiges Sechseck mit einer Induktion
Ordinate 2-22: Gleichseitiges Sechseck mit zwei Induktionen
Ordinate 2-23: Gleichseitiges Sechseck mit drei Induktionen
Ordinate 2-24: Gleichseitiges Sechseck mit vier Induktionen
Ordinate 2-25: Gleichseitiges Sechseck mit fünf und mehr Induktionen

### Abszisse 2-61: Doppelt symmetrisches Sechseck

Ordinate 2-11: Doppelt symmetrisches Sechseck mit leichter Verlängerung der senkrechten Symmetrieachse
Ordinate 2-12: Doppelt symmetrisches Sechseck mit Verlängerung der senkrechten Symmetrieachse
Ordinate 2-13: Doppelt symmetrisches Sechseck mit starker Verlängerung der senkrechten Symmetrieachse
Ordinate 2-14: Doppelt symmetrisches Sechseck mit extremer Verlängerung der Symmetrieachse
Ordinate 2-15: Doppelt symmetrisches Sechseck mit unregelmäßiger Verlängerung der Symmetrieachse
Ordinate 2-21: Doppelt symmetrisches Sechseck mit einer Induktion
Ordinate 2-22: Doppelt symmetrisches Sechseck mit zwei Induktionen
Ordinate 2-23: Doppelt symmetrisches Sechseck mit drei Induktionen
Ordinate 2-24: Doppelt symmetrisches Sechseck mit vier Induktionen
Ordinate 2-25: Doppelt symmetrisches Sechseck mit fünf und mehr Induktionen

### Abszisse 2-62: Doppelt symmetrisches Sechseck mit verlängerter Längsachse

Ordinate 2-11: Doppelt symmetrisches Sechseck mit verlängerter Längsachse und mit leichter Verlängerung der senkrechten Symmetrieachse
Ordinate 2-12: Doppelt symmetrisches Sechseck mit verlängerter Längsachse und mit Verlängerung der senkrechten Symmetrieachse
Ordinate 2-13: Doppelt symmetrisches Sechseck mit verlängerter Längsachse und mit starker Verlängerung der senkrechten Symmetrieachse
Ordinate 2-14: Doppelt symmetrisches Sechseck mit verlängerter Längsachse und mit extremer Verlängerung der Symmetrieachse
Ordinate 2-15: Doppelt symmetrisches Sechseck mit verlängerter Längsachse und mit unregelmäßiger Verlängerung der Symmetrieachse
Ordinate 2-21: Doppelt symmetrisches Sechseck mit verlängerter Längsachse und mit einer Induktion
Ordinate 2-22: Doppelt symmetrisches Sechseck mit verlängerter Längsachse und mit zwei Induktionen
Ordinate 2-23: Doppelt symmetrisches Sechseck mit verlängerter Längsachse und mit drei Induktionen
Ordinate 2-24: Doppelt symmetrisches Sechseck mit verlängerter Längsachse und mit vier Induktionen
Ordinate 2-25: Doppelt symmetrisches Sechseck mit verlängerter Längsachse und mit fünf und mehr Induktionen

### Abszisse 2-63: Asymmetrisches Sechseck

Ordinate 2-11: Asymmetrisches Sechseck mit leichter Verlängerung der senkrechten Achse
Ordinate 2-12: Asymmetrisches Sechseck mit Verlängerung der senkrechten Achse
Ordinate 2-13: Asymmetrisches Sechseck mit starker Verlängerung der senkrechten Achse
Ordinate 2-14: Asymmetrisches Sechseck mit extremer Verlängerung der Achse
Ordinate 2-15: Asymmetrisches Sechseck mit unregelmäßiger Verlängerung der Achse
Ordinate 2-21: Asymmetrisches Sechseck mit einer Induktion
Ordinate 2-22: Asymmetrisches Sechseck mit zwei Induktionen
Ordinate 2-23: Asymmetrisches Sechseck mit drei Induktionen
Ordinate 2-24: Asymmetrisches Sechseck mit vier Induktionen
Ordinate 2-25: Asymmetrisches Sechseck mit fünf und mehr Induktionen

### Abszisse 2-64: Gleichseitige Vielecke, hier: Siebeneck

Ordinate 2-11: Gleichseitige Achtecke
Ordinate 2-12: Gleichseitige Neunecke
Ordinate 2-13: Gleichseitige Zehnecke
Ordinate 2-14: Gleichseitige Zwölfecke
Ordinate 2-15: Gleichseitige Zwanzigecke
Ordinate 2-21: Gleichseitige Vielecke mit einer Induktion
Ordinate 2-22: Gleichseitige Vielecke mit zwei Induktionen
Ordinate 2-23: Gleichseitige Vielecke mit drei Induktionen
Ordinate 2-24: Gleichseitige Vielecke mit vier Induktionen
Ordinate 2-25: Gleichseitige Vielecke mit fünf und mehr Induktionen

### Abszisse 2-65: Ungleichseitige, symmetrische Vielecke mit verlängerter Längsachse

Ordinate 2-11: Ungleichseitige, symmetrische Achtecke
Ordinate 2-12: Ungleichseitige, symmetrische Neunecke
Ordinate 2-13: Ungleichseitige, symmetrische Zehnecke
Ordinate 2-14: Ungleichseitige, symmetrische Zwölfecke
Ordinate 2-15: Ungleichseitige, symmetrische Zwanzigecke
Ordinate 2-21: Ungleichseitige, symmetrische Vielecke mit einer Induktion
Ordinate 2-22: Ungleichseitige, symmetrische Vielecke mit zwei Induktionen
Ordinate 2-23: Ungleichseitige symmetrische Vielecke mit drei Induktionen
Ordinate 2-24: Ungleichseitige, symmetrische Vielecke mit vier Induktionen
Ordinate 2-25: Ungleichseitige, symmetrische Vielecke mit fünf und mehr Induktionen

### Abszisse 2-66: Teilsymmetrische Vielecke mit unbestimmten Eckenzahlen

Ordinate 2-11: Teilsymmetrische Vielecke mit unbestimmten Eckenzahlen und einer schwach verlängerten Längsachse
Ordinate 2-12: Teilsymmetrische Vielecke mit unbestimmten Eckenzahlen und einer verlängerten Längsachse
Ordinate 2-13: Teilsymmetrische Vielecke mit unbestimmten Eckenzahlen und einer stark verlängerten Längsachse
Ordinate 2-14: Teilsymmetrische Vielecke mit unbestimmten Eckenzahlen und einer sehr stark verlängerten Längsachse
Ordinate 2-15: Teilsymmetrische Vielecke mit unbestimmten Eckenzahlen und einer extrem verlängerten Längsachse
Ordinate 2-21: Teilsymmetrische Vielecke mit unbestimmten Eckenzahlen und einer Induktion
Ordinate 2-22: Teilsymmetrische Vielecke mit unbestimmten Eckenzahlen und zwei Induktionen
Ordinate 2-23: Teilsymmetrische Vielecke mit unbestimmten Eckenzahlen und drei Induktionen
Ordinate 2-24: Teilsymmetrische Vielecke mit unbestimmten Eckenzahlen und vier Induktionen
Ordinate 2-25: Teilsymmetrische Vielecke mit unbestimmten Eckenzahlen und fünf und mehr Induktionen

### Abszisse 2-70: Voller Kreis

Ordinate 2-11: Voller Kreis mit leichter Verlängerung der senkrechten Symmetrieachse
Ordinate 2-12: Voller Kreis mit Verlängerung der senkrechten Symmetrieachse
Ordinate 2-13: Voller Kreis mit starker Verlängerung der senkrechten Symmetrieachse
Ordinate 2-14: Voller Kreis mit extremer Verlängerung der Symmetrieachse
Ordinate 2-15: Voller Kreis mit unregelmäßiger Verlängerung der Symmetrieachse
Ordinate 2-21: Voller Kreis mit einer leichten Induktion
Ordinate 2-22: Voller Kreis mit einer stärkeren Induktion
Ordinate 2-23: Voller Kreis mit einer starken Induktion
Ordinate 2-24: Voller Kreis mit einer sehr starken Induktion
Ordinate 2-25: Voller Kreis mit einer deformierenden Induktion

### Abszisse 2-71: Kreisförmige Figur

Ordinate 2-11: Kreisförmige einfach symmetrische Figur mit leichter Verlängerung der senkrechten Symmetrieachse
Ordinate 2-12: Kreisförmige, einfach symmetrische Figur mit Verlängerung der senkrechten Symmetrieachse
Ordinate 2-13: Kreisförmige, einfach symmetrische Figur mit starker Verlängerung der senkrechten Symmetrieachse
Ordinate 2-14: Kreisförmige, einfach symmetrische Figur mit extremer Verlängerung der Symmetrieachse
Ordinate 2-15: Kreisförmige, einfach symmetrische Figur mit unregelmäßiger Verlängerung der Symmetrieachse
Ordinate 2-21: Kreisförmige, einfach symmetrische Figur mit einer leichten Induktion
Ordinate 2-22: Kreisförmige, einfach symmetrische Figur mit einer stärkeren Induktion
Ordinate 2-23: Kreisförmige einfach symmetrische Figur mit einer starken Induktion
Ordinate 2-24: Kreisförmige, einfach symmetrische Figur mit einer sehr starken Induktion
Ordinate 2-25: Kreisförmige, einfach symmetrische Figur mit einer deformierenden Induktion

### Abszisse 2-72: Reduzierter Kreis

Ordinate 2-11: Kreisförmige Figur mit Bildung eines kleinen Innenhofes
Ordinate 2-12: Kreisförmige Figur mit Bildung eines Außenhofes
Ordinate 2-13: Kreisförmige Figur mit Bildung zweier Außenhöfe
Ordinate 2-14: Kreisförmige Figur mit Bildung dreier Außenhöfe
Ordinate 2-15: Kreisförmige Figur mit Bildung von vier und mehrerer Außenhöfe
Ordinate 2-21: Kreisförmige Figur mit einer tiefen Induktion
Ordinate 2-22: Kreisförmige Figur mit zwei tiefen Induktionen
Ordinate 2-23: Kreisförmige Figur mit drei tiefen Induktionen
Ordinate 2-24: Kreisförmige Figur mit vier tiefen Induktionen
Ordinate 2-25: Kreisförmige Figur mit fünf und mehr tiefen Induktionen

### Abszisse 2-73: Leerer Kreis

Ordinate 2-11: Leerer Kreis mit einer fingerartigen Verlängerung
Ordinate 2-12: Leerer Kreis mit einer längeren, fingerartigen Verlängerung
Ordinate 2-13: Leerer Kreis mit einer langen, fingerartigen Verlängerung
Ordinate 2-14: Leerer Kreis mit einer sehr langen, fingerartigen Verlängerung
Ordinate 2-15: Leerer Kreis mit einer linienförmig, fingerartigen Verlängerung
Ordinate 2-21: Leerer Kreis mit einem Halbschnitt
Ordinate 2-22: Leerer Kreis mit einem Ganzschnitt
Ordinate 2-23: Leerer Kreis mit zwei Ganzschnitten
Ordinate 2-24: Leerer Kreis mit einem Kreuzschnitt
Ordinate 2-25: Leerer Kreis mit einem Vielfachschnitt

### Tafel 3 Textur B:

Formen, entsprechend den Formen der Tafel 1 und/oder 2, wie sie während einer Kristallisation entstehen und sich einer Grundform oder Grundstruktur gemäß den Tafeln 1 und/oder 2 räumlich assoziieren.

### Abszisse 3-00: Punkt

Ordinate 3-11: In einer Linie verdoppelter Punkt
Ordinate 3-12: In einer Linie vervielfachter Punkt
Ordinate 3-13: Zwei, in einer Linie liegende Punktgruppen
Ordinate 3-14: Lange, regelmäßige Punktgruppe
Ordinate 3-15 Lange, unregelmäßige Punktgruppe
Ordinate 3-21: Vergrößerter Punkt mit einer Einkerbung (Induktion)
Ordinate 3-22: Vergrößerter Punkt mit zwei Induktionen
Ordinate 3-23: Vergrößerter Punkt mit drei Induktionen
Ordinate 3-24: Vergrößerter Punkt mit vier Induktionen
Ordinate 3-25: Vergrößerter Punkt mit fünf und mehr Induktionen.

### Abszisse 3-01: in Längsrichtung und ansatzweise in Querrichtung ausgedehnter Punkt

Ordinate 3-11: Ausdehnung des Punktes zu einem symmetrischen Oval
Ordinate 3-12: Längsausdehnung des Punktes zu einem symmetrischen Oval
Ordinate 3-13: Weiter Längsausdehnung des Punktes zu einem symmetrischen Oval
Ordinate 3-14: Auflösung der korpuskulären Struktur des Oval
Ordinate 3-15: Unregelmäßige Längsausdehung des Ovals zur räumlichen Linie
Ordinate 3-21: Einfache Induktion der Linie (Bogen)
Ordinate 3-22: Zweifache Induktion der Linie (Schlange)
Ordinate 3-23: Dreifache Induktion der Linie (Zickzack)
Ordinate 3-24: Vierfache Induktion der Linie mit leichter Glättung der Ecken
Ordinate 3-25: Fünf- und mehrfache Induktion der Linie mit Glättung der Ecken

### Abszisse 3-10: in Längsrichtung ausgedehnter Punkt: Linie

Ordinate 3-11: Gerade
Ordinate 3-12: einfach unterbrochene Gerade
Ordinate 3-13: Mehrfach unterbrochene Gerade aus regelmäßigen Geraderesten
Ordinate 3-14: Mehrfach unterbrochene Gerade aus unregelmäßigen Geraderesten
Ordinate 3-15: Gebrochene Gerade aus vielfachen, unregelmäßigen Geraderesten
Ordinate 3-21: Gerade mit einer winkeligen Induktion
Ordinate 3-22: Gerade mit zwei winkeligen Induktionen
Ordinate 3-23: Gerade mit drei und mehr winkeligen Induktionen
Ordinate 3-24: Gerade mit vier und mehr winkeligen Induktionen mit Überstand am Scheitel
Ordinate 3-25: Auflösende Gerade mit fünf und mehr winkeligen Induktionen

### Abszisse 3-11: Linie mit freiwinkeliger Abzweigung

Ordinate 3-11: Gerade mit spitzwinkeliger Abzweigung
Ordinate 3-12: Gerade mit sehr spitzwinkeliger Abzweigung
Ordinate 3-13: Gerade mit extrem spitzwinkeliger Abzweigung
Ordinate 3-14: Gerade mit in die Gerade einlaufender Abzweigung
Ordinate 3-15: Unregelmäßig ausgedehnte Gerade mit in sie einlaufender Abzweigung
Ordinate 3-21: Gerade mit spitzwinkeliger Abzweigung und einer Induktion
Ordinate 3-22: Gerade mit spitzwinkeliger Abzweigung und zwei Induktionen
Ordinate 3-23: Gerade mit spitzwinkeliger Abzweigung und drei Induktionen
Ordinate 3-24: Gerade mit spitzwinkeliger Abzweigung ung vier Induktionen
Ordinate 3-25: Gerade mit spitzwinkeliger Abzweigung und fünf oder mehr Induktionen

### Abszisse 3-12: Linie mit rechtwinkeliger Abzweigung

Ordinate 3-11: Gerade mit einer rechtwinkeligen, längeren Abzweigung
Ordinate 3-12: Gerade mit mehreren rechtwinkeligen Abzweigungen
Ordinate 3-13: Gerade mit mehreren, rechtwinkeligen, gegenständigen Abzweigungen
Ordinate 3-14: Gerade mit mehreren, rechtwinkeligen, wechselständigen Abzweigungen
Ordinate 3-15: Gerade mit mehreren, rechtwinkeligen, unterschiedlichständigen Abzweigungen
Ordinate 3-21: Gerade mit zwei rechtwinkeligen Brechungen
Ordinate 3-22: Gerade mit drei rechtwinkligen Brechungen
Ordinate 3-23: Gerade mit vier rechtwinkligen Brechungen
Ordinate 3-24: Gerade mit fünf und mehr rechtwinkligen Brechungen
Ordinate 3-25: Gerade mit beliebig vielen rechtwinkligen Brechungen

### Abszisse 3-13: Linie mit gleichschenkliger Abzweigung

Ordinate 3-11: Symmetrisches Winkelgerade mit großem Winkel
Ordinate 3-12: Symmetrische Winkelgerade mit kleinem Winkel
Ordinate 3-13: Symmetrische Winkelgerade mit sehr kleinem Winkel
Ordinate 3-14: Symmetrisches Winkelgerade mit extrem kleinem Winkel
Ordinate 3-15: Symmetrische, unregelmäßig verlängerte Winkelgerade mit minimalem Winkel
Ordinate 3-21: Symmetrische Winkelgerade mit einer Induktion
Ordinate 3-22: Symmetrische Winkelgerade mit zwei Induktionen
Ordinate 3-23: Symmetrische Winkelgerade mit drei Induktionen
Ordinate 3-24: Symmetrisches Winkelgerade mit vier, symmetrischen Induktionen
Ordinate 3-25: Symmetrische Winkelgerade mit vier, symmetrisch reduzierenden Induktionen

### Abszisse 3-20: Gleichseitiges Dreieck

Ordinate 3-11; Gleichseitiges Dreieck mit leichter Verlängerung der senkrechten Symmetrieachse
Ordinate 3-12: Gleichseitiges Dreieck mit Verlängerung der senkrechten Symmetrieachse
Ordinate 3-13: Gleichseitiges Dreieck mit starker Verlängerung der senkrechten Symmetrieachse
Ordinate 3-14: Gleichseitiges Dreieck mit extremer Verlängerung der Symmetrieachse
Ordinate 3-15: Gleichseitiges Dreieck mit unregelmäßiger Verlängerung der Symmetrieachse
Ordinate 3-21: Gleichseitiges Dreieck mit einer Induktion
Ordinate 3-22: Gleichseitiges Dreieck mit zwei Induktionen
Ordinate 3-23: Gleichseitiges Dreieck mit drei Induktionen
Ordinate 3-24: Gleichseitiges Dreieck mit vier Induktionen
Ordinate 3-25: Gleichseitiges Dreieck mit fünf und mehr Induktionen

### Abszisse 3-21: Ungleichseitiges Dreieck

Ordinate 3-11: Ungleichseitiges Dreieck mit leichter Verlängerung der senkrechten Achse
Ordinate 3-12: Ungleichseitiges Dreieck mit Verlängerung der senkrechten Achse
Ordinate 3-13: Ungleichseitiges Dreieck mit starker Verlängerung der senkrechten Achse
Ordinate 3-14: Ungleichseitiges Dreieck mit extremer Verlängerung der Achse
Ordinate 3-15: Ungleichseitiges Dreieck mit unregelmäßiger Verlängerung der Achse
Ordinate 3-21: Ungleichseitiges Dreieck mit einer Induktion
Ordinate 3-22: Ungleichseitiges Dreieck mit zwei Induktionen
Ordinate 3-23: Ungleichseitiges Dreieck mit drei Induktionen
Ordinate 3-24: Ungleichseitiges Dreieck mit vier Induktionen
Ordinate 3-25: Ungleichseitiges Dreieck mit fünf und mehr Induktionen

### Abszisse 3-22: Rechtwinkeliges Dreieck

Ordinate 3-11; Rechtwinkeliges Dreieck mit leichter Verlängerung der senkrechten Achse
Ordinate 3-12: Rechtwinkeliges Dreieck mit Verlängerung der senkrechten Achse
Ordinate 3-13: Rechtwinkeliges Dreieck mit starker Verlängerung der senkrechten Achse
Ordinate 3-14: Rechtwinkeliges Dreieck mit extremer Verlängerung der Achse
Ordinate 3-15: Rechtwinkeliges Dreieck mit unregelmäßiger Verlängerung der Achse
Ordinate 3-21: Rechtwinkeliges Dreieck mit einer Induktion
Ordinate 3-22: Rechtwinkeliges Dreieck mit zwei Induktionen
Ordinate 3-23: Rechtwinkeliges Dreieck mit drei Induktionen
Ordinate 3-24: Rechtwinkeliges Dreieck mit vier Induktionen
Ordinate 3-25: Rechtwinkeliges Dreieck mit fünf und mehr Induktionen

### Abszisse 3-23: Spitzwinkeliges Dreieck

Ordinate 3-11: Spitzwinkeliges Dreieck mit leichter Verlängerung der senkrechten Achse
Ordinate 3-12: Spitzwinkeliges Dreieck mit Verlängerung der senkrechten Achse
Ordinate 3-13: Spitzwinkeliges Dreieck mit starker Verlängerung der senkrechten Achse
Ordinate 3-14: Spitzwinkeliges Dreieck mit extremer Verlängerung der Achse
Ordinate 3-15: Spitzwinkeliges Dreieck mit unregelmäßiger Verlängerung der Achse
Ordinate 3-21: Spitzwinkeliges Dreieck mit einer Induktion
Ordinate 3-22: Spitzwinkeliges Dreieck mit zwei Induktionen
Ordinate 3-23: Spitzwinkeliges Dreieck mit drei Induktionen
Ordinate 3-24: Spitzwinkeliges Dreieck mit vier Induktionen
Ordinate 3-25: Spitzwinkeliges Dreieck mit fünf und mehr Induktionen

### Abszisse 3-24: Spitzwinkeliges Dreieck mit einer gespreizten Ecke zum beginnenden Viereck

Ordinate 3-11: Spitzwinkeliges Dreieck mit leichter Verlängerung der senkrechten Achse
Ordinate 3-12: Spitzwinkeliges Dreieck mit Verlängerung der senkrechten Achse
Ordinate 3-13: Spitzwinkeliges Dreieck mit starker Verlängerung der senkrechten Achse
Ordinate 3-14: Spitzwinkeliges Dreieck mit extremer Verlängerung der Achse
Ordinate 3-15: Spitzwinkeliges Dreieck mit unregelmäßiger Verlängerung der Achse
Ordinate 3-21: Spitzwinkeliges Dreieck mit einer Induktion
Ordinate 3-22: Spitzwinkeliges Dreieck mit zwei Induktionen
Ordinate 3-23: Spitzwinkeliges Dreieck mit drei Induktionen
Ordinate 3-24: Spitzwinkeliges Dreieck mit vier Induktionen
Ordinate 3-25: Spitzwinkeliges Dreieck mit fünf und mehr Induktionen

### Abszisse 3-30: Gleichseitiges Viereck, Quadrat

Ordinate 3-11: Gleichseitiges Viereck mit leichter Verlängerung der senkrechten Symmetrieachse
Ordinate 3-12: Gleichseitiges Viereck mit Verlängerung der senkrechten Symmetrieachse
Ordinate 3-13: Gleichseitiges Viereck mit starker Verlängerung der senkrechten Symmetrieachse
Ordinate 3-14: Gleichseitiges Viereck mit extremer Verlängerung der Symmetrieachse
Ordinate 3-15: Gleichseitiges Viereck mit unregelmäßiger Verlängerung der Symmetrieachse
Ordinate 3-21: Gleichseitiges Viereck mit einer Induktion
Ordinate 3-22: Gleichseitiges Viereck mit zwei Induktionen
Ordinate 3-23: Gleichseitiges Viereck mit drei Induktionen
Ordinate 3-24: Gleichseitiges Viereck mit vier Induktionen
Ordinate 3-25: Gleichseitiges Viereck mit fünf und mehr Induktionen

### Abszisse 3-31: Regelmäßiges, symmetrisches Viereck, Rechteck

Ordinate 3-11: Regelmäßiges Viereck mit leichter Verlängerung der senkrechten Symmetrieachse
Ordinate 3-12: Regelmäßiges Viereck mit Verlängerung der senkrechten Symmetrieachse
Ordinate 3-13: Regelmäßiges Viereck mit starker Verlängerung der senkrechten Symmetrieachse
Ordinate 3-14: Regelmäßiges Viereck mit extremer Verlängerung der Symmetrieachse
Ordinate 3-15: Regelmäßiges Viereck mit unregelmäßiger Verlängerung der Symmetrieachse
Ordinate 3-21: Regelmäßiges Viereck mit einer Induktion
Ordinate 3-22: Regelmäßiges Viereck mit zwei Induktionen
Ordinate 3-23: Regelmäßiges Viereck mit drei Induktionen
Ordinate 3-24: Regelmäßiges Viereck mit vier Induktionen
Ordinate 3-25: Regelmäßiges Viereck mit fünf und mehr Induktionen

### Abszisse 3-32: Unsymmetrisches Viereck

Ordinate 3-11: Unsymmetrisches Viereck mit leichter Verlängerung der senkrechten Symmetrieachse
Ordinate 3-12: Unsymmetrisches Viereck mit Verlängerung der senkrechten Symmetrieachse
Ordinate 3-13: Unsymmetrisches Viereck mit starker Verlängerung der senkrechten Symmetrieachse
Ordinate 3-14: Unsymmetrisches Viereck mit extremer Verlängerung der Symmetrieachse
Ordinate 3-15: Unsymmetrisches Viereck mit unregelmäßiger Verlängerung der Symmetrieachse
Ordinate 3-21: Unsymmetrisches Viereck mit eine Induktion
Ordinate 3-22: Unsymmetrisches Viereck mit zwei Induktionen
Ordinate 3-23: Unsymmetrisches Viereck mit drei Induktionen
Ordinate 3-24: Unsymmetrisches Viereck mit vier Induktionen
Ordinate 3-25: Unsymmetrisches Viereck mit fünf und mehr Induktionen

### Abszisse 3-33: Stark unsymmetrisches Viereck

Ordinate 3-11: Stark unsymmetrisches Viereck mit leichter Verlängerung der senkrechten Symmetrieachse
Ordinate 3-12: Stark unsymmetrisches Viereck mit Verlängerung der senkrechten Symmetrieachse
Ordinate 3-13: Stark unsymmetrisches Viereck mit starker Verlängerung der senkrechten Symmetrieachse
Ordinate 3-14: Stark unsymmetrisches Viereck mit extremer Verlängerung der Symmetrieachse
Ordinate 3-15: Stark unsymmetrisches Viereck mit Stark unregelmäßiger Verlängerung der Symmetrieachse
Ordinate 3-21: Stark unsymmetrisches Viereck mit einer Induktion
Ordinate 3-22: Stark unsymmetrisches Viereck mit zwei Induktionen
Ordinate 3-23: Stark unsymmetrisches Viereck mit drei Induktionen
Ordinate 3-24: Stark unsymmetrisches Viereck mit vier Induktionen
Ordinate 3-25: Stark unsymmetrisches Viereck mit fünf und mehr Induktionen

### Abszisse 3-34: Liegende Raute

Ordinate 3-11: Liegende Raute mit leichter Verlängerung der senkrechten Symmetrieachse
Ordinate 3-12: Liegende Raute mit Verlängerung der senkrechten Symmetrieachse
Ordinate 3-13: Liegende Raute mit starker Verlängerung der senkrechten Symmetrieachse
Ordinate 3-14: Liegende Raute mit extremer Verlängerung der Symmetrieachse
Ordinate 3-15: Liegende Raute mit unregelmäßiger Verlängerung der Symmetrieachse
Ordinate 3-21: Liegende Raute mit einer Induktion
Ordinate 3-22: Liegende Raute mit zwei Induktionen
Ordinate 3-23: Liegende Raute mit drei Induktionen
Ordinate 3-24: Liegende Raute mit vier Induktionen
Ordinate 3-25: Liegende Raute mit fünf und mehr Induktionen

### Abszisse 3-35: Trapez

Ordinate 3-11: Trapez mit leichter Verlängerung der senkrechten Symmetrieachse
Ordinate 3-12: Trapez mit Verlängerung der senkrechten Symmetrieachse
Ordinate 3-13: Trapez mit starker Verlängerung der senkrechten Symmetrieachse
Ordinate 3-14: Trapez mit extremer Verlängerung der Symmetrieachse
Ordinate 3-15: Trapez mit unregelmäßiger Verlängerung der Symmetrieachse
Ordinate 3-21: Trapez mit einer Induktion
Ordinate 3-22: Trapez mit zwei Induktionen
Ordinate 3-23: Trapez mit drei Induktionen
Ordinate 3-24: Trapez mit vier Induktionen
Ordinate 3-25: Trapez mit fünf und mehr Induktionen

### Abszisse 3-40: Stehende Raute

Ordinate 3-11: Stehende Raute mit leichter Verlängerung der senkrechten Symmetrieachse
Ordinate 3-12: Stehende Raute mit Verlängerung der senkrechten Symmetrieachse
Ordinate 3-13: Stehende Raute mit starker Verlängerung der senkrechten Symmetrieachse
Ordinate 3-14: Stehende Raute mit extremer Verlängerung der Symmetrieachse
Ordinate 3-15: Stehende Raute mit unregelmäßiger Verlängerung der Symmetrieachse
Ordinate 3-21: Stehende Raute mit einer Induktion
Ordinate 3-22: Stehende Raute mit zwei Induktionen
Ordinate 3-23: Stehende Raute mit drei Induktionen
Ordinate 3-24: Stehende Raute mit vier Induktionen
Ordinate 3-25: Stehende Raute mit fünf und mehr Induktionen

### Abszisse 3-41: Asymmetrische, stehende Raute

Ordinate 3-11: Asymmetrisch stehende Raute mit leichter Verlängerung der senkrechten Achse
Ordinate 3-12: Asymmetrisch stehende Raute mit Verlängerung der senkrechten Achse
Ordinate 3-13: Asymmetrisch stehende Raute mit starker Verlängerung der senkrechten Achse
Ordinate 3-14: Asymmetrisch stehende Raute extremer Verlängerung der Achse
Ordinate 3-15: Asymmetrisch stehende Raute mit unregelmäßiger Verlängerung der Achse
Ordinate 3-21: Asymmetrisch stehende Raute mit einer Induktion Ordinate 3-22: Asymmetrisch stehende Raute mit zwei Induktionen
Ordinate 3-23: Asymmetrisch stehende Raute mit drei Induktionen
Ordinate 3-24: Asymmetrisch stehende Raute mit vier Induktionen
Ordinate 3-25: Asymmetrisch stehende Raute, mit fünf und mehr Induktionen

### Abszisse 3-42: Stark asymmetrische, stehende Raute

Ordinate 3-11: Stark asymmetrisch stehende Raute mit leichter Verlängerung der senkrechten Achse
Ordinate 3-12: Stark asymmetrisch stehende Raute mit Verlängerung der senkrechten Achse
Ordinate 3-13: Stark asymmetrisch stehende Raute mit starker Verlängerung der senkrechten Achse
Ordinate 3-14: Stark asymmetrisch stehende Raute mit extremer Verlängerung der Achse
Ordinate 3-15: Stark asymmetrisch stehende Raute mit unregelmäßiger Verlängerung der Achse
Ordinate 3-21: Stark asymmetrisch stehende Raute mit einer Induktion
Ordinate 3-22: Stark asymmetrisch stehende Raute mit zwei Induktionen
Ordinate 3-23: Stark asymmetrisch stehende Raute mit drei Induktionen
Ordinate 3-24: Stark asymmetrisch stehende Raute mit vier Induktionen
Ordinate 3-25: Stark asymmetrisch stehende Raute mit fünf und mehr Induktionen

### Abszisse 3-43: Stark asymmetrische, stehende Raute mit einer gespreizten Ecke zum Fünfeck

Ordinate 3-11: Stark asymmetrisch stehende Raute mit einer gespreizten Ecke zum Fünfeck und mit leichter Verlängerung der senkrechten Achse
Ordinate 3-12: Stark asymmetrisch stehende Raute mit mit einer gespreizten Ecke zum Fünfeck und mit Verlängerung der senkrechten Achse
Ordinate 3-13: Stark asymmetrisch stehende Raute mit einer gespreizten Ecke zum Fünfeck und mit starker Verlängerung der senkrechten Achse
Ordinate 3-14: Stark asymmetrisch stehende Raute mit einer gespreizten Ecke zum Fünfeck und mit extremer Verlängerung der Achse
Ordinate 3-15: Stark asymmetrisch stehende Raute mit einer gespreizten Ecke zum Fünfeck und mit unregelmäßiger Verlängerung der Achse
Ordinate 3-21: Stark asymmetrisch stehende Raute mit einer gespreizten Ecke zum Fünfeck und mit einer Induktion
Ordinate 3-22: Stark asymmetrisch stehende Raute mit einer gespreizten Ecke zum Fünfeck und mit zwei Induktionen
Ordinate 3-23: Stark asymmetrisch stehende Raute mit einer gespreizten Ecke zum Fünfeck und mit drei Induktionen
Ordinate 3-24: Stark asymmetrisch stehende Raute mit einer gespreizten Ecke zum Fünfeck und mit vier Induktionen
Ordinate 3-25: Stark asymmetrisch stehende Raute mit einer gespreizten Ecke zum Fünfeck und mit fünf und mehr Induktionen

### Abszisse 3-50: Gleichseitiges Fünfeck

Ordinate 3-11: Gleichseitiges Fünfeck mit leichter Verlängerung der senkrechten Symmetrieachse
Ordinate 3-12: Gleichseitiges Fünfeck mit Verlängerung der senkrechten Symmetrieachse
Ordinate 3-13: Gleichseitiges Fünfeck mit starker Verlängerung der senkrechten Symmetrieachse
Ordinate 3-14: Gleichseitiges Fünfeck mit extremer Verlängerung der Symmetrieachse
Ordinate 3-15: Gleichseitiges Fünfeck mit unregelmäßiger Verlängerung der Symmetrieachse
Ordinate 3-21: Gleichseitiges Fünfeck mit einer Induktion
Ordinate 3-22: Gleichseitiges Fünfeck mit zwei Induktionen
Ordinate 3-23: Gleichseitiges Fünfeck mit drei Induktionen
Ordinate 3-24: Gleichseitiges Fünfeck mit vier Induktionen
Ordinate 3-25: Gleichseitiges Fünfeck mit fünf und mehr Induktionen

### Abszisse 3-51: Asymmetrisches Fünfeck

Ordinate 3-11: Asymmetrisches Fünfeck mit leichter Verlängerung der senkrechten Achse
Ordinate 3-12: Asymmetrisches Fünfeck mit Verlängerung der senkrechten Achse
Ordinate 3-13: Asymmetrisches Fünfeck mit starker Verlängerung der senkrechten Achse
Ordinate 3-14: Asymmetrisches Fünfeck mit extremer Verlängerung der Achse
Ordinate 3-15: Asymmetrisches Fünfeck mit unregelmäßiger Verlängerung der Achse
Ordinate 3-21: Asymmetrisches Fünfeck mit einer Induktion
Ordinate 3-22: Asymmetrisches Fünfeck mit zwei Induktionen
Ordinate 3-23: Asymmetrisches Fünfeck mit drei Induktionen
Ordinate 3-24: Asymmetrisches Fünfeck mit vier Induktionen
Ordinate 3-25: Asymmetrisches Fünfeck mit fünf und mehr Induktionen

### Abszisse 3-52: Stark asymmetrisches Fünfeck

Ordinate 3-11: Stark asymmetrisches Fünfeck mit leichter Verlängerung der senkrechten Symmetrieachse
Ordinate 3-12: Stark asymmetrisches Fünfeck mit Verlängerung der senkrechten Symmetrieachse
Ordinate 3-13: Stark asymmetrisches Fünfeck mit starker Verlängerung der senkrechten Symmetrieachse
Ordinate 3-14: Stark asymmetrisches Fünfeck mit extremer Verlängerung der Symmetrieachse
Ordinate 3-15: Stark asymmetrisches Fünfeck mit unregelmäßiger Verlängerung der Symmetrieachse
Ordinate 3-21: Stark asymmetrisches Fünfeck mit einer Induktion
Ordinate 3-22: Stark asymmetrisches Fünfeck mit zwei Induktionen
Ordinate 3-23: Stark asymmetrisches Fünfeck mit drei Induktionen
Ordinate 3-24: Stark asymmetrisches Fünfeck mit vier Induktionen
Ordinate 3-25: Stark asymmetrisches Fünfeck mit fünf und mehr Induktionen

### Abszisse 3-53: Einfach symmetrisches Fünfeck

Ordinate 3-11: Einfach symmetrisches Fünfeck mit leichter Verlängerung der senkrechten Symmetrieachse
Ordinate 3-12: Einfach symmetrisches Fünfeck mit Verlängerung der senkrechten Symmetrieachse
Ordinate 3-13: Einfach symmetrisches Fünfeck mit starker Verlängerung der senkrechten Symmetrieachse
Ordinate 3-14: Einfach symmetrisches Fünfeck mit extremer Verlängerung der Symmetrieachse
Ordinate 3-15: Einfach symmetrisches Fünfeck mit unregelmäßiger Verlängerung der Symmetrieachse
Ordinate 3-21: Einfach symmetrisches Fünfeck mit einer Induktion
Ordinate 3-22: Einfach symmetrisches Fünfeck mit zwei Induktionen
Ordinate 3-23: Einfach symmetrisches Fünfeck mit drei Induktionen
Ordinate 3-24: Einfach symmetrisches Fünfeck mit vier Induktionen
Ordinate 3-25: Einfach symmetrisches Fünfeck mit fünf und mehr Induktionen

### Abszisse 3-60: Gleichseitiges Sechseck

Ordinate 3-11: Gleichseitiges Sechseck mit leichter Verlängerung der senkrechten Symmetrieachse
Ordinate 3-12: Gleichseitiges Sechseck mit Verlängerung der senkrechten Symmetrieachse
Ordinate 3-13: Gleichseitiges Sechseck mit starker Verlängerung der senkrechten Symmetrieachse
Ordinate 3-14: Gleichseitiges Sechseck mit extremer Verlängerung der Symmetrieachse
Ordinate 3-15: Gleichseitiges Sechseck mit unregelmäßiger Verlängerung der Symmetrieachse
Ordinate 3-21: Gleichseitiges Sechseck mit einer Induktion
Ordinate 3-22: Gleichseitiges Sechseck mit zwei Induktionen
Ordinate 3-23: Gleichseitiges Sechseck mit drei Induktionen
Ordinate 3-24: Gleichseitiges Sechseck mit vier Induktionen
Ordinate 3-25: Gleichseitiges Sechseck mit fünf und mehr Induktionen

### Abszisse 3-61: Doppelt symmetrisches Sechseck

Ordinate 3-11: Doppelt symmetrisches Sechseck mit leichter Verlängerung der senkrechten Symmetrieachse
Ordinate 3-12: Doppelt symmetrisches Sechseck mit Verlängerung der senkrechten Symmetrieachse
Ordinate 3-13: Doppelt symmetrisches Sechseck mit starker Verlängerung der senkrechten Symmetrieachse
Ordinate 3-14: Doppelt symmetrisches Sechseck mit extremer Verlängerung der Symmetrieachse
Ordinate 3-15: Doppelt symmetrisches Sechseck mit unregelmäßiger Verlängerung der Symmetrieachse
Ordinate 3-21: Doppelt symmetrisches Sechseck mit einer Induktion
Ordinate 3-22: Doppelt symmetrisches Sechseck mit zwei Induktionen
Ordinate 3-23: Doppelt symmetrisches Sechseck mit drei Induktionen
Ordinate 3-24: Doppelt symmetrisches Sechseck mit vier Induktionen
Ordinate 3-25: Doppelt symmetrisches Sechseck mit fünf und mehr Induktionen

### Abszisse 3-62: Doppelt symmetrisches Sechseck mit verlängerter Längsachse

Ordinate 3-11: Doppelt symmetrisches Sechseck mit verlängerter Längsachse und mit leichter Verlängerung der senkrechten Symmetrieachse
Ordinate 3-12: Doppelt symmetrisches Sechseck mit verlängerter Längsachse und mit Verlängerung der senkrechten Symmetrieachse
Ordinate 3-13: Doppelt symmetrisches Sechseck mit verlängerter Längsachse und mit starker Verlängerung der senkrechten Symmetrieachse
Ordinate 3-14: Doppelt symmetrisches Sechseck mit verlängerter Längsachse und mit extremer Verlängerung der Symmetrieachse
Ordinate 3-15: Doppelt symmetrisches Sechseck mit verlängerter Längsachse und mit unregelmäßiger Verlängerung der Symmetrieachse
Ordinate 3-21: Doppelt symmetrisches Sechseck mit verlängerter Längsachse und mit einer Induktion
Ordinate 3-22: Doppelt symmetrisches Sechseck mit verlängerter Längsachse und mit zwei Induktionen
Ordinate 3-23: Doppelt symmetrisches Sechseck mit verlängerter Längsachse und mit drei Induktionen
Ordinate 3-24: Doppelt symmetrisches Sechseck mit verlängerter Längsachse und mit vier Induktionen
Ordinate 3-25: Doppelt symmetrisches Sechseck mit verlängerter Längsachse und mit fünf und mehr Induktionen

### Abszisse 3-63: Asymmetrisches Sechseck

Ordinate 3-11: Asymmetrisches Sechseck mit leichter Verlängerung der senkrechten Achse
Ordinate 3-12: Asymmetrisches Sechseck mit Verlängerung der senkrechten Achse
Ordinate 3-13: Asymmetrisches Sechseck mit starker Verlängerung der senkrechten Achse
Ordinate 3-14: Asymmetrisches Sechseck mit extremer Verlängerung der Achse
Ordinate 3-15: Asymmetrisches Sechseck mit unregelmäßiger Verlängerung der Achse
Ordinate 3-21: Asymmetrisches Sechseck mit einer Induktion
Ordinate 3-22: Asymmetrisches Sechseck mit zwei Induktionen
Ordinate 3-23: Asymmetrisches Sechseck mit drei Induktionen
Ordinate 3-24: Asymmetrisches Sechseck mit vier Induktionen
Ordinate 3-25: Asymmetrisches Sechseck mit fünf und mehr Induktionen

### Abszisse 3-64: Gleichseitige Vielecke, hier: Siebeneck

Ordinate 3-11: Gleichseitige Achtecke
Ordinate 3-12: Gleichseitige Neunecke
Ordinate 3-13: Gleichseitige Zehnecke
Ordinate 3-14: Gleichseitige Zwölfecke
Ordinate 3-15: Gleichseitige Zwanzigecke
Ordinate 3-21: Gleichseitige Vielecke mit einer Induktion
Ordinate 3-22: Gleichseitige Vielecke mit zwei Induktionen
Ordinate 3-23: Gleichseitige Vielecke mit drei Induktionen
Ordinate 3-24: Gleichseitige Vielecke mit vier Induktionen
Ordinate 3-25: Gleichseitige Vielecke mit fünf und mehr Induktionen

### Abszisse 3-65: Ungleichseitige, symmetrische Vielecke mit verlängerter Längsachse

Ordinate 3-11: Ungleichseitige symmetrische Achtecke
Ordinate 3-12: Ungleichseitige symmetrische Neunecke
Ordinate 3-13: Ungleichseitige symmetrische Zehnecke

Ordinate 3-14: Ungleichseitige, symmetrische Zwölfecke
Ordinate 3-15: Ungleichseitige symmetrische Zwanzigecke
Ordinate 3-21: Ungleichseitige symmetrische Vielecke mit einer Induktion
Ordinate 3-22: Ungleichseitige, symmetrische Vielecke mit zwei Induktionen
Ordinate 3-23: Ungleichseitige, symmetrische Vielecke mit drei Induktionen
Ordinate 3-24: Ungleichseitige, symmetrische Vielecke mit vier Induktionen
Ordinate 3-25: Ungleichseitige, symmetrische Vielecke mit fünf und mehr Induktionen

### Abszisse 3-66: Teilsymmetrische Vielecke mit unbestimmten Eckenzahlen

Ordinate 3-11: Teilsymmetrische Vielecke mit unbestimmten Eckenzahlen und einer schwach verlängerten Längsachse
Ordinate 3-12: Teilsymmetrische Vielecke mit unbestimmten Eckenzahlen und einer verlängerten Längsachse
Ordinate 3-13: Teilsymmetrische Vielecke mit unbestimmten Eckenzahlen und einer schwach verlängerten Längsachse
Ordinate 3-14: Teilsymmetrische Vielecke mit unbestimmten Eckenzahlen und einer sehr stark verlängerten Längsachse
Ordinate 3-15: Teilsymmetrische Vielecke mit unbestimmten Eckenzahlen und einer extrem verlängerten Längsachse
Ordinate 3-21: Teilsymmetrische Vielecke mit unbestimmten Eckenzahlen und einer Induktion
Ordinate 3-22: Teilsymmetrische Vielecke mit unbestimmten Eckenzahlen und zwei Induktionen
Ordinate 3-23: Teilsymmetrische Vielecke mit unbestimmten Eckenzahlen und drei Induktionen
Ordinate 3-24: Teilsymmetrische Vielecke mit unbestimmten Eckenzahlen und vier Induktionen
Ordinate 3-25: Teilsymmetrische Vielecke mit unbestimmten Eckenzahlen und fünf und mehr Induktionen

### Abszisse 3-70: Voller Kreis

Ordinate 3-11: Voller Kreis mit leichter Verlängerung der senkrechten Symmetrieachse
Ordinate 3-12: Voller Kreis mit Verlängerung der senkrechten Symmetrieachse
Ordinate 3-13: Voller Kreis mit starker Verlängerung der senkrechten Symmetrieachse
Ordinate 3-14: Voller Kreis mit extremer Verlängerung der Symmetrieachse
Ordinate 3-15: Voller Kreis mit unregelmäßiger Verlängerung der Symmetrieachse
Ordinate 3-21: Voller Kreis mit einer leichten Induktion

Ordinate 3-22: Voller Kreis mit einer stärkeren Induktion
Ordinate 3-23: Voller Kreis mit einer starken Induktion
Ordinate 3-24: Voller Kreis mit einer sehr starken Induktion
Ordinate 3-25: Voller Kreis mit einer deformierenden Induktion

### Abszisse 3-71: Kreisförmige Figur

Ordinate 3-11: Kreisförmige, einfach symmetrische Figur mit leichter Verlängerung der senkrechten Symmetrieachse
Ordinate 3-12: Kreisförmige, einfach symmetrische Figur mit Verlängerung der senkrechten Symmetrieachse
Ordinate 3-13: Kreisförmige, einfach symmetrische Figur mit starker Verlängerung der senkrechten Symmetrieachse
Ordinate 3-14: Kreisförmige, einfach symmetrische Figur mit extremer Verlängerung der Symmetrieachse
Ordinate 3-15: Kreisförmige, einfach symmetrische Figur mit unregelmäßiger Verlängerung der Symmetrieachse
Ordinate 3-21: Kreisförmige, einfach symmetrische Figur mit einer leichten Induktion
Ordinate 3-22: Kreisförmige, einfach symmetrische Figur mit einer stärkeren Induktion
Ordinate 3-23: Kreisförmige, einfach symmetrische Figur mit einer starken Induktion
Ordinate 3-24: Kreisförmige, einfach symmetrische Figur mit einer sehr starken Induktion
Ordinate 3-25: Kreisförmige, einfach symmetrische Figur mit einer deformierenden Induktion

### Abszisse 3-72: Reduzierter Kreis

Ordinate 3-11: Kreisförmige, Figur mit Bildung eines kleinen Innenhofes
Ordinate 3-12: Kreisförmige, Figur mit Bildung eines Außenhofes
Ordinate 3-13: Kreisförmige, Figur mit Bildung zweier Außenhöfe
Ordinate 3-14: Kreisförmige, Figur mit Bildung dreier Außenhöfe
Ordinate 3-15: Kreisförmige, Figur mit Bildung von vier und mehrerer Außenhöfe
Ordinate 3-21: Kreisförmige, Figur mit einer tiefen Induktion
Ordinate 3-22: Kreisförmige, Figur mit zwei tiefen Induktionen
Ordinate 3-23: Kreisförmige, Figur mit drei tiefen Induktionen
Ordinate 3-24: Kreisförmige, Figur mit vier tiefen Induktionen
Ordinate 3-25: Kreisförmige, Figur mit fünf und mehr tiefen Induktionen

### Abszisse 3-73: Leerer Kreis

Ordinate 3-11: Leerer Kreis mit einer fingerartigen Verlängerung
Ordinate 3-12: Leerer Kreis mit einer längeren, fingerartigen Verlängerung
Ordinate 3-13: Leerer Kreis mit einer langen, fingerartigen Verlängerung
Ordinate 3-14: Leerer Kreis mit einer sehr langem, fingerartigen Verlängerung
Ordinate 3-15: Leerer Kreis mit einer linienförmig, fingerartigen Verlängerung
Ordinate 3-21: Leerer Kreis mit einem Halbschnitt
Ordinate 3-22: Leerer Kreis mit einem Ganzschnitt
Ordinate 3-23: Leerer Kreis mit zwei Ganzschnitten
Ordinate 3-24: Leerer Kreis mit einem Kreuzschnitt
Ordinate 3-25: Leerer Kreis mit einem Vielfachschnitt

### Tafel 14 Lumen a:

Die Tafel zeigt acht Felder unterschiedlicher Flächen- und Raumkonstellationen, wie sie während einer Kristallisation und der Ausbildung von kristallinen Formen und Texturen der Tafel 1 als Verhältnis von Vorder-und Hintergrund entstehen.
Feld 4-10: Helle, kleine, formbeliebige Struktur auf größerer, dunkler, formbeliebiger Struktur
Feld 4-11: Helle, große, formbeliebige Struktur auf schmalerer, dunkler, formbeliebiger Struktur
Feld 4-20: Hellere, formbeliebige Struktur auf hellerer, größerer und formbeliebiger Struktur, wobei diese sich von einem dunkleren Hintergrund darstellt
Feld 4-21: Dunkle, formbeliebige Struktur auf hellerer, größerer und formbeliebiger Struktur, wobei diese sich vor einem dunkleren Hintergrund darstellt
Feld 4-30: Gegenüber dem Hintergrund als leicht dunkler differenzierte Struktur
Feld 4-31: Gegenüber dem Hintergrund als deutlich dunkler differenzierte Struktur
Feld 4-40: Helle, plastische Struktur mit deutlichen Grenzen auf einer ebenso hellen, plastischen Struktur mit deutlichen Grenzen in einer formbeliebigen, hellen Struktur, die auf einem dunkleren Hintergrund liegt
Feld 4-41: Helle, große und formbeliebige Struktur auf einem dunklen Hintergrund.

### Tafel 22 Lumen b:

Die Tafel zeigt acht Felder unterschiedlicher Flächen- und Raumkonstellationen, wie sie während einer Kristallisation und der Ausbildung von kristallinen Formen und Texturen der Tafel 2 wie Tafel 14.
Feld 10: Helle, kleine, formbeliebige Struktur auf größerer, dunkler, formbeliebiger Struktur
Feld 11: Helle, größe, formbeliebige Struktur auf schmalerer, dunkler, formbeliebiger Struktur
Feld 20: Hellere, formbeliebige Struktur auf hellerer, größerer und formbeliebiger Struktur, wobei diese sich vor einem dunkleren Hintergrund darstellt
Feld 21: Dunkle, formbeliebige Struktur auf hellerer, größerer und formbeliebiger Struktur, wobei diese sich vor einem dunkleren Hintergrund darstellt
Feld 30: Gegenüber dem Hintergrund als leicht dunkler differenzierte Struktur
Feld 31: Gegenüber dem Hintergrund als deutlich dunkler differenzierte Struktur
Feld 40: Helle, plastische Struktur mit deutlichen Grenzen auf einer ebenso hellen, plastischen Struktur mit deutlichen Grenzen in einer formbeliebigen, hellen Struktur, die auf einem dunkleren Hintergrund liegt
Feld 41: Helle, große und formbeliebige Struktur auf einem dunklen Hintergrund.

### Tafel 32 Lumen c :

Die Tafel zeigt acht Felder unterschiedlicher Flächen- und Raumkonstellationen, wie sie während einer Kristallisation und der Ausbildung von kristallinen Formen und Texturen der Tafel 3 wie Tafel 14.
Feld 10: Helle, kleine, formbeliebige Struktur auf größerer, dunkler, formbeliebiger Struktur
Feld 11: Helle, größe, formbeliebige Struktur auf schmalerer, dunkler, formbeliebiger Struktur
Feld 20: Hellere, formbeliebige Struktur auf hellerer, größerer und formbeliebiger Struktur, wobei diese sich vor einem dunkleren Hintergrund darstellt
Feld 21: Dunkle, formbeliebige Struktur auf hellerer, größerer und formbeliebige Struktur, wobei diese sich vor einem dunkleren Hintergrund darstellt
Feld 30: Gegenüber dem Hintergrund als leicht dunkler differenzierte Struktur
Feld 31: Gegenüber dem Hintergrund als deutlich dunkler differenzierte Struktur
Feld 40: Helle, plastische Struktur mit deutlichen Grenzen auf einer ebenso hellen, plastischen Struktur mit deutlichen Grenzen in einer formbeliebigen, hellen Struktur, die auf einem dunkleren Hintergrund liegt
Feld 41: Helle, größe und formbeliebige Struktur auf einem dunklen Hintergrund.

Das Verfahren der Erfindung bietet den Vorteil der charakteristischen Erfassung komplexer und spezifischer Zusammenhänge bei Kristallclustern ohne redundante Daten zu benötigen. Dieses bietet weiter den Vorteil, dass Diagnoseergebnisse ohne aufwendige laborantische Technik durch vergleichende Mustererkennung durch Ermittlung Numerischer Sequenzen erstmalig erhalten werden.

Dieses bietet weiter den Vorteil, dass eine wirtschaftliche Rechnerleistung bei optimaler Rechnerzeit erreicht wird.

## Patentansprüche

1. Verfahren zur Erzeugung von Kristallisaten aus Blut oder anderen, menschlichen oder tierischen Flüssigkeiten, sowie zur Auswertung der resultierenden Kristallcluster für diagnostische Zwecke dadurch gekennzeichnet, dass Kristallisate aus Blut oder anderen menschlichen oder tierischen Flüssigkeiten erzeugt und deren Formen und Texturen in Abszissen und Ordinaten und daraus resultierend, in numerische Sequenzen umgesetzt werden zum Mustervergleich mit den ermittelten numerischen Sequenzen eines Kristallclusters aus klinischen oder laborantischen Diagnosen als probabilistisches Ergebnis mit den folgenden Verfahrensstufen:
1) Einfüllen von arteriellem oder kapillarem oder venösem Blut in einen Aufschlußkolben in einer Menge von 0,1 - 10 ml, vorzugsweise 2 ml, Vermischen mit zwei- bis vierfach destilliertem Wasser, vorzugweise bidestilliertem Wasser, in einer Menge von 10 bis 50 ml, vorzugsweise 25 ml, Rotieren der Lösung mit 60 - 120 U/min, vorzugweise 85 U/min in einer Zeit von 30 - 90 min, vorzugsweise 60 min, bei einer Temperatur von 16 - 24°C, vorzugweise 20°C,
2) Einleiten von hochgesättigtem, mehrfach, vorzugsweise 4- fach, destilliertem Wasserdampf, durch ein Einleitungsrohr in die Flüssigkeit unter Aufwirbeln, Kondensieren des aufsteigenden Wasserdampfes durch einem Intensivkühler, Auffangen des Kondensates in einer Menge von 8 - 25 ml, vorzugsweise 10 ml, in ein vorgelegtes Gefäss unter Bildung eines Wasserdampf- Macerates, (WDM),
3) Vermischen des WDM in einer Menge von 10 - 30 µl, vorzugsweise 24 µl, mit 5-20 mg, vorzugsweise mit 10 mg, eines bei 460-940°C, vorzugsweise bei 640°C, unter Luftabschluss veraschten und dann fein verriebenen Gemischen aus löslichen, anorganischen, einwertigen Salzen, insbesondere NaCl, tierischem Protein, Fruktose, Glukose, Stärke im Gewichtsverhältnis von 1:1:1.1: 2 als Trägermaterial zu einer praktisch gesättigten Suspension und deren Filtration durch ein Mikrofilter,
4) Auftropfen des Filtrates in einer Menge von 10-50 µl, vorzugsweise 24 µl, mit einer Pipette auf einem gläsernen Objektträger zu einem Troptfen mit einem Durch-messer von etwa 8-12 mm, Auflegen des Objektträgers mit dem Filtrat auf eine Präzisionsheizplatte mit einer Temperatur von 20-25°C, vorzugsweise 24°C, Abdampfen des Wassers bis zur kristallinen Phase,
5) Scannen des Kristallclusters unter dem Lichtmikroskop oder Elektronenmikroskop im Dunkelfeld bei Abbildungsmasstäben zwischen 4 bis 16, und Bildspeicherung, danach automatische Durchmusterung mit einem Programm zur Erkennung von Bildmustern zur Ermittlung folgender Werte : Form ; Fraktal a,b,c ; Cluster a,b,c,d ; Kontur a,b,c,d ; Präfix A ; Textur A ; Postfix A ; Präfix B ; Textur B ; Postfix B ; Lumen a,b,c; gemäß Tafeln 1,11,111,1111,12,121, 122,123,13,131,132, 133, 21, 2, 23, 31, 3, 33, 14, 22 und 32 der Beschreibung.
6) Ermittlung der Koordinaten der Form als Element möglicher Texturen mit Tafel 1, Form,
danach Ermittlung der Koordinaten der Anordnung der Formen aus Tafel 1, bezogen auf die Fläche x, y, mit Tafel 11,
Fraktal a,
danach Ermittlung der vektoriellen Ausrichtung der Texturstämme aus Tafel 11 mit Tafel 111,
Fraktal b,
danach Ermittlung der vektoriellen Ausrichtung der Texturzweige aus Tafel 11 mit Tafel 1111,
Fraktal c,
danach Ermittlung der auf den Raum x, y, z bezogenen Anordnung der Formen aus Tafel 1 mit Tafel 12,
Cluster a,
danach Ermittlung der inneren Strukturierung der durch Tafel 12 bezeichneten Texturen mit Tafel 121,
Cluster b,
danach Ermittlung der äusseren Grenzstrukturen der durch Tafel 12 bezeichneten Texturen mit Tafel 122,
Cluster c,
danach Ermittlung der relativen Dichte und/ oder Strukturierung der durch die Tafel 12 definierten Textur mit Tafel 123,
Cluster d,
danach Ermittlung der Grenzbildung der durch die Tafel 12 definierten Formen und/ oder Texturen durch Kontrast gegenüber der Umgebung mit Tafel 13,
Kontur a,
danach Ermittlung der inneren Kontrastbildung der durch die Tafel 12 definierten Formen und/ oder Texturen mit Tafel 131,
Kontur b,
Kontur c,
danach Ermittlung des Schwerpunktes des Ortes der durch die Tafel 12 definierten Formen und/ oder Texturen, ausgedrückt in Sektoren mit Tafel 132,
danach Ermittlung der Gesamtform, die von allen gleichartigen Formen und/ oder Texturen mit Tafel 12 gebildet wird, ausgedrückt in Tafel 133,
Kontur d,
danach Ermittlung des Verhältnisses, in dem die Formen und/ oder Texturen aus Tafel 2 zu den Formen und/ Texturen aus Tafel 1 stehen mit Tafel 21,
Präfix A,
danach Ermittlung der Formen und/ Texturen, die in einem durch Tafel 21 definierten, räumlichen Verhältnis zu den Formen oder Texturen der Tafel 1 stehen mit Tafel 2,
Textur A,
danach Ermittlung der Anzahl des Auftretens der Formen und/ oder Texturen aus Tafel 2 und/ oder des besonderen Winkels, in dem die Formen und/ oder Texturen aus Tafel 2 zu den Formen und/ oder Texturen aus Tafel 1 stehen mit Tafel 23,
Postfix A,
danach Ermittlung des Verhältnisses, in dem die Formen und/ oder Texturen aus Tafel 3 zu den Formen und/ oder Texturen aus Tafel 1 stehen mit Tafel 31,
Präfix B,
danach Ermittlung der Formen und/ oder Texturen, die in einem definierten, räumlichen Verhältnis zu den Formen und/ oder Texturen der Tafel 1 und/ oder Tafel 2 stehen mit Tafel 3,
Textur B,
danach Ermittlung der Anzahl des Auftretens der Formen und/oder Texturen aus Tafel 3 und/oder des besonderen Winkels, in dem die Form und/oder Textur aus Tafel 3 zu den Formen und/ oder Texturen aus Tafel 1 und/oder 2 stehen mit Tafel 33,
Postfix B,
danach Ermittlung des Abbildungsverhältnisses der Formen und/ oder Texturen aus Tafel 1 gegenüber ihrem Untergrund oder Hintergrund mit Tafel 14,
Lumen a,
danach Ermittlung des Abbildungsverhältnisses der Textur A aus Tafel 2 gegenüber ihrem Untergrund oder Hintergrund mit Tafel 22,
Lumen b,
danach Ermittlung des Abbildungsverhältnisses der Textur B aus Tafel 3 gegenüber ihrem Untergrund oder Hintergrund mit Tafel 32,
Lumen c,
zur Numerischen Sequenz, wobei die Ermittlung maschinell oder manuell durchgeführt wird,
danach Eingabe der Numerischen Sequenz in eine Datenbank als Schnittstelle, in welcher die Numerischen Sequenzen aus klinisch gesicherten Diagnosen abgespeichert sind zur Ermittlung durch probabilistischen Vergleich einer prophylaktischen und/oder präventiven und/ oder Verdachtsdiganose.

2. Verfahren zur Erzeugung von Kristallisaten aus Blut oder anderen, menschlischen oder tierischen Flüssigkeiten, sowie zur Auswertung der resultierenden Kristall-cluster für diagnostische Zwecke in Abänderung des Verfahrens nach Anspruch 1, dadurch gekennzeichent, dass Kristallisate aus Blut oder anderen menschlischen oder tierischen Flüssigkeiten erzeugt und deren Formen und Texturen in Abszissen und Ordinaten und daraus resultierend, in numerische Sequenzen umgesetzt werden zum Mustervergleich mit den ermittelten numerischen Sequenzen eines Kristallclusters aus klinischen oder laborantischen Diagnosen als probabilistisches Ergebnis mit den folgenden Verfahrensstufen:
1) Einfüllen von arteriellem oder kapillarem oder venösem Blut in einen Aufschlußkolben in einer Nenge von 0,1 - 10 ml, vorzugsweise 2 ml, Vermischen mit zwei- bis vierfach destilliertem Wasser, vorzugsweise bidestilliertem Wasser, in einer Nenge von 10 bis 50 ml, vorzugsweise 25 ml, Rotieren der Lösung mit 60 - 120 U/min, vorzugsweise 85 U/min in einer Zeit von 30 - 90 min, vorzugsweise 60 min, bei einer Temperatur von 16 - 24°C, vorzugsweise 20°C,
2) Einteilen von hochgesättightem, mehrfach, vorzugsweise 4-fach, destilliertem Wasserdampf, durch ein Eintellungsrohr in die Flüssigkeit unter Aufwirbeln, Kondensieren des aufsteigenden Wasserdampfes durch einen Intensivküler, Auffangen des Kondensates in einer Menge von 8 - 25 ml, vorzugsweise 10 ml, in ein vorgelegtes Gefäss unter Bildung eines Waseerdampf- Macerates, (WDM),
3) Vermischen des WDM in einer Menge von 10 - 30 µl, vorzugsweise 24 µl, mit 5-20 mg, vorzugsweise mit 10 mg, eines bei 460-940°C, vorzugsweise bei 640°C, unter Luftabschluss veraschten und dann fein verriebenen Gemisches aus NaCl, tierischem Protein, Fruktose, Glukose, Stärke im Gewichts verhältnis von 1 : 1 : 1 : 1 : 2 als Trägermaterial zu einer praktisch gesättigten Suspension und deren Filtrieren durch einen Mikrofilter,
4) Auftropfen des Filtrates in einer Menge von 10-50 µl, vorzugsweise 24 µl, mit einer Pipette auf einem gläsernen Objekt-träger zu einem Tropfen mit einem Durchmesser von etwa 8-12 mm, Auflegen des Objektträgers mit dem Filtrat auf eine Präzisionsheizplatte mit einer Temperatur von 20 - 25°C, vorzugsweise 24°C, Abdampfen des Wassers bis zur kristallinen Phase,
5) Scannen der kristallinen Phase unter einem Lichtmikroskop im Dunkelfeld oder einem Elektronenrastermikroskop oder einem Laserrastermikroskop, insbesondere zwischen den Abbildungsmaßstäben von 4 bis 16 und Speicherung als Bilddatei,
6) automatische Durchmusterung fer Bilddatei oder kristallinen Phase auf dem Obkektträger zur Erkennung von Bildmustern auf Formen und/oder Testuren mit gemeinsamer, geometrischer Gleichartigkeit,
7) automatische Selektiereung der durchmusterten und erkannten Bildmuster von Formen und/oder Texturen zur Ermittlung der Form und/oder Textur, die am differenziertesten bei geringstem Raumbedarf ist.
8) Projizierung der selektierten Form und/oder Textur auf den Monitor oder Zwischenspeicher eines Rechners und Durchmusterung einer Datenbank, in welcher die geometrischen Formen und Formen der Tafeln 1,11,111,1111,12,121,122,123,13,131,132,133,21,2,23,31,3, 33,14, 22,32 der Beschreibung gespeichert sind, auf die der selektierten Form und/oder Textur ähnlichte oder gleiche Form und/oder Textur,
9) Definieren der selektierten Form und/oder Textur in den Koordinaten der ähnlichsten oder gleichen Form und/oder Textur der Tafeln 1,11,111,1111,12,121,122,123,13,131,132,133,21,2,23,31,3,33,14, 22,32 der Beschreibung zu einer vollständigen Reihe, bestehend aus Form, Fraktal a,b,c, Cluster a,b,c,d, Kontur a,b,c,d, Präfix a, Textur a, Postfix a, Präfix b, Textur b, Postfix b, Lumen a,b,c, als Numerische Sequenz der definierten Form und/oder Textur,
10) Eingeben der Numerischen Sequenz in eine Datenbank, in der gesicherte, klinische Diagnosen Numerischen Sequenzen zugeordnet abgespeichert sind, zur Erstellung einer probabilistischen Diagnose für die Prophylaxe und Prävention.

3. Verfahren nach Anspruch 1 und 2, dadurch gekennzeichnet, dass zur bildhaften Darstellung einfacher und komplexer Formen und Texturen und zu deren Umsetzung in numerische Sequenzen aus einem Kristallisat die geometrischen, tabellarischen Darstellungen gemäß Tafeln 1, 11, 111, 1111, 12, 121, 122, 123, 13, 131, 132, 133, 21, 2, 23, 31, 3, 33, 14, 22, und 32 der Beschreibung verwendet werden.

4. Verfahren nach den Ansprüchen 1 - 3, dadurch gekennzeichnet,dass die durch Abszissen und Ordinaten identifizierten Formeln der Tafeln 1, 11, 111, 1111, 12, 121, 122, 123, 13, 131, 132, 133, 2, 3, 14, 22, und 32 gemäß dem Abschnitt "Auflistung der Formen und/oder Texturen" der Beschreibung definiert werden.

5. Anwendung des Verfahrens nach den Ansprüchen 1 und 2, dadurch gekennzeichnet, dass die Stufen 5 und 6 des Anspruches 1 und die Stufen 5 bis 10 des Anspruches 2 zur Ermittlung Numerischer Sequenzen auf der Basis entsprechender Ausgungsbilddateien im Rahmen anderer, bildgebender, bild- verarbeitender, mustererkennender, mustervergleichender Prozesse verschiedener Herkunft zu deren Klassierung und Prognostizierung eingesetzt werden, insbesondere im Rahmen solcher Prozesse, denen ein alineares Funktionsmuster zugrunde liegt.

## Claims

1. Process for production of crystals from blood or other human or animal liquids, as well as for the analysis of the resulting crystal clusters for diagnostic purposes characterized by crystals made of blood or other human or animal liquids and their forms and textures in abscissa and ordinate and resulting from this, and converted into numeral sequences for the comparison of patterns with the determinated numeral sequences of a crystal cluster from clinical or laboratory diagnoses as probabilistic result with the following process steps:
1. Introducing a quantity of 0.1 - 10 ml, preferably 2 ml, of arterial or capillary or venous blood in a retort, mixing with two to four times distilled water, preferably bidistilled water, in a quantity from 10 to 50 ml, preferably 25 ml, rotating of the solution with 60 - 120 min⁻¹, preferably 85 min⁻¹, during a period of 30 - 90 min, preferably 60 min, at a temperature of 16 - 24 °C, preferably 20 °C,
2. Introducing of high-saturated, several times, preferably 4 times, distilled water steam, by means of a introducing tube into the liquid by swirling, condensing of the rising water steam by means of an intensive cooler, collecting a quantity of 8 - 25 ml, preferably 10 ml, of condensate into a prepared receptacle by forming a water steam macerate, (WDM),
3. Mixing of the WDM into a quantity of 10 - 30 µl, preferably 24 µl, with 5 - 20 mg, preferably 10 mg, at 460 - 940 °C, preferably at 640 °C, airtight calcined and than fine ground mixture of soluble, inorganic, univalent acids, especially NaCI, animal protein, fructose, glucose, starch in a weight ratio of 1 : 1 : 1 : 1 : 2 as base material for a nearly saturated suspension and its filtration through a micro-filter,
4. Dripping of the filtrate in a quantity of 10 - 50 µl, preferably 24 µl, by means of a pipette on a glass slide of a drop with a diameter of about 8 - 12 mm, placing of the slide with the filtrate on a precision heating plate with a temperature of 20 - 25 °C, preferably 24 °C, evaporation of the water until the crystal phase,
5. Scanning of the crystal cluster under the light-optical microscope or electron microscope in the dark field for reproduction scales from 4 to 16, and picture storing, then automatic scanning operation by means of a program for recognition of picture patterns for determination of the following values:
Form; Fraktal a, b, c; Cluster a, b, c, d; Kontur a, b, c, d; Präfix A; Texture A; Postfix A; Präfix B; Texture B; Postfix B. Lumen a, b, c; according to table 1,11,111, 1111, 12,121, 122, 123, 13, 131, 132, 133, 21, 2, 23, 31,3, 33, 14, 22 and 32 of the description,
6. Determination of the coordinates of the Form as elements of possible textures with table 1,
Form, then determination of the coordinates of the order of the Forms from table 1, relating to the surface x, y, with table 11,
Fraktal a,
then determination of the vectorial orientation of the Texture lines from table 11 with table 111,
Fraktal b,
then determination of the vectorial orientation of the Texture branches from table 11 with table 1111,
Fraktal c,
then determination of the order of the Forms from table 1 with table 12 relating to the space x, y, z,
Cluster a,
then determination by means of table 121 of the internal structure of the Textures described by table 12,
Cluster b,
then determination by means of table 122 of the external limit structures of the Textures described by table 12,
Cluster c,
then determination by means of table 123 of the relative density and/or structure of the Texture defined by table 12,
Cluster d,
then determination by means of table 13 of limit shape of the Forms and/or Textures defined by table 12 by means of contrast compared to the environment, Kontur a,
then determination by means of table 131 of the internal contrast of the Forms and/or Textures defined by table 12,
Kontur b,
then determination by means of table 132 of the centre of gravity of the place of the Forms and/or Textures defined by table 12, described in sectors,
Kontur c,
then determination of the total Form, formed by all similar Forms and/or Textures with table 12, shown in table 133,
Kontur d,
then determination by means of table 1 of the ratio of the Forms and/or Textures of table 2 and the Forms and/or Textures of table 1,
Präfix A,
then determination by means of table 2 of the Forms and/or Textures, placed in a space ratio defined by table 21 compared to the Forms or Textures of table 1,
Texture A,
then determination by means of table 23 of the number of appearances of the Forms and/or Textures from table 2 and/or of the special angle, in which the Forms and/or Textures of table 2 are compared to the Forms and/or Textures of table 1,
Postfix A,
then determination by means of table 31 of the ratio in which the Forms and/or Textures of table 3 are compared to the Forms and/or Textures of table 2 ,
Präfix B,
then determination by means of table 3 of the Forms and/or Textures which are in a defined space ratio compared to the Forms and/or Textures of table 1 and/or table 2,
Texture B,
then determination by means of table 33 of the number of appearances of the Forms and/or Textures of table 3 and/or of the special angle, in which the Forms and/or Textures of table 3 are compared to the Forms and/or Textures of table 1 and/or 2,
Postfix B,
then determination by means of table 14 of the reproduction ratio of the Forms and/or Textures of table 1 compared to their ground or background,
Lumen a,
then determination by means of table 22 of the reproduction ratio of the Texture A of table 2 compared to its ground or background,
Lumen b,
then determination by means of table 32 of the reproduction ratio of the Texture B of table 3 compared to its ground or background,
Lumen c, concerning the numeric sequence, for which the determination is made mechanically or manually,
then input of the numeric sequences into a database as interface in which the numeric sequences are stored from clinically guaranteed diagnoses for determination by means of a probabilistic comparison of prophylactic and/or preventive and/or suspicion diagnosis.

2. Process for production of crystals of blood or other human or animal liquids as well as for determination of the resulting crystal Cluster for diagnostic reasons in modification to the process according to claim 1, produced by crystals of blood or other human or animal liquids and their Forms and Textures in abscissas and ordinates and resulting form it they are transformed in numeric sequences for comparison of patterns with the determined numeric sequences of a crystal Cluster from clinical or laboratory diagnoses as probabilistic result with the following process steps:
1. Introducing a quantity of 0.1 - 10 ml, preferably 2 ml, of arterial or capillary or venous blood in a retort, mixing with two to four times distilled water, preferably bidistilled water, in a quantity from 10 to 50 ml, preferably 25 ml, rotating of the solution with 60 - 120 min⁻¹, preferably 85 min⁻¹, during a period of 30 - 90 min, preferably 60 min, at a temperature of 16 - 24 °C, preferably 20 °C,
2. Introducing of high-saturated, several times, preferably 4 times, distilled water steam, by means of a introducing tube into the liquid by swirling, condensing of the rising water steam by means of an intensive cooler, collecting a quantity of 8 - 25 ml, preferably 10 ml, of condensate into a prepared receptacle by forming a water steam macerate, (WDM),
3. Mixing of the WDM into a quantity of 10 - 30 µl, preferably 24 µl, with 5 - 20 mg, preferably 10 mg, at 460 - 940 °C, preferably at 640 °C, airtight calcined and than fine ground mixture of NaCI, animal protein, fructose, glucose, starch in a weight ratio of 1 : 1 : 1 : 1 : 2 as base material for a nearly saturated suspension and its filtration through a micro-filter,
4. Dripping of the filtrate in a quantity of 10 - 50 µl, preferably 24 µl, by means of a pipette on a glass slide of a drop with a diameter of about 8 - 12 mm, placing of the slide with the filtrate on a precision heating plate with a temperature of 20 - 25 °C, preferably 24 °C, evaporation of the water until the crystal phase,
5. Scanning of the crystal phase under the light-optical microscope in the dark field or a scanning electron microscope or a scanning laser-microscope, especially between the reproduction scales from 4 to 16 and storing as picture file,
6. Automatic scanning of the picture file or crystal phase on the slide for recognition of picture files on Forms and/or Textures with common geometric homogeneity,
7. Automatic selection of the scanned and recognized picture patterns concerning Forms and/or Textures for determination of the Form and/or the Texture which is the most differentiated at lowest space need,
8. Projection of the selected Form and/or Texture on a monitor or intermediate memory of a computer and scanning of a data base, in which the geometrical Forms and Forms of the tables
1,11,111,1111,12,121, 122,123,13,131,132,133, 21,2, 23, 31, 3,33, 14,22,32 are stored as the most similar or similar Form and/or Texture compared to the selected Form and/or Texture,
9. Defining of the selected Form and/or Texture in the coordinates of the most similar or similar Form and/or Texture of the tables
1,11,111, 1111,12,121,122,123,13, 131, 132,133,21,2, 23, 31, 3, 33, 14, 22, 32 to complete series consisting of Form; Fraktal a, b, c; Cluster a, b, c; Kontur a, b, c, d; Präfix a; Texture a; Postfix a; Präfix b; Texture b; Postfix b; Lumen a, b, c as numeric sequence of the defined Form and/or Texture,
10. Input of the numeric sequences into a database in which clinically guaranteed diagnoses are assigned to numeric sequences and stored for working out of a probabilistic diagnosis for prophylaxis and prevention.

3. Process according to claim 1 and 2, defined by a depiction of simple and complex Forms and Textures and their transformation in numeric sequences from a crystal by means of the geometric, serial descriptions according to the tables 1,11,111, 1111,12, 121, 122, 123, 13, 131, 132, 133, 21, 2, 23, 31,3, 33, 14, 22 and 32.

4. Process according to the claims 1 - 3, wherein the formulas, identified by the abscissas and ordinates of the tables 1,11, 111, 1111,12,121,122,123,13, 131,132,133,2,3,14,22 and 32 identified by the abscissas and ordinates according to the section ,,List of Forms and/or Textures" of the description are defined.

5. Application of the process according to claim 1 and 2, wherein the steps 5 and 6 of the claim 1 and step 5 to 10 of claim 2 for determination of numeric sequences on the basis of corresponding departure picture files within the framework of other, picture furnishing, picture processing, pattern recognition, pattern comparing processes of various destination are used for their classification and prognostication, especially in the framework of processes based on non-linear function models.

## Revendications

1. Procédé de production de cristaux provenant de sang ou d'autres liquides humains ou animaux, comme pour l'analyse des Clusters de cristal en résultants pour des raisons de diagnostic, caractérisé par le fait que le cristal produit de sang ou d'autres liquides humains ou animaux et que leurs Formes et Textures sont traduits en séquences numériques pour la comparaison des échantillons avec les séquences numériques d'un Cluster de cristal obtenus de diagnostic cliniques ou de laboratoires comme résultat probabiliste avec les étapes de procédé suivantes:
1. Introduction d'une quantité de 0.1 - 10 ml, de préférence 2 ml, de sang artériel ou capillaire ou veineux dans un alambic, mélanger avec de l'eau distillée de deux à quatre fois, de préférence de l'eau distillée deux fois, d'une quantité de 10 à 50 ml, de préférence de 25 ml, tourner la solution de 60 à 120 min⁻¹, de préférence à 85 min⁻¹, pendant une durée de 30 - 90 min, de préférence de 60 min, avec une température de 16 - 24 °C, de préférence de 20 °C,
2. Introduction de vapeur d'eau distillée, hautement saturée, plusieurs fois, de préférence 4 fois, à l'aide d'un tube d'introduction dans le liquide en tourbillonnant, condensation de la vapeur d'eau à l'aide d'un réfrigérant chemisé à serpentins, réception d'une quantité de 8 - 12 ml du produit de condensation, de préférence de 10 ml, dans un récipient en formant un macéré de vapeur d'eau, (WDM),
3. Mélanger le WDM dans un quantité de 10 - 30 µl, de préférence de 24 µl, avec 5 - 20 mg, de préférence de 10 mg, à 460 - 940 °C, de préférence à 640 °C, de mélanges calcinés à l'abri de l'air et ensuite des acides solubles, minérales et univalents, spécialement du NaCI, de la protéine animale, de la fructose, de la glucose, de l'amidon finement broyés d'une proportion de poids de 1:1:1:1: 2 comme support pour une suspension pratiquement saturée et sa filtration par un micro filtre,
4. Verser une goutte de produit filtré d'une quantité de 10 - 50 µl, de préférence de 24 µl, à l'aide d'une pipette sur une lame en verre, la goutte devra avoir un diamètre d'approximativement 8 - 12 mm, placer la lame sur une plaque chauffante de précision ayant une température de 20 - 25 °C, de préférence de 24 °C, évaporer l'eau jusqu'à la phase cristalline,
5. Scanner le Cluster de cristal sous le microscope optique ou le microscope d'électrons sur fond noir pour des échelles de reproduction de 4 à 16, et images sauvegardées, ensuite exploration automatique à l'aide d'un programme pour reconnaître les échantillons d'images pour déterminer les valeurs suivantes:
Forme; Fraktal a, b, c; Cluster a, b, c, d; Kontur a, b, c, d; Präfix A; Texture A; Postfix A; Präfix B; Texture B; Postfix B. Lumen a, b, c; selon les tableaux 1,11,111,1111,12,121, 122,123, 13, 131,132,133,21,2,23,31, 3,33, 14,22 et 32 de la description,
6. Recherche des coordonnées de la Forme comme élément de Textures possible à l'aide du tableau 1,
Forme, ensuite recherche des coordonnées de la disposition des Formes selon tableau 1, par rapport à la surface x, y, à l'aide du tableau 11,
Fraktal a,
ensuite recherche de l'orientation vectorielle des troncs de Texture selon tableau 11 à l'aide du tableau 111,
Fraktal b,
ensuite recherche de l'orientation vectorielle des branches de Texture selon tableau 11 à l'aide du tableau 1111,
Fraktal c,
ensuite recherche de l'orientation des Formes par rapport à l'espace x, y, z selon tableau 1 à l'aide du tableau 12
Cluster a,
ensuite recherche de l'organisation interne des Textures décrites par le tableau 12 à l'aide du tableau 121,
Cluster b,
ensuite recherche des structures limites des Textures décrites par le tableau 12 à l'aide du tableau 122,
Cluster c,
ensuite recherche de la densité relative et/ou de l'organisation des Textures définies par le tableau 12 à l'aide du tableau 123,
Cluster d,
ensuite recherche de la forme limite des Formes et/ou
Textures définies par tableau 12 en se servant du contraste par rapport à l'environnement à l'aide du tableau 13,
Kontur a,
ensuite recherche de la formation interne de contraste de Formes et/ou Textures définies par le tableau 12 à l'aide du tableau 131,
Kontur b,
ensuite recherche du centre de gravité du lieu des Formes et/ou Textures définies par le tableau 12, exprimés en secteurs à l'aide du tableau 132,
Kontur c,
ensuite recherche de la forme complète, formée par toutes les Formes et/ou Textures similaires à l'aide du tableau 12, exprimée dans le tableau 133,
Kontur d,
ensuite recherche du rapport des Formes et/ou Textures du tableau 2 comparé aux Formes et/ou Textures du tableau 1 à l'aide du tableau 21,
Präfix A,
ensuite recherche des Formes et/ou Textures qui ont un rapport de l'espace, définie par le tableau 21, comparé aux Formes ou Textures du tableau 1 par rapport au tableau 2, Texture A,
ensuite recherche du nombres d'apparition des Formes et/ou Textures du tableau 2 et/ou de l'angle particulier dans lequel se trouvent les Formes et /ou Textures du tableau 2 par rapport aux Formes et ou Textures du tableau 1 à l'aide du tableau 23,
Postfix A,
ensuite recherche du rapport dans lequel se trouvent les Formes et/ou Textures du tableau 3 par rapport aux Formes et/ou Textures du tableau 1 à l'aide du tableau 31,
Präfix B,
ensuite recherche des Formes et/ou Textures qui se trouvent dans un rapport de l'espace défini par rapport aux Formes et/ou Textures du tableau 1 et/ou du tableau 2 à l'aide du tableau 3,
Texture B,
ensuite recherche du nombre d'apparitions des Formes et/ou Textures du tableau 3 et/ou de l'angle particulier dans lequel se trouve la Forme et/ou la Texture du tableau 1 et/ou 2 à l'aide du tableau 33,
Postfix B,
ensuite recherche de la relation de la représentation des Formes et/ou Textures du tableau 1 par rapport à leur fond ou leur arrière-plan à l'aide du tableau 14,
Lumen a,
ensuite recherche de la relation de la représentation de la Texture 1 du tableau 2 par rapport à son fond ou son arrière-plan à l'aide du tableau 22,
Lumen b,
ensuite recherche de la relation de la représentation de la Texture B du tableau 3 par rapport à son fond ou son arrière-plan l'aide du tableau 32,
Lumen c,
concernant la séquence numérique, pour laquelle la recherche sera fait mécaniquement ou manuellement, ensuite entrée de la séquence numérique dans une banque de données comme interface, dans laquelle sont sauvegardées les séquences numériques des diagnostics cliniques assurés pour la recherche à l'aide d'une comparaison probabiliste et/ou d'un diagnostic présomptif prophylactique et/ou préventif.

2. Procédé de production de cristaux provenant de sang ou d'autres liquides humains ou animaux, comme pour l'analyse des Clusters de cristal en résultants pour des raisons de diagnostic, en modification du procédé selon revendication 1, caractérisé par le fait que le cristal produit de sang ou d'autres liquides humains ou animaux et que leurs Formes et Textures en abscisses et ordonnées et en résultant, sont traduits en séquences numériques pour la comparaison des échantillons avec les séquences numériques d'un Cluster de cristal obtenus de diagnostics cliniques ou de laboratoires comme résultat probabiliste avec les étapes de procédé suivantes:
1. Introduction d'une quantité de 0.1 - 10 ml, de préférence 2 ml, de sang artériel ou capillaire ou veineux dans un alambic, mélanger avec de l'eau distillée de deux à quatre fois, de préférence de l'eau distillés deux fois, d'une quantité de 10 à 50 ml, de préférence de 25 ml, tourner la solution de 60 à 120 min⁻¹, de préférence à 85 min⁻¹, pendant une durée de 30 - 90 min, de préférence de 60 min, avec une température de 16 - 24 °C, de préférence de 20 °C,
2. Introduction de vapeur d'eau distillée, hautement saturée, plusieurs fois, de préférence 4 fois, à l'aide d'un tube d'introduction dans le liquide en tourbillonnant, condensation de la vapeur d'eau à l'aide d'un réfrigérant chemisé à serpentins, réception d'une quantité de 8 - 12 ml du produit de condensation, de préférence de 10 ml, dans un récipient en formant un macéré de vapeur d'eau, (WDM),
3. Mélanger le WDM dans un quantité de 10 - 30 µl, de préférence de 24 µl, avec 5 - 20 mg, de préférence de 10 mg, à 460 - 940 °C, de préférence à 640 °C, de mélanges calcinés à l'abri de l'air et ensuite du NaCI, de la protéine animale, de la fructose, de la glucose, de l'amidon finement broyés d'une proportion de poids de 1 : 1 : 1 : 1 : 2 comme support pour une suspension pratiquement saturée et sa filtration par un micro filtre,
4. Verser une goutte de produit filtré d'une quantité de 10 - 50 µl, de préférence de 24 µl, à l'aide d'une pipette sur une lame en verre, la goutte devra avoir un diamètre d'approximativement 8 - 12 mm, placer la lame en verre sur une plaque chauffante de précision ayant une température de 20 - 25 °C, de préférence de 24 °C, évaporer l'eau jusqu'à la phase cristalline,
5. Scanner la phase cristalline sous un microscope optique ou un microscope d'électrons à balayage ou un microscope laser à balayage, spécialement pour les échelles de reproduction de 4 à 16 et mémorisation comme données images,
6. Exploration automatique des données images ou de la phase cristalline sur la lame pour la reconnaissance des structures des Formes et/ou Textures des images avec une similitude géométrique commune,
7. Sélection automatique des structures explorées et reconnues de Formes et/ou Textures pour la recherche de la Forme et/ou Texture qui est la plus nuancée pour le plus petit besoins de place,
8. Projection des Formes et/ou Textures sélectionnées sur un moniteur ou bibliothèque intermédiaire d'un ordinateurs et exploration d'une banque de données dans laquelle les Formes géométriques et les Formes des tableaux
1, 11, 111, 1111,12,121,122,123,13, 131,132,133,21,2, 23, 31,3, 33,14, 22, 32
de la description sont sauvegardées comme Forme et/ou Texture la plus similaire à la Forme et/ou Textures sélectionnée,
9. Définition de la Forme et/ou Texture sélectionnée dans les coordonnées de la Forme et/ou Texture la plus similaire ou égale des tableaux
1,11,111,1111,12,121, 122,123,13,131,132,133, 21,2, 23, 31,3, 33,14, 22, 32
pour une série complète composée de Forme; Fraktal a, b, c; Cluster a, b, c; Kontur a, b, c, d; Präfix a; Texture a; Postfix a; Präfix b; Texture b; Postfix b; Lumen a, b, c comme séquence numérique de la Forme et/ou Texture définie,
10. Introduction de la séquence numérique dans une banque de donnée dans laquelle sont sauvegardées des diagnostics cliniques assurés comme séquences numériques pour la l'établissement d'un diagnostic probabiliste pour la prophylaxie et la prévention.

3. Procédé selon la revendication 1 et 2, dans lequel est utilisé pour la reproduction en image de Formes et Textures simples et complexes et pour leur transformation en séquences numériques d'un cristal les représentations géométriques, tabellaires selon les tableaux 1,11,111,1111,12,121, ,122,123,13,131 132, 133, 21, 2, 23, 31, 3, 33, 14, 22 et 32 de la description.

4. Procédé selon les revendications 1 - 3, dans lequel sont définis les formules identifiées par les abscisses et ordonnées des tableaux 1,11,111,1111,12,121, 122, 123, 13, 131, 132, 133, 2, 3, 14, 22 et 32 selon le paragraphe" Listes des Formes et/ou Textures" de la description.

5. Utilisation du procédé selon les revendications 1 et 2, dans lesquelles les phases 5 et 6 de la revendication 1 et les phases 5 à 10 de la revendication 2 pour la recherche des séquences numériques sur la bases des données images de départ correspondantes, d'autres processus qui fournissent les images et les traitent, qui reconnaissent les échantillons et les comparent et qui sont d'origines variées sont utilisés pour leur classification et pronostic spécialement à l'occasion de processus qui ont à leur base des modes de fonctionnement non-linéaires.
